# EUROPEAN PATENT SPECIFICATION

(11) **EP 3 002 287 B1**
(45) Date of publication and mention of the grant of the patent: **31.01.2018**
(21) Application number: 14187461.0
(22) Date of filing: 02.10.2014
(51) Int. Cl.: C07D 495/04, A61K 31/4743, A61P 35/00, A61P 35/02

(54) **Thiochromeno[2,3-c]quinolin-12-one derivatives and their use as topoisomerase inhibitors**
Thiochromeno[2,3-c]chinolin-12-one Derivative und ihre Verwendung als Topoisomerase-Hemmer
Dérivés de thiochromeno[2,3-c]quinolin-12-one utilisés comme inhibiteurs de la topoisomerase

(43) Date of publication of application: 06.04.2016
(73) Proprietor: National Defense Medical Center, Taipei City 114 (TW)
(72) Inventor: Huang, Hsu-Shan, 114 Taipei City (TW); Yu, Dah-Shyong, 100 Taipei City (TW); Chen, Tsung-Chih, 551 Mingjian Township, Nantou County (TW)
(74) Representative: Copsey, Timothy Graham

(56) References cited:
- CUSHMAN M ET AL: "Synthesis of New Indeno[1,2-c]isoquinolines: Cytotoxic Non-Camptothecin Topoisomerase I Inhibitors", JOURNAL OF MEDICINAL CHEMISTRY, AMERICAN CHEMICAL SOCIETY, US, vol. 43, 1 January 2000 (2000-01-01), pages 3688-3698, XP002263884, ISSN: 0022-2623, DOI: 10.1021/JM000029D
- DIRK STRUMBERG ET AL: "Synthesis of cytotoxic indenoisoquinoline topoisomerase I poisons", JOURNAL OF MEDICINAL CHEMISTRY, AMERICAN CHEMICAL SOCIETY, US, vol. 42, 1 January 1999 (1999-01-01), pages 446-457, XP002431048, ISSN: 0022-2623, DOI: 10.1021/JM9803323
- KHADKA D B ET AL: "Topoisomerase inhibitors as anticancer agents: A patent update", EXPERT OPINION ON THERAPEUTIC PATENTS, INFORMA HEALTHCARE, GB, vol. 23, no. 8, 1 August 2013 (2013-08-01) , pages 1033-1056, XP009173107, ISSN: 1354-3776, DOI: 10.1517/13543776.2013.790958
- MARTINE CROISY-DELCEY ET AL: "Synthesis of 11-Aminosubstituted 6,8-Dimethyl-12H-[1]benzo[5,6]thiopyrano[2 ,3-c]quinolin-12-ones as Benzo Analogues of Lucanthone", HETEROCYCLES : AN INTERNATIONAL JOURNAL FOR REVIEWS AND COMMUNICATIONS IN HETEROCYCLIC CHEMISTRY, JAPAN INSTITUTE OF HETEROCYCLIC CHEMISTRY, JP, vol. 32, no. 10, 1 January 1991 (1991-01-01), pages 1933-1945, XP008173296, ISSN: 0385-5414, DOI: 10.3987/COM-91-5808

## Description

### BACKGROUND OF THE INVENTION

### 1. FIELD OF THE INVENTION

The invention relates to development of cancer drug, especially relates to the development of novel thiochromeno[2,3-*c*]quinolin-12-one derivatives, preparation method and application thereof.

### 2. DESCRIPTION OF THE PRIOR ART

Telomerase is the enzyme that synthesizes telomeric DNA, the terminal DNA at chromosome ends which, together with telomere-binding proteins, confers stability to chromosomes. In most of organism, the replication and maintenance of the length of telomere has to rely on telomerase. The telomerase is composed of RNA and protein subunits. At present, part of important telomerase subunits had been identified. The composition of human telomerase comprising: human telomerase reverse transcriptase (hTERT) having reverse transciptase activity, human telomerase RNA component used as a template, and some telomere-binding proteins such as human telomerase-associated protein, p23, hsp90, hsp40, hsp70 and the like.

Many research studies had indicated that the activity of human telomerase can only be detected in cells having high proliferation ability, for example, germ cells, hemopoietic cells, part of stem cells, most of immortalized cells and most of tumor cells. In the somatic cell, the telomere will be shorten gradually as the number of cell division increased, which may be considered as the mitotic clock for counting the number of cell division. When a telomere is shortened to a certain extent, cell will stop division and entering aging stage, stay at this stage for a period of time, and then goes to death. This period of time is called mortality stage 1 (M1 stage). When a tumor suppressor gene such as p53 or Rb is mutated within M1 stage, the cell might escape from aging stage and keeps on cell division in this period of time which is called mortality stage 2 (M2 stage). If a cell lacks of telomerase activity during this period, the length of a telomere will be reduced still, the telomere will not be able to protect the the terminal end of the chromosome, and this might result into the instability of the chromosome, as well as the cell can not transfer genetic information completely and enters apoptosis in the end. Therefore, M2 stage is also called a crisis satge. Most of cells will die in M2 stage, except small part of cells with telomerase activity will survive. This small part of cells will continue to divide without limitation and become an immortalized cell (or a cancer cell).

In view of the foregoing, it is thought generally that the activation of telomerase can maintain the length of a telomere so as to prevent a cell from entering the ageing stage; or the inhibition of telomerase activity can be used to limit the division of a cancer cell. Both thought may become the key factors in the development of a cell toward immortalization or cancerization. In summary, using the telomerase inhibitors to treat the cancer have been considered as a novel cancer-specific therapy, as most tumor cells have high expression of telomerase, whereas most normal somatic cells express low or undetectable levels of telomerase and is therefore an attractive target for the design of anticancer agents.

Cancers arise from abnormal proliferation of DNA. Therefore, selectively destroy the DNA of cancer cells without damaging the DNA of normal cells is highly desired. However, it is difficult to differentiate the DNAs between normal cells and cancer cells. Consequently, specific 'targeted therapy' was developed following identification of the differences between normal cells and cancer cells, and when combined with other chemotherapies or radiation therapies, targeted therapy can significantly reduce side effects and provide better treatment outcomes. Thus, targeted therapy currently is a popular field in studying cancer treatments. Because topoisomerases have been found to play an indispensible role in DNA replication, they have become the objects of targeted therapy for anticancer treatments. The anticancer drug camptothecin discovered by M.E. Wall and M.C. Wani in 1966 through systematic screening of natural substances is an inhibitor for type I topoisomerases.

Unfortunately, camptothecin has numerous disadvantages and thus cannot be used for clinical treatment. For example, the lactone ring can be easily hydrolyzed to hydroxycarboxylate *in vivo* at the normal pH and then binds to serum albumin and lose its effect of inhibiting the function of type I topoisomerases. In addition, the structure of the tricomplex of camptothecin-Top I-DNA is not stable because the complex is not maintained by covalent bonds and water solubility of camptothecin is poor which causes lower bioavailability. The p-glycoprotein (MDR1, ABC1) efflux transporter proteins in the cell membrane transported the drugs out of the cells and more important is that some tumor cells have slowly developed resistance and adverse drug side effects against camptothecin. As a result, a number of water-soluble semi-synthetic drugs were developed even after commercialization of camptothecin such as Topotecan (HYCAMTIN®) which is used for treating ovarian cancer and Irinotecan (CAMPTO®) which is used for treating colon cancer and both have issues when used for clinical treatment.

Other topoisomerase inhibitors have been available in the prior art (Cushman, et al. J. Med. Chem., 2000, 43 (20), pp 3688-3698; Strumberg, et al. J. Med. Chem., 1999, 42 (3), pp 446-457; Khadka & Cho. Expert Opin. Ther. Pat., 2013 Aug;23(8):1033-56; Croisy-Delcey, et al. Heterocycles, 1991, vol. 32, no10, pp. 1933-1945). However, there still remains a considerable need for new inhibitors that overcome the drawbacks of existing ones.

Hence, based on the importance of topoisomerase inhibitors in development of anticancer drugs, the inventor of this application developed a series of novel thiochromeno[2,3-*c*]quinolin-12-one derivatives and disclosed the preparation methods as well as relevant applications herein after a number of innovative improvements.

### SUMMARY OF THE INVENTION

In one aspect, present invention provides a compound as shown in formulation (I): 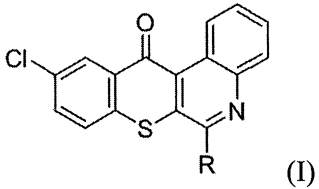 wherein the R is selected from the groups consisting of:
i) halo, amino, hydroxyl and thiol groups;
ii) nitrogen-containing linear alkyl chains of NH(CH₂)ₙH, alkyl groups with substituted side chains, alkyl side chains with a substituted amino group and alkyl side chains with a substituted hydroxyl group, wherein 1 ≤ n ≤ 10;
iii) O(CH₂)ₙH, N(CH₃)₂, NH(CH₂)ₙNH(CH₂)ₙOH, wherein 1 ≤ n ≤ 10;
iv) nitrogen-containing cycloalkyl groups and heterocyclic compounds of C₃₋₁₂ which contain 1 to 3 heteroatoms selected from the group consisting of O, S and N, wherein the ortho-, para- and meta- position can be further selected independently from one of the groups consisting of: hydrogen group, (CH₂)ₙH alkyl groups, (CH₂)ₙ hydroxyl groups, (CH₂)ₙC₃₋₁₂ cycloalkyl groups, (CH₂)ₙC₃₋₁₂ nitrogen-containing cycloalkyl groups, (CH₂)ₙ benzene rings, formyl group and (CH₂)ₙCOC₃₋₁₂ nitrogen-containing cycloalkyl groups, wherein 0 ≤ n ≤ 10;
v) NH(CH₂)ₙR₁, 0 ≤ n ≤ 10, wherein R₁ is selected from the groups consisting of: N(CH₃)₂, C(NH₂)₂, linear alkyl chains of NH(CH₂)ₙH, alkyl groups with substituted side chains, alkyl side chains with a substituted amino group and alkyl side chains with a substituted hydroxyl group;
vi) NH(CH₂)ₙR₂, 0 ≤ n ≤ 10, wherein R₂ is selected from the groups consisting of: benzene rings, C₃₋₁₂ cycloalkyl groups and heterocyclic groups of which contain 1 to 3 heteroatoms selected from the group consisting of O, S and N, wherein the ortho-, para- and meta- position can be further selected independently from one of the groups consisting of: Methoxyl group, amino group, benzene rings, alkyl, amino, nitro,hydroxyl groups with substituted C1-C3 side chains and C₃₋₁₂ heterocyclic groups; wherein the C₃₋₁₂ heterocyclic groups which contain 1 to 3 heteroatoms selected from the group consisting of O, S and N;
and their pharmaceutically acceptable salts, stereoisomers and enantiomers.

According to the invention, wherein the R group consisting of i) ∼ vi) are selected from the group consisting of chlorine, hydroxyl, methoxyl, dimethylamino, piperazin-1-yl, 4-methylpiperazin-1-yl, 4-ethylpiperazin-1-yl, 4-(2-hydroxyethyl)piperazin-1-yl, 4-Benzylpiperazin-1-yl, 4-phenylpiperazin-1-yl, morpholino, thiomorpholino, piperidin-1-yl, 4-hydroxypiperidin-1-yl, 4-Benzylpiperidin-1-yl, (1,4'-Bipiperidin)-1'-yl, 4-(3-(piperidin-4-yl)propyl)piperidin-1-yl, pyrrolidin-1-yl, 2-oxopiperidin-1-yl, methylamino, ethylamino, propylamino, butylamino, isobutylamino, pentan-3-ylamino, (2-(dimethylamino)ethyl)amino, (2-(diethylamino)ethyl)amino, 2-ethanolamino, 3-propanolamino, 5-pentanolamino, (1-hydroxybutan-2-yl)amino, (4-methylpentan-2-yl)amino, (2-Aminoethyl)amino, (2-((2-hydroxyethyl)amino)ethyl)amino, (2-morpholinoethyl)amino, (3-(dimethylamino)propyl)amino, (3-(diethylamino)propyl)amino, (3-((2-hydroxyethyl)amino)propyl)amino, (2,3-dihydro-1H-inden-2-yl)amino, cyclohexylamino, (1-Benzylpiperidin-4-yl)amino, (thiophen-2-ylmethyl)amino, (cyclohexylmethyl)amino, benzylamino, (pyridin-2-ylmethyl)amino, (Benzo[d][1,3]dioxol-5-ylmethyl)amino, (2-methoxybenzyl)amino, (3,4-dimethoxybenzyl)amino, phenethylamino, (4-methoxyphenethyl)amino, (4-aminophenethyl)amino, guanidine and piperidin-1-ylamino.

According to the invention, wherein the compound is selected from the group consisting of:
3-((4-Chlorophenyl)thio)-2-hydroxyquinoline-4-carboxylic acid,
6,9-Dichloro-12H-thiochromeno[2,3-c]quinolin-12-one,
10-Chloro-6-hydroxy-12H-thiochromeno[2,3-c]quinolin-12-one,
10-Chloro-6-methoxy-12H-thiochromeno[2,3-c]quinolin-12-one 10-C hloro-6-dimethylamino-12H-thiochromeno[2,3-c]quinolin-12-one,
10-Chloro-6-(piperazin-1-yl)-12H-thiochromeno[2,3-c]quinolin-12-on e,
10-Chloro-6-(4-methylpiperazin-1-yl)-12H-thiochromeno[2,3-c]quino lin-12-one,
10-Chloro-6-(4-ethylpiperazin-1-yl)-12H-thiochromeno[2,3-c]quinoli n-12-one,
10-Chloro-6-(4-(2-hydroxyethyl)piperazin-1-yl)-12H-thiochromeno[2, 3-c]quinolin-12one,
6-(4-Benzylpiperazin-1-yl)-10-chloro-12H-thiochromeno[2,3-c]quinol in-12-one,
10-Chloro-6-(4-phenylpiperazin-1-yl)-12H-thiochromeno[2,3-c]quino lin-12-one,
10-Chloro-6-morpholino-12H-thiochromeno[2,3-c]quinolin-12-one,
10-Chloro-6-thiomorpholino-12H-thiochromeno[2,3-c]quinolin-12-on e,
10-Chloro-6-(piperidin-1-yl)-12H-thiochromeno[2,3-c]quinolin-12-on e,
10-Chloro-6-(4-hydroxypiperidin-1-yl)-12H-thiochromeno[2,3-c]quin olin-12-one,
6-(4-Benzylpiperidin-1-yl)-10-chloro-12H-thiochromeno[2,3-c]quinol in-12-one,
6-([1,4'-Bipiperidin]-1'-yl)-10-chloro-12H-thiochromeno[2,3-c]quinoli n-12-one,
10-Chloro-6-(4-(3-(piperidin-4-yl)propyl)piperidin-1--yl)-12H-thiochr omeno[2,3-c]quinolin-12-one,
10-Chloro-6-(pyrrolidin-1-yl)-12H-thiochromeno[2,3-c]quinolin-12-o ne,
10-Chloro-6-(2-oxopiperidin-1-yl)-12H-thiochromeno[2,3-c]quinolin-12-one,
10-Chloro-6-methylamino-12H-thiochromeno[2,3-c]quinolin-12-one,
10-Chloro-6-ethylamino-12H-thiochromeno[2,3-c]quinolin-12-one,
10-Chloro-6-propylamino-12H-thiochromeno[2,3-c]quinolin-12-one,
6-(Butylamino)-10-chloro-12H-thiochromeno[2,3-c]quinolin-12-one,
10-Chloro-6-isobutylamino-12H-thiochromeno[2,3-c]quinolin-12-one,
10-Chloro-6-(pentan-3-ylamino)-12H-thiochromeno[2,3-c]quinolin-1 2-one,
10-Chloro-6-((2-(dimethylamino)ethyl)amino)-12H-thiochromeno[2,3 -c]quinolin-12-one,
10-Chloro-6-((2-(diethylamino)ethyl)amino)-12H-thiochromeno[2,3-c ]quinolin-12-one,
10-Chloro-6-(2-ethanolamino)-12H-thiochromeno[2,3-c]quinolin-12-one,
10-Chloro-6-(3-propanolamino)-12H-thiochromeno[2,3-c]quinolin-12 -one,
10-Chloro-6-(5-pentanolamino)-12H-thiochromeno[2,3-c]quinolin-12 -one,
10-Chloro-6-((1-hydroxybutan-2-yl)amino)-12H-thiochromeno[2,3-c] quinolin-12-one,
10-Chloro-6-((4-methylpentan-2-yl)amino)-12H-thiochromeno[2,3-c] quinolin-12-one,
6-((2-Aminoethyl)amino)-10-chloro-12H-thiochromeno[2,3-c]quinoli n-12-one,
10-Chloro-6-((2-((2-hydroxyethyl)amino)ethyl)amino)-12H-thiochro meno[2,3-c]quinolin-12-one,
10-Chloro-6-((2-morpholinoethyl)amino)-12H-thiochromeno[2,3-c]qu inolin-12-one,
10-Chloro-6-((3-(dimethylamino)propyl)amino)-12H-thiochromeno[2, 3-c]quinolin-12-one,
10-Chloro-6-((3-(diethylamino)propyl)amino)-12H-thiochromeno[2,3 -c]quinolin-12-one,
10-Chloro-6-((3-((2-hydroxyethyl)amino)propyl)amino)-12H-thiochro meno[2,3-c]quinolin-12-one,
10-Chloro-6-((2,3-dihydro-1H-inden-2-yl)amino)-12H-thiochromeno[ 2,3-c]quinolin-12-one,
10-Chloro-6-(cyclohexylamino)-12H-thiochromeno[2,3-c]quinolin-12 -one,
6-((1-Benzylpiperidin-4-yl)amino)-10-chloro-12H-thiochromeno[2,3-c]quinolin-12-one,
10-Chloro-6-((thiophen-2-ylmethyl)amino)-12H-thiochromeno[2,3-c] quinolin-12-one,
10-Chloro-6-((cyclohexylmethyl)amino)-12H-thiochromeno[2,3-c]qui nolin-12-one,
6-(Benzylamino)-10-chloro-12H-thiochromeno[2,3-c]quinolin-12-one,
10-Chloro-6-((pyridin-2-ylmethyl)amino)-12H-thiochromeno[2,3-c]q uinolin-12-one,
6-((Benzo[d][1,3]dioxol-5-ylmethyl)amino)-10-chloro-12H-thiochrom eno[2,3-c]quinolin-12-one,
10-Chloro-6-((2-methoxybenzyl)amino)-12H-thiochromeno[2,3-c]qui nolin-12-one,
10-Chloro-6-((3,4-dimethoxybenzyl)amino)-12H-thiochromeno[2,3-c] quinolin-12-one,
10-Chloro-6-(phenethylamino)-12H-thiochromeno[2,3-c]quinolin-12-one,
10-Chloro-6-((4-methoxyphenethyl)amino)-12H-thiochromeno[2,3-c] quinolin-12-one,
6-((4-Aminophenethyl)amino)-10-chloro-12H-thiochromeno[2,3-c]qu inolin-12-one,
2-(10-Chloro-12-oxo-12*H*-thiochromeno[2,3-*c*]quinolin-6-yl)guanidin e,
10-Chloro-6-(piperidin-1-ylamino)-12*H-*thiochromeno[2,3-*c*]quinolin-12-one,
and their salts.

In another aspect, the invention provides a pharmaceutical composition comprising an effective amount of the abovementioned compound and at least one pharmaceutically acceptable vehicle, diluent or excipient.

In another aspect, the invention provides the presently disclosed compound for use in a method for inhibiting the Topoisomerase I activity of cancer cell, the method comprising administrating an effective amount of said compound.

In another aspect, the invention provides the presently disclosed compound for use in a method for inhibiting the Topoisomerase II activity of cancer cell, the method comprising administrating an effevtive amount of said compound.

In another aspect, the invention relates to the compound described herein for use in a method for the treatment of cancer, the method comprising administrating an effective amount of said compound.

According to the invention,, wherein the cancer is selected from the groups consisting of leukemia, non-small cell lung cancer, colorectal cancer, central nervous system (CNS) cancer, melanoma, ovarian cancer, renal cancer, prostate cancer and breast cancer.

In another aspect, the invention provides a method for preparation of thiochromeno[2,3-*c*]quinolin-12-one derivatives, the method comprising:
(1) mix isatin, 2-((4-chlorophenyl)thio)acetic acid and sodium acetate was heated at 150 °C for 1 h, after cooling the mixture was added acetic acid, the precipitate was collected, washed with acetic acid, water and n-hexane, and obtained compound 2 (3-((4-Chlorophenyl)thio)-2-hydroxyquinoline-4-carboxylic acid);
(2) a solution of compound 2 (3-((4-Chlorophenyl)thio)-2-hydroxyquinoline-4-carboxylic acid) in phosphoryl trichloride was heated at 150 °C for 48 h, after cooling the mixture was poured into water 0 °C, the precipitate was collected by filtration, then added into 10% NaHCO₃ with vigorous stirring for 1 h, the resulting precipitate was collected and washed with H₂O, the crude solid was recrystallized by dichloromethane to give compound 3 (6,9-Dichloro-12H-thiochromeno[2,3-c] quinolin-12-one);
(3) a solution of compound 3 (6,9-Dichloro-12H-thiochromeno [2,3-c]quinolin-12-one) in DMF was added conc. HCl and refluxed, after 6 hours, the conc. HCl was added dropwise and refluxed for another 12 hours, the mixture was evaporated in vacuo and treated with H₂O, after filtered the crude solid was washed with EtOH to give compound 4 (10-Chloro-6-hydroxy-12H-thiochromeno[2,3-c]quinolin-12-one);
(4) a suspension of compound 3 (6,9-Dichloro-12H-thiochromeno[2,3-c]quinolin-12-one) and sodium methoxide in methanol was refluxed for 16 h, after cooled the solvent was removed, filtrated and washed with ethanol and n-hexane to collect compound 5 (10-Chloro-6-methoxy-12H-thiochromeno[2,3-c]quinolin-12-one);
(5) a solution of compound 3 (6,9-Dichloro-12H-thiochromeno[2,3-c]quinolin-12-one), appropriate secondary amines and sodium carbonate in DMSO was refluxed for 10 hours, then the reaction was added ice-water, the precipitate was filtered, washed with water/methanol to collect the following compound:
   10-Chloro-6-dimethylamino-12H-thiochromeno[2,3-c]quinoli n-12-one,
   10-Chloro-6-(piperazin-1-yl)-12H-thiochromeno[2,3-c]quinol in-12-one,
   10-Chloro-6-(4-methylpiperazin-1-yl)-12H-thiochromeno[2,3 -c]quinolin-12-one,
   10-Chloro-6-(4-ethylpiperazin-1-yl)-12H-thiochromeno[2,3-c ]quinolin-12-one,
   10-Chloro-6-(4-(2-hydroxyethyl)piperazin-1-yl)-12H-thiochr omeno[2,3-c]quinolin-12one,
   6-(4-Benzylpiperazin-1-yl)-10-chloro-12H-thiochromeno[2,3-c]quinolin-12-one,
   10-Chloro-6-(4-phenylpiperazin-1-yl)-12H-thiochromeno[2,3 -c]quinolin-12-one,
   10-Chloro-6-morpholino-12H-thiochromeno[2,3-c]quinolin-1 2-one,
   10-Chloro-6-thiomorpholino-12H-thiochromeno[2,3-c]quinol in-12-one,
   10-Chloro-6-(piperidin-1-yl)-12H-thiochromeno[2,3-c]quinol in-12-one,
   10-Chloro-6-(4-hydroxypiperidin-1-yl)-12H-thiochromeno[2, 3-c]quinolin-12-one,
   6-(4-Benzylpiperidin-1-yl)-10-chloro-12H-thiochromeno[2,3-c]quinolin-12-one,
   6-([1,4'-Bipiperidin]-1'-yl)-10-chloro-12H-thiochromeno[2,3-c]quinolin-12-one,
   10-Chloro-6-(4-(3-(piperidin-4-yl)propyl)piperidin-1-yl)-12H -thiochromeno[2,3-c]quinolin-12-one,
   10-Chloro-6-(pyrrolidin-1-yl)-12H-thiochromeno[2,3-c]quino lin-12-one, or
   10-Chloro-6-(2-oxopiperidin-1-yl)-12H-thiochromeno[2,3-c] quinolin-12-one respectively;
(6) a solution of compound 3 (6,9-Dichloro-12H-thiochromeno[2,3-c]quinolin-12-one) in DMSO was added appropriate primary amines and refluxed for 8 hours, after cooled the reaction was added water, the precipitate was filtered and washed with water and methanol to collect the following compound:
   10-Chloro-6-methylamino-12H-thiochromeno[2,3-c]quinolin-12-one,
   10-Chloro-6-ethylamino-12H-thiochromeno[2,3-c]quinolin-1 2-one,
   10-Chloro-6-propylamino-12H-thiochromeno[2,3-c]quinolin-12-one,
   6-(Butylamino)-10-chloro-12H-thiochromeno[2,3-c]quinolin-12-one,
   10-Chloro-6-isobutylamino-12H-thiochromeno[2,3-c]quinoli n-12-one,
   10-Chloro-6-(pentan-3-ylamino)-12H-thiochromeno[2,3-c]qu inolin-12-one,
   10-Chloro-6-((2-(dimethylamino)ethyl)amino)-12H-thiochro meno[2,3-c]quinolin-12-one,
   10-Chloro-6-((2-(diethylamino)ethyl)amino)-12H-thiochrome no[2,3-c]quinolin-12-one,
   10-Chloro-6-(2-ethanolamino)-12H-thiochromeno[2,3-c]quin olin-12-one,
   10-Chloro-6-(3-propanolamino)-12H-thiochromeno[2,3-c]qui nolin-12-one,
   10-Chloro-6-(5-pentanolamino)-12H-thiochromeno[2,3-c]qui nolin-12-one,
   10-Chloro-6-((1-hydroxybutan-2-yl)amino)-12H-thiochromen o[2,3-c]quinolin-12-one,
   10-Chloro-6-((4-methylpentan-2-yl)amino)-12H-thiochromen o[2,3-c]quinolin-12-one,
   6-((2-Aminoethyl)amino)-10-chloro-12H-thiochromeno[2,3-c ]quinolin-12-one,
   10-Chloro-6-((2-((2-hydroxyethyl)amino)ethyl)amino)-12H-t hiochromeno[2,3-c]quinolin-12-one,
   10-Chloro-6-((2-morpholinoethyl)amino)-12H-thiochromeno[ 2,3-c]quinolin-12-one,
   10-Chloro-6-((3-(dimethylamino)propyl)amino)-12H-thiochr omeno[2,3-c]quinolin-12-one,
   10-Chloro-6-((3-(diethylamino)propyl)amino)-12H-thiochro meno[2,3-c]quinolin-12-one,
   10-Chloro-6-((3-((2-hydroxyethyl)amino)propyl)amino)-12H -thiochromeno[2,3-c]quinolin-12-one,
   10-Chloro-6-((2,3-dihydro-1H-inden-2-yl)amino)-12H-thioch romeno[2,3-c]quinolin-12-one,
   10-Chloro-6-(cyclohexylamino)-12H-thiochromeno[2,3-c]qui nolin-12-one,
   6-((1-Benzylpiperidin-4-yl)amino)-10-chloro-12H-thiochrom eno[2,3-c]quinolin-12-one,
   10-Chloro-6-((thiophen-2-ylmethyl)amino)-12H-thiochromen o[2,3-c]quinolin-12-one,
   10-Chloro-6-((cyclohexylmethyl)amino)-12H-thiochromeno[ 2,3-c]quinolin-12-one,
   6-(Benzylamino)-10-chloro-12H-thiochromeno[2,3-c]quinoli n-12-one,
   10-Chloro-6-((pyridin-2-ylmethyl)amino)-12H-thiochromeno [2,3-c]quinolin-12-one,
   6-((Benzo[d][1,3]dioxol-5-ylmethyl)amino)-10-chloro-12H-t hiochromeno[2,3-c]quinolin-12-one,
   10-Chloro-6-((2-methoxybenzyl)amino)-12H-thiochromeno[2 ,3-c]quinolin-12-one,
   10-Chloro-6-((3,4-dimethoxybenzyl)amino)-12H-thiochrome no[2,3-c]quinolin-12-one,
   10-Chloro-6-(phenethylamino)-12H-thiochromeno[2,3-c]quin olin-12-one,
   10-Chloro-6-((4-methoxyphenethyl)amino)-12H-thiochromen o[2,3-c]quinolin-12-one,
   6-((4-Aminophenethyl)amino)-10-chloro-12H-thiochromeno[ 2,3-c]quinolin-12-one,
   2-(10-Chloro-12-oxo-12*H*-thiochromeno[2,3-*c*]quinolin-6-yl) guanidine, or
   10-Chloro-6-(piperidin-1-ylamino)-12*H-*thiochromeno[2,3-*c*] quinolin-12-one respectively.

These features and advantages of the present invention will be fully understood and appreciated from the following detailed description of the accompanying Drawings.

### BRIEF DESCRIPTION OF THE DRAWINGS

Fig. 1 depicts the general scheme for a series of thiochromeno [2,3-c]quinolin-12-one derivatives.
Fig. 2 is the effect of compounds **5, 7**, **8**, **16**, **19** and CPT on DNA relaxation catalyzed by TOP I at a concentration of 25 µM and 50 µM.
Fig. 3 is the effect of compounds **N2, N7, N8, N9, N14-N19, N25,** and CPT on DNA relaxation catalyzed by TOP I at a concentration of 50 µM.
Fig. 4a-d are the effects of compounds **7, N7, N14, N15, N18, N19** and **N25** on TOP I mediated supercoiled DNA relaxation.
Fig. 5 is the effect of compounds **5, 7, 8, 16, 19** and VP-16 on DNA relaxation catalyzed by TOP II at a concentration of 25 µM and 50 µM.
Fig. 6 is the effect of compounds **N2, N7, N8, N9, N14-N19, N25,** and VP-16 on DNA relaxation catalyzed by TOP II at a concentration of 50 µM.
Fig. 7a-d are the effects of compounds **7, N7, N8, N14, N15, N18,** and **N19** on TOP II mediated supercoiled DNA relaxation.

### DETAILED DESCRIPTION OF THE PREFERRED EMBODIMENT

Unless defined otherwise, all technical and scientific terms used herein have the meaning commonly understood by a person skilled in the art to which this invention belongs. As used herein, the following terms have the meanings ascribed to them unless specified otherwise. The present invention will now be described more specifically with reference to the following embodiments, which are provided for the purpose of demonstration rather than limitation.

The term **"treatment", "under treatment"** and similar terms refer to the methods which ameliorate, improve, reduce or reverse the patient's disease or any relevant symptoms caused by the disease, or methods which can prevent onset of such diseases or any resulting symptoms.

The term **""pharmaceutically acceptable"** is used to describe substances to be used in the composition must be compatible with other ingredients in the formulation and be harmless to the subject.

The inventive composition can be prepared into a dosage form for suitable application of the inventive composition by using technology commonly understood by a person skilled in the art through formulating the abovementioned *Lactobacillus* isolated strain(s) with a pharmaceutically acceptable vehicle, wherein the excipients include, but are not limited to, solution, emulsion, suspension, powder, tablet, pill, lozenge, troche, chewing gum, slurry, and other suitable forms.

The pharmaceutically acceptable vehicle may contain one or several reagents selecting form the following list: solvents, emulsifiers, suspending agents, decomposers, binding agents, excipients, stabilizing agents, chelating agents, diluents, gelling agents, preservatives, lubricants, surfactants and other agents suitable for use in the invention.

In the abovementioned compositions, one or more dissolving aids, buffers, preservatives, colorants, fragrances, flavoring agents and the like, which are commonly used for formulation can be added as desired.

The term **"pharmaceutically acceptable excipients",** as used herein, refers to substances known by persons skilled in the art, which are physiologically inert, pharmacologically inactive and are compatible with the physical as well as chemical characteristics of sorafenib or GW5074. Pharmaceutically acceptable excipients include, but are not limited to, polymers, resins, plasticizers, fillers, lubricants, diluents, binders, disintegrants, solvents, co-solvents, surfactants, preservatives, sweetening agents, flavoring agents, pharmaceutical grade dyes or pigments, and viscosity agents.

The term **"pharmaceutical composition"** is used to describe solid or liquid compositions in a form, concentration and purity that are suitable for administration in patients (e.g. humans or animals) and can induce desired physiological changes following administration. Pharmaceutical compositions are typically sterile and non-pyrogenic.

The present invention will now be described more specifically with reference to the following embodiments, which are provided for the purpose of demonstration rather than limitation. The drugs as well as biomaterials used in the invention are all commercially available materials and the sources disclosed below are merely examples.

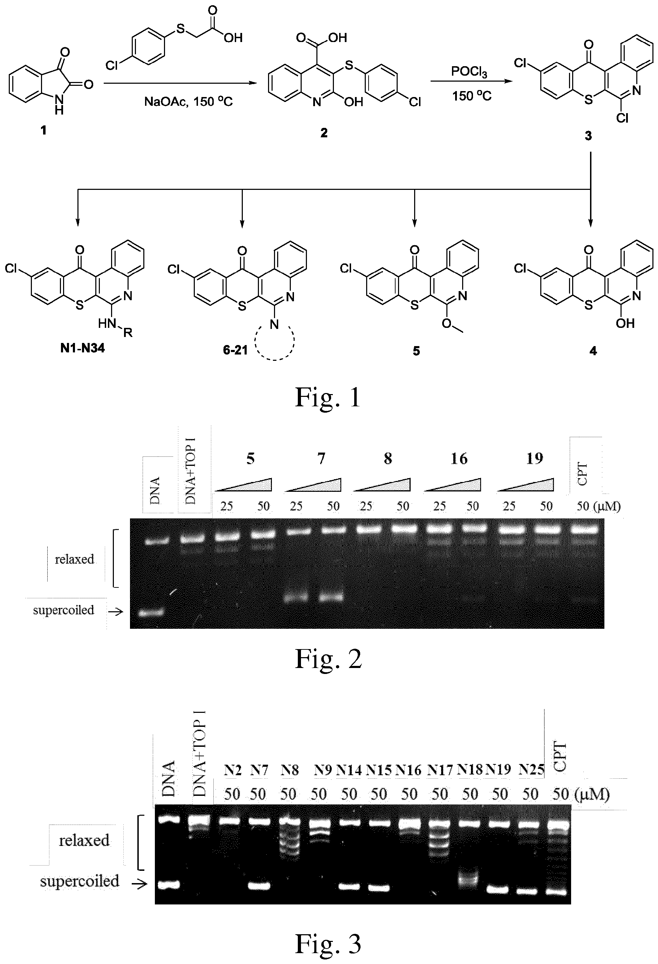

All reactions were monitored by thin-layer chromatography (TLC) coated with silica gel 60 F₂₅₄. Melting points of all synthetic compounds were measured with Büchi B-545 melting point apparatus. ¹H NMR: Varian GEMINI-300 (300 MHz) or Agilent 400 MR DD2 (400 MHz); δ values are in ppm relative to tetramethylsilane (TMS) as an internal standard (0 ppm). Multiplicities are recorded as s (singlet), d (doublet), t (triplet), q (quartet), quin (quintuplet), sext (sextet), sep (septet), m (multiplet), dd (doublet of doublet), dt (doublet of triplet), td (triplet of doublet), qd (quartet of doublet) and br (broadened). Mass spectra: High resolution electrospray ionization (HRESI): Finnigan MAT 95S (Instrumentation Center, National Taiwan University, Taipei, Taiwan). X-ray Single Crystal Diffraction: Bruker Enraf-Nonius APEX II diffractometer (Department of Chemistry, National Taiwan Normal University). Typical experiments illustrating the general procedures for the preparation of the thiochromenoquinolones are described below (Fig. 1).

### General procedures for chemical synthesis

### General procedure A : Preparation of compound 2

A mixture of isatin (1) (0.44 g, 2.99 mmol), 2-((4-chlorophenyl)thio)acetic acid (0.70 g, 3.47 mmol), and sodium acetate (0.05 g) was heated at 150 °C in miniclave for 1 h (TLC monitored). After cooling, the mixture was added acetic acid 10 mL, and the gray precipitate was collected, washed with acetic acid, water and n-hexane, and obtained light purple compound.

### General procedure B : Preparation of compound 3

A solution of compound 2 (0.55 g, 2.1 mmol) in phosphoryl trichloride (5 mL) was heated at 150 °C for 48 h. After cooling, the mixture was poured into ice (50 mL) at 0 °C. The resulting green precipitate that separated was collected by filtration. The filtered cake was suspended in 10% NaHCO₃ solution (50 mL) with vigorous stirring for 1 h. The resulting precipitate was collected and washed with H₂O. The crude solid was recrystallized from dichloromethane to give yellow product.

### General procedure C : Preparation of compound 4

To a solution of compound 3 (0.32 g, 0.96 mmol) in DMF (20 mL) was added conc. HCl (3 mL) and refluxed. After 6 h, the conc. HCl (6 mL) was added dropwise and refluxed for another 12 h. The mixture was evaporated in vacuo and treated with H₂O (20 mL), after filtered the crude solid was washed with EtOH to give yellow solid.

### General procedure D : Preparation of compound 5

A suspension of compound 3 (0.33 g, 1.0 mmol) and sodium methoxide (0.55g, 10 mmol) in methanol (20 mL) was refluxed for 16 h. After cooled, the solvent was removed by rotarvapor vacuum, filtrated and washed with ethanol and n-hexane to collect the white solid.

### General procedure E : Preparation of compounds 6-21

Compound 3 (0.33 g, 1.0 mmol), appropriate secondary amines (1.1 mmol) and sodium carbonate (5 mmol) in DMSO (20 mL) was refluxed for 10 h (TLC monitored). After 30 min, the reaction was added ice-water (100 mL). The precipitate was filtered, washed with water/methanol and collected to get the yellow solid.

### General procedure F : Preparation of compounds N1-N32

To a solution of compound 3 (0.33 g, 1.0 mmol) in DMSO (30 mL) was added appropriate primary amines (1.1 mmol) and refluxed for 8 h (TLC monitored). After cooled, the reaction was added water (100 mL). The precipitate was filtered and washed with water and hot methanol to collect the yellow solid.

### Example 1

### 3-((4-Chlorophenyl)thio)-2-hydroxyquinoline-4-carboxylic acid (TC-SCl) (2)

The pure compound was obtained as a gray solid (yield 86%). (*R_{f}* = 0.5 at EA: AcOH = 20: 1). Mp 306-308 °C. ¹H NMR (300 MHz, DMSO-*d*₆): δ (ppm) 7.26 (3H, t, *J* = 7.6 Hz, Ar-H), 7.34 (2H, d, *J* = 6.0 Hz, Ar-H), 7.39 (1H, d, *J* = 8.0 Hz, Ar-H), 7.46 (1H, d, *J* = 8.0 Hz, Ar-H), 7.62 (1H, t, *J* = 8.0 Hz, Ar-H), 12.22 (1H, s, -COOH). ¹³C NMR (100 MHz, DMSO-*d*₆): δ (ppm) 115.58, 116.26, 120.36, 123.21, 126.21, 129.33, 130.30, 131.47, 132.54, 134.36, 140.11, 151.69, 159.37, 166.80 (CO). HRMS (ESI) calcd for C₁₆H₁₀NO₃SCl [M]⁺ 331.0070; found [M+H]⁺ 332.0147 (100), [M+H+2]⁺ 334.0122 (33); found [M-H]⁻ 330.0002.

### Example 2

### 6,9-Dichloro-12H-thiochromeno[2,3-c]quinolin-12-one (3)

The yellow solid material was isolated in 90% yield (*R_{f}* = 0.50 at CH₂Cl₂: n-hexane = 1: 1). Mp 259-261°C (CH₂Cl₂). ¹H NMR (400 MHz, CDCl₃): δ (ppm) 7.71 (2H, m, Ar-H), 7.77-7.85 (2H, m, Ar-H), 8.10-8.13 (m, 1H, Ar-H), 8.60 (t, 1H, *J* = 1.2 Hz, Ar-H), 9.67-9.71 (1H, m, Ar-H). ¹³C NMR (100 MHz, CDCl₃): δ (ppm) 124.87, 126.28, 127.85, 129.17, 129.39, 129.93, 130.31, 131.20, 131.90, 133.01, 133.09, 133.38, 134.43, 145.27, 146.61, 180.64 (CO). HRMS (ESI) calcd for C₁₆H₇NOSCl₂ [M]⁺ 330.9625; found [M+H]⁺ 331.9699 (100), [M+H+2]⁺ 333.9672 (67), [M+H+4]⁺ 335.9645 (11).

### Example 3

### 10-Chloro-6-hydroxy-12H-thiochromeno[2,3-c]quinolin-12-one (4)

The yellow solid material was isolated in 95% yield (*R_{f}* = 0.40 at EA). Mp > 410 °C. ¹H NMR (400 MHz, DMSO-*d*₆): δ ppm 7.35 (1H, td, *J* = 7.2, 1.2 Hz, Ar-H), 7.47 (1H, dd, *J* = 8.4, 1.2 Hz, Ar-H), 7.59 (1H, td, *J* = 7.2 Hz, 1.6 Hz, Ar-H), 7.89 (1H, dd, *J* = 8.4 Hz, 2.4 Hz, Ar-H), 8.10 (1H, d, *J* = 8.8 Hz, Ar-H), 8.38 (1H, d, *J* = 2.4 Hz, Ar-H), 9.35 (1H, dd, *J* = 8.4, 2.4 Hz, Ar-H), 12.73 (br, 1H, -OH). ¹³C NMR (75 MHz, CDCl₃): 8 (ppm) 116.61, 117.52, 123.65, 126.82, 128.44, 130.22, 130.49, 130.54, 132.52, 133.00, 133.42, 135.09, 136.27, 138.90, 158.70, 180.38 (CO). HRMS (ESI) m/z calcd for C₁₆H₈NO₂SCl [M]⁺: 312.9964, found, 314.0051.

### Example 4

### 10-Chloro-6-methoxy-12H-thiochromeno[2,3-c]quinolin-12-one (5)

The gray solid material was isolated in 91% yield (*R_{f}* = 0.52 at CH₂Cl₂: n-hexane = 1: 1). Mp 227-228 °C. ¹H NMR (400 MHz, CDCl₃): δ (ppm) 4.27 (3H, s, -OCH₃), 7.60 (1H, td, *J* = 7.6, 1.2 Hz, Ar-H), 7.37 (1H, d, *J* = 2.0 Hz, Ar-H), 7.70 (1H, td, *J* = 7.6 Hz, 1.6 Hz, Ar-H), 7.94 (1H, dd, *J* = 8.0 Hz, 1.2 Hz, Ar-H), 8.60 (1H, d, *J* = 1.6 Hz, Ar-H), 9.64 (1H, dd, *J* = 8.8 Hz, 1.2 Hz, Ar-H). ¹³C NMR (100 MHz, CDCl₃): δ (ppm) 54.83 (OCH₃), 122.91, 126.23, 126.54, 126.76, 127.66, 127.95, 129.23, 129.50, 130.54, 132.49, 133.48, 133.85, 143.82, 156.06, 180.47 (CO). HRMS (ESI) m/z calcd for C₁₇H₁₀NO₂SCl [M]⁺ 327.0121; found [M+H]⁺ 328.0203, [M+H+2]⁺ 330.0172.

### Example 5

### 10-Chloro-6-dimethylamino-12H-thiochromeno[2,3-c]quinolin-12-one (6)

Product 6 was prepared from 3 and dimethylamine. The light-yellow solid material was isolated in 85% yield (*R_{f}* = 0.45 at CH₂Cl₂: n-hexane = 1: 1). Mp 194-195 °C. ¹H NMR (400 MHz, CDCl₃): δ (ppm) 3.06 (6H, s, -CH₃), 7.59-7.67 (3H, m, Ar-H), 7.71 (1H, t, *J* = 7.2 Hz, Ar-H), 8.00 (1H, d, *J* = 8.4 Hz, Ar-H), 8.59 (1H,d, *J* = 1.2 Hz, Ar-H), 9.60 (1H, d, *J* = 8.4 Hz, Ar-H). ¹³C NMR (100 MHz, CDCl₃): δ (ppm) 43.00, 123.44, 125.71, 127.22, 127.78 128.47, 129.07, 129.37, 130.59, 130.67, 132.23, 132.46, 133.61, 134.36, 144.84, 158.32, 181.52 (CO). HRMS (ESI) calcd for C₁₈H₁₃N₂OSCl [M]⁺ 340.0437; found [M+H]⁺ 341.0517 (100), [M+H+2]⁺ 343.0501 (33).

### Example 6

### 10-Chloro-6-(piperazin-1-yl)-12H-thiochromeno[2,3-c]quinolin -12-one (7)

Product 7 was prepared from 3 and piperazine. The dark-yellow solid material was isolated in 69% yield (*R_{f}* = 0.12 at EA: MeOH: ammonia water = 20: 5: 1). Mp 211-213 °C. ¹H NMR (400 MHz, CDCl₃): δ (ppm) 3.20 (4H, t, *J* = 4.8 Hz, -CH₂-), 3.36 (4H, t, *J* = 4.8 Hz, -CH₂-), 7.60-7.66 (3H, m, Ar-H), 7.70 (1H, td, *J* = 8.0, 1.2 Hz, Ar-H), 7.99 (1H, dd, *J* = 8.4, 0.8 Hz, Ar-H), 8.56 (1H, d, *J* = 2.0 Hz, Ar-H), 9.60 (1H, dd, *J* = 8.4, 0.8 Hz, Ar-H). ¹³C NMR (100 MHz, CDCl₃): δ (ppm) 45.95, 52.31, 123.62, 125.81, 127.58, 127.86, 128.72, 129.10, 129.37, 130.65, 130.99, 132.17,132.47, 133.63, 134.33, 144.94, 157.61, 181.45 (CO). HRMS (ESI) calcd for C₂₀H₁₆N₃OSCl [M]⁺ 381.0703; found [M+H]⁺ 382.0783.

### Example 7

### 10-Chloro-6-(4-methylpiperazin-1-yl)-12H-thiochromeno[2,3-c]q uinolin-12-one (8)

Product 8 was prepared from 3 and 1-methylpiperazine. The green-yellow solid material was isolated in 80% yield (*R_{f}* = 0.24 at EA: methanol = 5: 1). Mp 212-214 °C. ¹H NMR (400 MHz, CDCl₃): δ (ppm) 2.51 (3H, s, -CH₃), 2.84 (4H, br, -CH₂-), 3.50 (4H, t, *J* = 4.5 Hz, -CH₂-), 7.60-7.66 (3H, m, Ar-H), 7.68-7.72 (1H, td, *J* = 8.1, 1.5 Hz, Ar-H), 8.01 (1H, dd, *J* = 8.1, 1.5 Hz, Ar-H), 8.56 (1H, d, *J* = 1.5 Hz, Ar-H), 9.60 (1H, dd, *J* = 8.4, 1.5 Hz, Ar-H). ¹³C NMR (100 MHz, CDCl₃): δ (ppm) 45.73, 50.31, 54.65, 123.70, 125.90, 127.60, 127.80, 128.82, 129.20, 129.39, 130.62, 130.84, 132.36, 132.47, 133.78, 134.24, 145.08, 157.18, 181.42 (CO). HRMS (ESI) calcd for C₂₁H₁₈N₃OSCl [M]⁺ 395.0859; found [M+H]⁺ 396.0926.

### Example 8

### 10-Chloro-6-(4-ethylpiperazin-1-yl)-12H-thiochromeno[2,3-c]q uinolin-12-one (9)

Product **9** was prepared from **3** and 1-ethylpiperazine. The yellow solid material was isolated in 74% yield (*R_{f}* = 0.48 at EA: MeOH = 10: 1). Mp 196-198 °C. ¹H NMR (400 MHz, CDCl₃): δ (ppm) 1.19 (3H, t, *J* = 7.2 Hz, -CH₃), 2.58 (2H, q, *J* = 7.2 Hz, -CH₂-), 2.78 (4H, br, -CH₂-), 3.46 (4H, *t, J* = 4.4 Hz, -CH₂-), 7.61-7.66 (3H, m, Ar-H), 7.68-7.73 (1H, td, *J* = 8.4, 1.6 Hz, Ar-H), 8.01 (1H, dd, *J* = 8.0, 1.2 Hz, Ar-H), 8.59 (1H, d, *J* = 4.0 Hz, Ar-H), 9.62 (1H, dd, *J* = 8.4, 0.8 Hz, Ar-H). ¹³C NMR (100 MHz, CDCl₃): δ (ppm) 12.07, 50.88, 52.46, 52.67, 123.55, 125.77, 127.48, 127.83, 128.78, 129.12, 129.36, 130.62, 130.76, 132.21, 132.47, 133.62, 134.34, 144.97, 157.34, 181.50 (CO). HRMS (ESI) calcd for C₂₂H₂₀N₃OSCl [M]⁺ 409.1016; found [M+H]⁺ 410.1069.

### Example 9

### 10-Chloro-6-(4-(2-hydroxyethyl)piperazin-1-yl)-12H-thiochrom eno[2,3-c]quinolin-12one (10)

Product **10** was prepared from **3** and 2-(piperazin-1-yl)ethanol. The green-yellow solid material was isolated in 60% yield (*R_{f}* = 0.37 at EA: MeOH= 2: 1). Mp 211-213 °C. ¹H NMR (400 MHz, CDCl₃): δ (ppm) 2.74 (2H, *t, J* = 5.2 Hz, -CH₂-), 2.87 (4H, t, *J* = 3.6 Hz, -CH₂-), 3.45 (4H, t, *J* = 3.6 Hz, -CH₂-), 3.72 (2H, t, *J* = 5.2 Hz, -CH₂O-), 7.62-7.67 (3H, m, Ar-H), 7.72 (1H, td, *J* = 7.2, 1.6 Hz, Ar-H), 8.01 (1H, dd, *J* = 8.4, 1.2 Hz, Ar-H), 8.59 (1H, d, *J* = 0.6 Hz, Ar-H), 9.62 (1H, dd, *J* = 4.8, 1.2 Hz, Ar-H). ¹³C NMR (100 MHz, CDCl₃): δ (ppm) 50.85, 52.72, 57.74, 59.35, 123.65, 125.81, 127.66, 127.82, 128.73, 129.14, 129.44, 130.68, 130.77, 132.19, 132.51, 133.69, 134.21, 144.90, 157.26, 181.43 (CO). HRMS (ESI) calcd for C₂₂H₂₀N₃O₂SCl [M]⁺ 425.0965; found [M+H]⁺ 426.1024.

### Example 10

### 6-(4-Benzylpiperazin-1-yl)-10-chloro-12H-thiochromeno[2,3-c]q uinolin-12-one (11)

Product **11** was prepared from **3** and 4-benzylpiperazine. The yellow solid material was isolated in 81 % yield (*R_{f}* = 0.43 at EA: n-hexane = 1: 4). Mp 191-193 °C. ¹H NMR (400 MHz, CDCl₃): δ (ppm) 2.78 (4H, br, -CH₂N-), 3.43 (4H, t, *J* = 4.85 Hz, - NCH₂-), 3.68 (2H, s, -CH₂-), 7.27-7.42 (5H, m, Ar'-H), 7.61-7.67 (3H, m, Ar-H), 7.71 (1H, td, *J* = 7.6, 1.6 Hz, Ar-H), 8.00 (1H, dd, *J* = 8.4, 1.2 Hz, Ar-H), 8.5 8 (1H, d, *J* = 2.0 Hz, Ar-H), 9.61 (1H, dd, *J* = 8.4, 1.2 Hz, Ar-H). ¹³C NMR (100 MHz, CDCl₃): δ (ppm) 50.94, 52.96, 63.08, 123.57, 125.78, 127.18, 127.48, 127.83, 128.33, 128.73, 129.09, 129.17, 129.34, 130.58, 130.90, 132.18, 132.44, 133.60, 134.36, 138.11, 144.95, 157.48, 181.47 (CO). HRMS (ESI) calcd for C₂₇H₂₂N₃OSCl [M]⁺ 471.1172; found [M+H]⁺ 472.1241.

### Example 11

### 10-Chloro-6-(4-phenylpiperazin-1-yl)-12H-thiochromeno[2,3-c] quinolin-12-one (12)

Product **12** was prepared from **3** and 1-phenylpiperazine. The yellow solid material was isolated in 77% yield (*R_{f}* = 0.73 at EA: n-hexane = 1: 4). Mp 236-237 °C. ¹H NMR (300 MHz, CDCl₃): δ (ppm) 3.50-3.60 (8H, m, -CH₂-), 6.94 (1H, t, *J* = 7.2 Hz, Ar'-H), 7.04 (2H, d, *J* = 8.4 Hz, Ar'-H), 7.33 (2H, *t, J* = 7.5 Hz, Ar'-H), 7.63-7.67 (3H, m, Ar-H), 7.71 (1H, t, *J* = 7.2 Hz, Ar-H), 8.02 (1H, d, *J* = 7.2 Hz, Ar-H), 8.58 (1H, s, Ar-H), 9.63 (1H, d, *J* = 8.1 Hz, Ar-H). ¹³C NMR (75 MHz, CDCl₃): δ (ppm) 48.60, 50.33, 115.77,119.51, 123.21, 125.38, 127.14, 127.30, 128.27, 128.65, 128.71, 128.88, 130.23, 131.77, 131.97, 133.22, 133.70, 144.52, 150.90, 156.83, 159.91, 180.91 (CO). HRMS (ESI) calcd for C₂₆H₂₀N₃OSCl [M]⁺ 457.1016; found [M+H]⁺ 458.1095.

### Example 12

### 10-Chloro-6-morpholino-12H-thiochromeno[2,3-c]quinolin-12-one (13)

Product **13** was prepared from **3** and morpholine. The yellow solid material was isolated in 70% yield (*R_{f}* = 0.42 at CH₂Cl₂). Mp 217-218 °C. ¹H NMR (300 MHz, CDCl₃): δ (ppm) 3.41 (4H, t, J = 4.5 Hz, -NCH₂-), 4.02 (4H, t, *J* = 4.5 Hz, -CH₂O-), 7.62-7.70 (3H, m, Ar-H), 7.73 (1H, td, *J* = 7.5, 1.5 Hz, Ar-H), 8.03 (1H, dd, *J* = 8.4, 1.5 Hz, Ar-H), 8.59 (1H, dd, *J* = 2.1, 0.6 Hz, Ar-H), 9.35 (1H, dd, *J* = 8.7, 1.8 Hz, Ar-H). ¹³C NMR (75 MHz, CDCl₃): δ (ppm) 51.36, 66.88, 123.92, 126.06, 127.85, 127.91, 128.94, 129.34, 129.52, 130.71, 131.05, 132.50, 132.61, 133.95, 134.29, 145.23, 157.29, 181.51 (CO). HRMS (ESI) calcd for C₂₀H₁₅N₂O₂SCl [M]⁺ 382.8633; found [M+H]⁺ 383.0620.

### Example 13

### 10-Chloro-6-thiomorpholino-12H thiochromeno[2,3-c]quinolin-12-one (14)

Product **14** was prepared from **3** and thiomorpholine. The yellow solid material was isolated in 86% yield (*R_{f}* = 0.77 at EA: n-hexane = 1: 4). Mp 219-220 °C. ¹H NMR (300 MHz, CDCl₃): δ (ppm) 2.98 (4H, t, *J* = 4.8 Hz, -NCH₂-), 3.64 (4H, t, *J* = 5.1 Hz, -SCH₂-), 7.62-7.84 (3H, m, Ar-H), 7.73 (1H, td, *J* = 8.4, 1.8 Hz, Ar-H), 8.06 (1H, dd, *J* = 8.1, 1.5 Hz, Ar-H), 8.57 (1H, dd, *J* = 1.8, 0.6 Hz, Ar-H), 9.61 (1H, dd, *J* = 7.8, 1.2 Hz, Ar-H). ¹³C NMR (75 MHz, CDCl₃): δ (ppm) 27.06, 52.64, 114.95, 123.23, 125.40, 127.27, 127.31, 128.30, 128.68, 128.87, 130.55, 131.81, 131.96, 133.29, 133.70, 144.93, 157.43, 180.86 (CO). HRMS (ESI) m/z calcd for C₂₀H₁₅N₂S₂OCl⁺ [M]⁺ 398.0314, found [M+H]⁺ 399.0420, [M+H+2]⁺ 401.0394.

### Example 14

### 10-Chloro-6-(piperidin-1-yl)-12H-thiochromeno[2,3-c]quinolin-12-one (15)

Product **15** was prepared from **3** and piperidine. The yellow solid material was isolated in 86% yield (*R_{f}* = 0.75 at EA). Mp 187-188 °C. ¹H NMR (400 MHz, CDCl₃): δ (ppm) 1.71-1.74 (2H, m, -CH₂-), 1.88 (4H, p, *J* = 4.5 Hz, -CH₂-), 3.31 (4H, t, *J* = 4.2 Hz, -NCH₂-), 7.60-7.64 (2H, m, Ar-H), 7.61 (1H, d, *J* = 6.3 Hz, Ar-H), 7.69 (1H, td, *J* = 5.1, 1.2 Hz, Ar-H), 7.99 (1H, dd, *J* = 5.4, 0.6 Hz, Ar-H), 8.58 (1H, d, *J* = 1.5 Hz, Ar-H), 9.62 (1H, dd, *J* = 6.3, 0.6 Hz, Ar-H) ¹³C NMR (100 MHz, CDCl₃): δ (ppm) 24.27, 25.89, 52.33, 123.47, 125.75, 127.28, 127.85, 128.56, 129.02, 129.23, 130.48, 131.62, 132.16, 132.33, 133.46, 134.67, 144.97, 158.50, 181.51 (CO). HRMS (ESI) calcd for C₂₁H₁₇N₂OSCl [M]⁺ 380.0750; found [M+H]⁺ 381.0816.

### Example 15

### 10-Chloro-6-(4-hydroxypiperidin-1-yl)-12H-thiochromeno[2,3-c ]quinolin-12-one (16)

Product **16** was prepared from **3** and 4-hydroxypiperidine. The gray-yellow solid material was isolated in 84% yield (*R_{f}* = 0.4 at EA: n-hexane = 1: 1). Mp 224-225 °C. ¹H NMR (400 MHz, CDCl₃): δ (ppm) 1.89 (1H, td, *J* = 7.2, 2.7 Hz, piperidine-CHₐ), 1.94 (1H, td, *J* = 6.9, 2.7 Hz, piperidine-CHₐ), 2.15-2.21 (2H, m, piperidine-CHₑ), 3.19 (2H, td, *J* = 8.4, 2.1 Hz, piperidine-NCHₐ), 3.60-3.65 (2H, m, piperidine-NCHₑ), 4.01 (1H, sext, *J* = 3.0 Hz, piperidine-CH), 7.61-7.67 (3H, m, Ar-H), 7.71 (1H, td, *J* = 6.3, 1.2 Hz, Ar-H), 7.99 (1H, dd, *J* = 6.3, 0.6 Hz, Ar-H), 8.59 (1H, dd, *J* = 1.5, 0.6 Hz, Ar-H), 9.64 (1H, dd, *J* = 6.3, 0.6 Hz, Ar-H). ¹³C NMR (100 MHz, CDCl₃): δ (ppm) 34.49, 48.90, 67.80, 123.62, 125.82, 127.54, 127.88, 128.64, 129.11, 129.36, 130.61, 131.29, 132.20, 132.47, 133.63, 134.43, 144.91, 157.83, 181.47 (CO). HRMS (ESI) calcd for C₂₁H₁₇N₂OSCl [M]⁺ 396.0699; found [M+H]⁺ 397.0757.

### Example 16

### 6-(4-Benzylpiperidin-1-yl)-10-chloro-12H-thiochromeno[2,3-c]q uinolin-12-one (17)

Product **17** was prepared from **3** and 4-benzylpiperidine. The yellow solid material was isolated in 90% yield (*R_{f}* = 0.57 at CH₂Cl₂: n-hexane = 2: 1). Mp 184-185 °C. ¹H NMR (400 MHz, CDCl₃): δ (ppm) 1.67 (2H, td, *J* = 9.3, 3.0 Hz, -CH₂-), 1.79-1.89 (1H, m, -CH-), 1.88 (2H, d, *J* = 6.9 Hz, piperidine-CH₂), 2.71 (2H, d, *J* = 5.1 Hz, piperidine-CH₂), 3.00 (2H, td, *J* = 9.3, 1.2 Hz, -NCH₂-), 3.65 (2H, d, *J* = 9.3 Hz, -NCH₂-), 7.20-7.25 (3H, m, Ar-H), 7.31-7.33 (2H, m, Ar-H), 7.60-7.72 (4H, m, Ar-H), 7.98 (1H, dd, *J* = 6.3, 0.6 Hz, Ar-H), 8.59 (1H, d, *J* = 1.8 Hz, Ar-H), 9.62 (1H, dd, *J* = 6.6, 0.6 Hz, Ar-H). ¹³C NMR (100 MHz, CDCl₃): δ (ppm) 32.21, 37.88, 43.30, 51.64, 123.52, 125.79, 125.99, 127.36, 127.87, 128.61, 129.09, 129.17, 129.29, 130.55, 131.49, 132.22, 132.41, 133.54, 134.62, 140.46, 144.98, 147.04, 158.25, 181.55 (CO). HRMS (ESI) calcd for C₂₈H₂₃N₂OSCl [M]⁺ 471.0130; found [M+H]⁺ 471.1276.

### Example 17

### 6-([1,4'-Bipiperidin]-1'-yl)-10-chloro-12H-thiochromeno[2,3-c]q uinolin-12-one (18)

Product 1**8** was prepared from **3** and 1,4'-bipiperidine. The yellow solid material was isolated in 92% yield (*R_{f}* = 0.15 at EA: MeOH= 5: 1). Mp 187-189 °C. ¹H NMR (400 MHz, CDCl₃): δ (ppm) 1.50-1.52 (2H, m, piperidine-H), 1.66-1.67 (3H, m, piperidine-H), 1.86-1.98 (2H, qd, *J* = 12.4, 2.8 Hz, piperidine-H), 2.06 (2H, d, *J* = 11.6 Hz, piperidine-H), 2.54 (1H, t, *J* = 10.8 Hz, piperidine-H), 2.65 (3H, br, piperidine-H), 3.05 (2H, t, *J* = 12 Hz, piperidine-H), 3.73 (2H, d, *J* = 12.8 Hz, piperidine-H), 7.60-7.66 (3H, m, Ar-H), 7.70 (1H, td, *J* = 8.0, 1.2 Hz, Ar-H), 7.98 (1H, d, *J* = 8.0 Hz, Ar-H), 8.58 (1H, s, Ar-H), 9.62 (1H, d, *J* = 8.8 Hz, Ar-H). ¹³C NMR (100 MHz, CDCl₃): δ (ppm) 24.79, 26.36, 28.24, 50.45, 51.16, 62.40, 123.57, 125.80, 127.42, 127.86, 128.61, 129.08, 129.30, 130.52, 131.41, 132.18, 132.43, 133.56, 134.54, 144.92, 157.92, 181.47 (CO). HRMS (ESI) calcd for C₂₆H₂₆N₃OSCl [M]⁺ 463.1485; found [M+H]⁺ 464.1593.

### Example 18

### 10-Chloro-6-(4-(3-(piperidin-4-yl)propyl)piperidin-1yl)-12H-thi ochromeno[2,3-c]quinolin-12-one (19)

Product 1**9** was prepared from **3** and 1,3-di(piperidin-4-yl)propane. The yellow solid material was isolated in 76% yield (*R_{f}* = 0.13 at CH₂Cl₂). Mp 164-165 °C. ¹H NMR (400 MHz, CDCl₃): δ (ppm) 1.16 (2H, qd, *J* = 11.6, 3.2 Hz, -CH₂-), 1.20-1.28 (2H, m, -CH₂-), 1.36-1.39 (4H, m, -CH₂-), 1.51-1.58 (4H, m, -CH₂-), 1.70 (2H, d, *J* = 13.6 Hz, -CH₂-), 1.87 (2H, d, *J* = 9.6 Hz, -CH2-), 2.43 (1H, br, -NH), 2.60 (2H, td, *J* = 12.0, 2.0 Hz, -CH₂-), 2.99 (2H, t, *J* = 11.2 Hz, -CH₂-), 3.10 (2H, d, *J* = 12 Hz, -CH₂-), 3.64 (2H, d, *J* = 12.4 Hz, -CH₂-), 7.59-7.65 (3H, m, Ar-H), 7.68 (1H, td, *J* = 8.0, 1.2 Hz, Ar-H), 8.00 (1H, dd, *J* = 11.2, 1.2 Hz, Ar-H), 8.57 (1H, d, *J* = 1.6 Hz, Ar-H), 9.61 (1H, d, *J* = 8.4 Hz, Ar-H). ¹³C NMR (100 MHz, CDCl₃): δ (ppm) 23.66, 32.40, 33.24, 35.73, 36.08, 36.86, 37.38, 46.58, 51.76, 123.49, 125.79, 127.30, 127.84, 128.57, 129.05, 129.26, 130.49, 131.55, 132.17, 132.36, 133.50, 134.64, 144.98, 158.34, 181.50 (CO). HRMS (ESI) calcd for C₂₉H₃₂N₃OSCl [M]⁺ 505.1955; found [M+H]⁺ 506.2004.

### Example 19

### 10-Chloro-6-(pyrrolidin-1-yl)-12H-thiochromeno[2,3-c]quinolin -12-one (20)

Product **20** was prepared from **3** and pyrrolidine. The solid material was isolated in 86% yield (*R_{f}* = 0.56 at CH₂Cl₂: n-hexane = 1: 1). Mp 170-171 °C. ¹H NMR (300 MHz, CDCl₃): δ (ppm) 2.05 (4H, quin, *J* = 3.6 Hz, -CH₂-), 3.76 (4H, t, *J* = 6.9 Hz, -NCH₂-), 7.50(1H, td, *J* = 7.2, 1.5 Hz, Ar-H), 7.61 (1H, d, *J* = 1.5 Hz, Ar-H₁₁), 7.65 (1H, td, *J* = 7.5, 1.5 Hz, Ar-H), 7.88 (1H, dd, *J* = 8.4, 1.5 Hz, Ar-H), 8.54 (1H, t, *J* = 1.5 Hz, Ar-H), 9.44 (1H, dd, *J* = 8.7, 1.5 Hz, Ar-H). ¹³C NMR (75 MHz, CDCl₃): δ (ppm) 24.89, 50.52, 121.90, 124.97, 125.17, 126.92, 127.33, 128.42, 128.84, 130.09, 131.77, 131.92, 133.10, 133.32, 144.73, 154.62, 159.91, 181.07 (CO). HRMS (ESI) calcd for C₂₀H₁₅N₂OSCl [M]⁺ 366.0594; found [M+H]⁺ 367.0659.

### Example 20

### 10-chloro-6-(2-oxopiperidin-1-yl)-12H-thiochromeno[2,3-c]quin olin-12-one (TC SCl-B-18) (21)

Product **21** was a yellow solid material which was isolated in 89% yield. Mp: 258-261 °C.¹H NMR (400 MHz, CDCl₃): δ ppm. 1.25 (1H, d, *J* = 4.8 Hz, piperidone-H), 2.44 (2H, quin, -CH₂-), 2.61 (1H, s, piperidone-H), 2.75 (2H, t, *J* = 8.4 Hz, -CH₂-), 4.11-4.14 (2H, m, -CH₂-), 7.60-7.67 (2H, m, Ar-H), 7.78-7.81 (2H, m, Ar-H), 8.09-8.12 (1H, m, Ar-H), 8.60 (1H, d, *J* = 2.0 Hz, Ar-H), 9.72-9.75 (1H, m, Ar-H). ¹³C NMR (100 MHz, CDCl₃): δ ppm. 19.37, 31.47, 41.05, 49.14, 125.25, 126.08, 127.65, 129.29, 129.51, 129.80, 129.88, 130.74, 131.96, 132.26, 132.74, 133.47, 133.91, 145.05, 148.08, 176.14, 181.01.

### Example 21

### 10-Chloro-6-methylamino-12H-thiochromeno[2,3-c]quinolin-12 -one (N1)

Product **N1** was prepared from **3** and methylamine. The pure compound was obtained as a yellow solid (yield 92%) (*R_{f}* = 0.65 at CH₂Cl₂). Mp 237-238 °C (MeOH). ¹H NMR (400 MHz, CDCl₃): δ (ppm) 3.26 (3H, d, *J* = 4.8 Hz, -CH₃), 4.92 (1H, d, *J* = 4.8 Hz, -NH-), 7.45 (1H, td, *J* = 11.2, 1.6 Hz, Ar-H), 7.58 (1H, d, *J* = 8.4 Hz, Ar-H), 7.69-7.65 (2H, m, Ar-H), 7.86 (1H, dd, *J* = 8.4, 0.8 Hz, Ar-H), 8.56 (1H, d, *J* = 1.6 Hz, Ar-H), 9.45 (1H, dd, *J* = 8.4, 1.2 Hz, Ar-H). ¹³C NMR (100 MHz, CDCl₃): δ (ppm) 29.38, 120.73, 123.68, 124.65, 125.87, 127.18, 127.49, 129.38, 129.51, 129.62, 131.04, 132.50, 132.53, 134.14, 145.64, 151.21, 180.96 (CO). HRMS (ESI) m/z calcd for C₁₇H₁₁N₂OSCl [M]⁺: 326.0281, found [M+H]⁺: 327.0356.

### Example 22

### 10-Chloro-6-ethylamino-12H-thiochromeno[2,3-c]quinolin-12-one (N2)

Product **N2** was prepared from **3** and ethylamine. The pure compound was obtained as a yellow solid (yield 91%) (*R_{f}* = 0.75 at CH₂Cl₂). Mp 204-205 °C (MeOH). ¹H NMR (400 MHz, CDCl₃): δ (ppm) 1.41 (3H, t, *J* = 7.2 Hz, -CH₃), 3.75 (2H, q, *J* = 1.6 Hz, -CH₂-), 4.81 (1H, br, -NH-), 7.44 (1H, td, *J* = 8.4, 1.6 Hz, Ar-H), 7.58-7.64 (3H, m, Ar-H), 7.83 (1H, d, *J* = 8.4 Hz, Ar-H), 8.56 (1H, d, *J* = 1.6 Hz, Ar-H), 9.44 (1H, d, *J* = 8.4 Hz, Ar-H). ¹³C NMR (100 MHz, CDCl₃): δ (ppm) 14.79, 37.28, 120.70, 123.50, 124.58, 125.84, 127.18, 127.47, 129.39, 129.47, 129.66, 131.08, 132.50, 132.53, 134.12, 145.67, 150.55, 181.00 (CO). HRMS (ESI) m/z calcd for C₁₈H₁₃N₂OSCl [M]⁺: 340.0437, found [M+H]⁺: 341.0493.

### Example 23

### 10-Chloro-6 propylamino-12H-thiochromeno[2,3-c]quinolin-1 2-one (N3)

Product **N3** was prepared from **3** and propylamine. The pure compound was obtained as a yellow solid (yield 85%) (*R_{f}* = 0.82 at CH₂Cl₂). Mp 178-179 °C (MeOH). ¹H NMR (400 MHz, CDCl₃): δ (ppm) 1.09 (3H, t, *J* = 7.2 Hz, -CH₃), 1.81 (2H, sext, *J* = 7.2 Hz, -CH₂-), 3.69 (2H, q, *J* = 7.2 Hz, -NCH₂-), 4.87 (1H, br, -NH-), 7.44 (1H, td, *J* = 8.0, 1.2 Hz, Ar-H), 7.58-7.64 (3H,m, Ar-H), 7.82 (1H, d, *J* = 8.0 Hz, Ar-H), 8.56 (1H,d, *J* = 1.2 Hz, Ar-H), 9.44 (1H, d, *J* = 8.8 Hz, Ar-H). ¹³C NMR (100 MHz, CDCl₃): δ (ppm) 11.68, 22.64, 44.13, 120.67, 123.53, 124.53, 125.82, 127.15, 127.46, 129.38, 129.46, 129.65, 131.05, 132.49, 134.10, 145.66, 150.62, 181.02 (CO). HRMS (ESI) m/z calcd for C₁₉H₁₅N₂OSCl [M]⁺: 354.0594, found [M+H]⁺: 355.0651.

### Example 24

### 6-(Butylamino)-10-chloro-12H-thiochromeno[2,3-c]quinolin-1 2-one (N4)

Product **N4** was prepared from **3** and butylamine. The pure compound was obtained as a yellow solid (yield 91%) (*R_{f}* = 0.85 at CH₂Cl₂). Mp 147-149 °C (MeOH). ¹H NMR (400 MHz, CDCl₃): δ (ppm) 1.03 (3H, t, *J* = 7.2 Hz,-CH₃), 1.53 (2H, sext, *J* = 7.2 Hz, -CH₂-), 1.76 (2H, quin, *J* = 7.2 Hz, -CH₂- ), 3.71 (2H, q, *J* = 6.8 Hz, -NCH₂-), 4.83 (1H, br, -NH-), 7.43 (1H, td, *J* = 7.6, 1.2 Hz, Ar-H), 7.57-7.64 (3H, m, Ar-H), 7.82 (1H, d, *J* = 8.4 Hz, Ar-H), 8.55 (1H, d, *J* = 1.6 Hz, Ar-H), 9.43 (1H, d, *J* = 8.4 Hz, Ar-H). ¹³C NMR (100 MHz, CDCl₃): δ (ppm) 13.97, 20.36, 31.54, 42.12, 120.67, 123.53, 124.52, 125.84, 127.16, 127.46, 129.38, 129.46, 129.65, 131.05, 132.48, 132.52, 134.11, 145.68, 150.62, 181.00 (CO). HRMS (ESI) m/z calcd for C₂₀H₁₇N₂OSCl [M]⁺: 368.0750, found [M+H]⁺: 369.0846.

### Example 25

### 10-Chloro-6-isobutylamino-12H-thiochromeno[2,3-c]quinolin-12-one (N5)

Product **N5** was prepared from **3** and isobutylamine. The pure compound was obtained as a yellow crystal (yield 61%) (*R_{f}* = 0.85 at CH₂Cl₂). Mp 159-160 °C (MeOH). ¹H NMR (400 MHz, CDCl₃): δ (ppm) 1.08 (6H, d, *J* = 6.8 Hz,-CH₃), 2.10 (1H, sep, *J* = 6.8 Hz,-CH₋), 3.56 (2H, *t, J* = 6.4 Hz, -CH₂₋), 4.94 (1H, br, -NH), 7.44(1H, t, *J* = 7.2 Hz, Ar-H), 7.59-7.64 (3H, m, Ar-H), 7.82 (1H, d, *J* = 8.4 Hz, Ar-H), 8.57 (1H, dd, *J* = 2.0, 0.8 Hz, Ar-H), 9.43 (1H, d, *J* = 8.4 Hz, Ar-H). ¹³C NMR (100 MHz, CDCl₃): δ (ppm) 20.50, 28.16, 49.72, 120.67, 123.54, 124.51, 125.82, 127.13, 127.47, 129.39, 129.47, 129.69, 131.02, 132.50, 134.11, 138.34, 145.62, 150.68, 181.02 (CO). HRMS (ESI) m/z calcd for C₂₀H₁₇N₂OSCl [M]⁺: 368.0750, found [M+H]⁺: 369.0825.

### Example 26

### 10-Chloro-6-(pentan-3-ylamino)-12H-thiochromeno[2,3-c]quin olin-12-one (N6)

Product **N6** was prepared from **3** and 3-aminopentane. The pure compound was obtained as a light yellow crystal (yield 65%) (*R_{f}* = 0.87 at CH₂Cl₂). Mp 160-161 °C (MeOH). ¹H NMR (400 MHz, CDCl₃): δ (ppm) 0.92 (6H, t, *J* = 7.6 Hz, -CH₃), 1.69 (4H, quin, *J* = 6.0 Hz, -CH₂-), 4.34 (1H, sext, *J* = 7.2 Hz, -CH-), 6.58 (1H, d, *J* = 8.0 Hz, Ar-H), 7.36(1H, t, *J* = 8.0 Hz, Ar-H), 7.58 (1H, t, *J* = 8.0 Hz, Ar-H), 7.65 (1H, d, *J* = 8.0 Hz, Ar-H), 7.90 (1H, dd, *J* = 8.4, 2.4 Hz, Ar-H), 8.01 (1H, d, *J* = 8.4 Hz, Ar-H), 8.40 (1H, d, *J* = 2.4 Hz, Ar-H), 9.34 (1H, d, *J* = 8.8 Hz, Ar-H). ¹³C NMR (100 MHz, CDCl₃): δ (ppm) 11.21, 26.86, 54.41, 120.16, 123.92, 125.13, 125.81, 127.05, 128.49, 129.02, 129.37, 129.73, 132.17, 132.55, 133.22, 133.29, 145.69, 151.64, 180.85 (CO). HRMS (ESI) m/z calcd for C₂₁H₁₉N₂OSCl [M]⁺: 382.0907, found [M+H]⁺: 383.0994, [M-H]⁻:381.0851.

### Example 27

### 10-Chloro-6-((2-(dimethylamino)ethyl)amino)-12H-thiochrome no[2,3-c]quinolin-12-one (N7)

Product **N7** was prepared from **3** and *N,N-*dimethylethylenediamine. The pure compound was obtained as a yellow crystal (yield 76%) (*R_{f}* = 0.82 at EA: MeOH: ammonia water= 10:5:1). Mp 156-157 °C (MeOH). ¹H NMR (400 MHz, CDCl₃): δ (ppm) 2.36 (6H, s, -N(CH₃)₂), 2.69 (2H, t, *J* = 6.0 Hz, -CH₂N-), 3.57 (2H, q, *J* = 5.6 Hz, -NCH₂-), 5.86 (1H, br, -NH), 7.44 (1H, td, *J* = 8.0, 1.6 Hz, Ar-H), 7.62 (2H, td, *J* = 7.6, 1.6 Hz, Ar-H), 7.65 (1H, dd, *J* = 8.0, 0.8 Hz, Ar-H), 7.82 (1H, dd, *J* = 8.4, 1.2 Hz, Ar-H), 8.58 (1H, dd, *J* = 1.6, 0.4 Hz, Ar-H), 9.46 (1H, dd, *J* = 8.8, 1.2 Hz, Ar-H). ¹³C NMR (100 MHz, CDCl₃): δ (ppm) 39.53, 45.28, 57.57, 120.71, 123.46, 125.90, 127.02, 127.27, 127.57, 129.35, 129.43, 129.56, 131.46, 132.43, 132.56, 134.02, 145.72, 150.90, 181.05. (CO). HRMS (ESI) m/z calcd for C₂₀H₁₈N₃OSCl [M]⁺: 383.0859, found [M+H]⁺: 384.0925.

### Example 28

### 10-Chloro-6-((2-(diethylamino)ethyl)amino)-12H-thiochromen o[2,3-c]quinolin-12-one (N8)

Product **N8** was prepared from **3** and *N,N-*diethylethylenediamine. The pure compound was obtained as a yellow crystal (yield 86%) (*R_{f}* = 0.8 at EA: MeOH: ammonia water= 10:5:1). Mp 152-153 °C (MeOH). ¹H NMR (400 MHz, CDCl₃): δ (ppm) 1.13 (6H, t, *J* = 7.2 Hz,-CH₃), 2.64 (4H, q, *J* = 6.8 Hz,-NCH₂-), 2.82 (2H, t, *J* = 6.0 Hz,-CH₂N-), 3.70 (2H, q, *J* = 5.2 Hz, -NCH₂-), 6.08 (1H, br, -NH-), 7.43(1H, t, *J* = 7.2 Hz, Ar-H), 7.59-7.64 (3H, m, Ar-H), 7.81 (1H, d, *J* = 8.4 Hz, Ar-H), 8.57 (1H, d, *J* = 1.2 Hz, Ar-H), 9.45 (1H, d, *J* = 8.4 Hz, Ar-H). ¹³C NMR (100 MHz, CDCl₃): δ (ppm) 12.34, 39.52, 46.81, 50.89, 120.64, 124.25, 124.34, 125.89, 126.96, 127.63, 129.32, 129.41, 129.53, 131.49, 132.37, 132.55, 133.98, 145.79, 150.96, 181.06 (CO). HRMS (ESI) m/z calcd for C₂₂H₂₂N₃OSCl [M]⁺: 411.1172, found [M+H]⁺: 412.1262.

### Example 29

### 10-Chloro-6-(2-ethanolamino)-12H-thiochromeno[2,3-c]quinol in-12-one (N9)

Product **N9** was prepared from **3** and ethanolamine. The pure compound was obtained as a yellow crystal (yield 77%) (*R_{f}* = 0.65 at EA). Mp 190-192 °C (MeOH). ¹H NMR (400 MHz, CDCl₃): δ (ppm) 3.93 (2H, q, *J* = 4.4 Hz, -NCH₂-), 4.00 (2H, t, *J* = 4.4 Hz, -CH₂O-), 4.23 (1H, br, -OH), 5.45 (1H, br, -NH), 7.48 (1H, td, *J* = 8.0, 1.6 Hz, Ar-H), 7.62-7.68 (3H, m, Ar-H),7.81 (1H, dd, *J* =7.6, 0.8 Hz, Ar-H), 8.58 (1H, dd, *J* = 1.6, 0.4 Hz, Ar-H), 9.45 (1H, dd, *J* = 8.4, 0.8 Hz, Ar-H). ¹³C NMR (100 MHz, CDCl₃): δ (ppm) 45.88, 63.59, 120.97, 123.67, 125.12, 125.92, 126.60, 127.50, 129.48, 129.83, 130.15, 130.91, 132.55, 132.69, 134.35, 144.65, 151.32, 180.87 (CO). HRMS (ESI) m/z calcd for C₁₈H₁₃N₂O₂SCl [M]⁺: 356.8260, found [M+H]⁺: 357.0476, [M+H+2]⁺: 359.0455.

### Example 30

### 10-Chloro-6-(3-propanolamino)-12H-thiochromeno[2,3-c]quin olin-12-one (N10)

Product **N10** was prepared from **3** and 3-amino-1-propanol. The pure compound was obtained as a yellow solid (yield 94%) (*R_{f}* = 0.66 at EA). Mp 201-202 °C (MeOH). ¹H NMR (400 MHz, CDCl₃): δ (ppm) 1.94 (2H, p, *J* = 6.0 Hz, -CH₂-), 3.72 (2H, t, *J* = 5.2 Hz, -NCH₂-), 3.93 (2H, q, *J* = 6.0 Hz, -CH₂O-), 4.41 (1H, br, -OH), 5.38 (1H, t, *J* = 5.2 Hz, -NH-), 7.45 (1H, td, *J* = 7.6, 1.2 Hz, Ar-H), 7.58-7.65 (3H, m, Ar-H), 7.78 (1H, dd, *J* = 8.4, 0.8 Hz, Ar-H), 8.56 (1H, dd, *J* = 2.0, 0.4 Hz, Ar-H), 9.42 (1H, dd, *J* = 8.4, 1.2 Hz, Ar-H). ¹³C NMR (100 MHz, CDCl₃): δ (ppm) 33.23, 38.94, 59.25, 120.72, 123.44, 124.84, 125.94, 126.32, 127.45, 129.44, 129.92, 130.11, 130.84, 132.49, 132.64, 134.31, 144.83, 151.33, 180.88 (CO). HRMS (ESI) m/z calcd for C₁₉H₁₅N₂O₂SCl [M]⁺: 370.0543, found [M+H]⁺: 371.0622.

### Example 31

### 10-Chloro-6-(5-pentanolamino)-12H-thiochromeno[2,3-c]quin olin-12-one (N11)

Product **N11** was prepared from **3** and 5-amino-1-pentanol. The pure compound was obtained as a yellow solid (yield 91%) (*R_{f}* = 0.7 at EA). Mp 158-160 °C (MeOH). ¹H NMR (400 MHz, CDCl₃): δ (ppm) 1.40 (1H, br, -OH),1.49-1.62 (2H, m, -CH₂-), 1.71 (2H, quin, -CH₂-), 1.83 (2H, quin, -CH₂-), 3.74 (4H, quin, -CH₂-), 4.91 (1H, br, -NH), 7.45(1H, td, *J* = 7.6, 1.2 Hz, Ar-H), 7. 59 (3H, m, Ar-H), 7.83 (1H, d, *J* = 8.4 Hz, Ar-H), 8.57 (1H, d, *J* = 1.2 Hz, Ar-H), 9.44 (1H, d, *J* = 8.4 Hz, Ar-H). ¹³C NMR (100 MHz, CDCl₃): δ (ppm) 23.39, 29.17, 32.40, 42.24, 62.83, 120.69, 123.53, 124.60, 125.84, 127.11, 127.48, 129.40, 129.51, 129.71, 131.02, 131.79, 132.53, 134.14, 145.60, 150.58, 181.02 (CO). HRMS (ESI) m/z calcd for C₂₁H₁₉N₂O₂SCl [M]⁺: 398.0856, found [M+H]⁺: 399.0914.

### Example 32

### 10-Chloro-6-((1-hydroxybutan-2yl)amino)-12H-thiochromeno[ 2,3-c]quinolin-12-one (N12)

Product **N12** was prepared from **3** and 2-amino-1-butanol. The pure compound was obtained as a yellow solid (yield 94%) (*R_{f}* = 0.8 at EA). Mp 203-204 °C (MeOH). ¹H NMR (400 MHz, CDCl₃): δ (ppm) 1.21 (3H, t, *J* = 7.6 Hz, -CH₃), 1.71-1.88 (2H, m, -CH₂-), 3.79 (1H, dd, *J* = 11.2, 1.6 Hz, -CH₂-), 3.99 (1H, dd, *J* = 11.2, 2.8 Hz, -CH₂-), 4.34 (1H, quin, *J* = 11.2 Hz, -NCH-), 4.59 (1H, br, -OH), 5.02 (1H, d, *J* = 6.0 Hz, -NH-), 7.44(1H, td, *J* = 8.0, 1.2 Hz, Ar-H), 7.56 (1H, d, *J* = 8.4 Hz, Ar-H), 7.59 (1H, td, *J* = 7.6, 1.2 Hz, Ar-H), 7.61 (1H, d, *J* = 8.4 Hz, Ar-H), 7.74 (1H, d, *J* = 8.4 Hz, Ar-H), 8.52 (1H, d, *J* = 2.0 Hz, Ar-H), 9.40 (1H, dd, *J* = 7.6, 1.2 Hz, Ar-H). ¹³C NMR (100 MHz, CDCl₃): δ (ppm) 10.95, 24.95, 56.89, 67.08, 120.83, 123.68, 124.98, 125.88, 126.51, 127.40, 129.39, 129.75, 130.02, 130.81, 132.38, 132.62, 134.28, 144.53, 151.09, 180.75 (CO). HRMS (ESI) m/z calcd for C₂₀H₁₇N₂O₂SCl [M]⁺: 384.0699, found [M+H]⁺: 385.0790.

### Example 33

### 10-Chloro-6-((4-methylpentan-2yl)amino)-12H-thiochromeno[ 2,3-c]quinolin-12-one (N13)

Product **N13** was prepared from **3** and 4-methylpentan-2-amine.The pure compound was obtained as a yellow solid (yield 94%) (*R_{f}* = 0.9 at CH₂Cl₂). Mp 176-177 °C (MeOH). ¹H NMR (400 MHz, CDCl₃): δ (ppm) 0.98 (3H, d, *J* = 6.8 Hz, -CH₃), 1.03 (3H, d, *J* = 6.8 Hz, -CH₃), 1.35 (3H, d, *J* = 6.4 Hz, -CH₃), 1.45 (1H, quin, *J* = 6.4 Hz, -CH₂-), 1.66 (1H, quin, *J* = 6.8 Hz, -CH₂-), 1.80 (1H, sep, *J* = 6.8 Hz, -CH-), 4.63 (1H, br, -NH), 4.63-4.66 (1H, m, -CH-), 7.43 (1H, td, *J* = 7.6, 1.2 Hz, Ar-H), 7.58-7.63 (3H, m, Ar-H), 7.81 (1H, d, *J* = 8.4 Hz, Ar-H), 8.56 (1H, d, *J* = 1.2 Hz, Ar-H), 9.43 (1H, d, *J* = 8.4 Hz, Ar-H). ¹³C NMR (100 MHz, CDCl₃): δ (ppm) 21.38, 22.87, 22.92, 25.40, 45.73, 46.80, 120.55, 123.42, 124.36, 125.80, 127.21, 127.44, 129.38, 129.71, 131.08, 132.47, 132.52, 134.07, 145.76, 150.01, 181.05 (CO). HRMS (ESI) m/z calcd for C₂₂H₁₉N₂OSCl [M]⁺: 396.1063, found [M+H]⁺: 397.1142.

### Example 34

### 6-((2-Aminoethyl)amino)-10-chloro-12H-thiochromeno[2,3-c]q uinolin-12-one (N14)

Product **N14** was prepared from **3** and 1,2-diaminoethane. The pure compound was obtained as a yellow solid (yield 90%) (*R_{f}* = 0.6 at EA: MeOH: ammonia water = 10: 5: 1). Mp 193-194 °C (MeOH). ¹H NMR (400 MHz, DMSO-*d₆*): δ (ppm) 2.90 (2H, t, *J* = 6.0 Hz, -CH₂-), 3.59 (2H, t, *J* = 6.0 Hz, -CH₂-), 7.36 (1H, t, *J* = 8.0 Hz, Ar-H), 7.59(1H, t, *J* = 8.0 Hz, Ar-H), 7.66 (1H, d, *J* = 8.0 Hz, Ar-H), 7.85 (1H, d, *J* = 7.2 Hz, Ar-H), 7.96 (1H, d, *J* = 8.8 Hz, Ar-H), 8.35 (1H, br, Ar-H), 9.32 (1H, d, *J* = 8.4 Hz, Ar-H). ¹³C NMR (100 MHz, DMSO-*d₆*): δ (ppm) 40.79, 45.06, 120.24, 124.08, 125.31, 125.84, 127.00, 128.39, 128.83, 129.32, 129.69, 132.06, 132.33, 133.12, 133.26, 145.58, 151.52, 180.65 (CO). HRMS (ESI) m/z calcd for C₁₈H₁₄N₃OSCl [M]⁺: 355.0546, found [M+H]⁺: 356.0641.

### Example 35

### 10-Chloro-6-((2-((2-hydroxyethyl)amino)ethyl)amino)-12H-thio chromen[2,3-c]quinolin-12-one (N15)

Product **N15** was prepared from **3** and *N*-(2-hydroxyethyl)ethylenediamine. The pure compound was obtained as a yellow solid (yield 58%) *(R_{f} =* 0.63 at EA: MeOH: ammonia water = 10: 5: 1). Mp 141-143 °C (MeOH). ¹H NMR (400 MHz, DMSO-*d₆*): δ (ppm) 2.69 (2H, t, *J* = 5.6 Hz, -CH₂-), 2.90 (2H, t, *J* = 6.0 Hz, -CH₂-), 3.51 (2H, t, *J* = 5.6 Hz, -CH₂-), 3.65 (2H, t, *J* = 6.0 Hz, -CH₂-), 7.10 (1H, br, -NH-), 7.32 (1H, t, *J* = 7.2 Hz, Ar-H), 7.55 (1H, t, *J* = 7.2 Hz, Ar-H), 7.62 (1H, d, *J* = 8.4 Hz, Ar-H), 7.76 (1H, t, *J* = 7.2 Hz, Ar-H), 7.86 (1H, d, *J* = 8.4 Hz, Ar-H), 8.25 (1H, d, *J* = 2.0 Hz, Ar-H), 9.26 (1H, d, *J* = 8.4 Hz, Ar-H). ¹³C NMR (100 MHz, DMSO-*d₆*): δ (ppm) 41.90, 48.16, 51.80, 60.72, 120.13, 123.96, 125.14, 125.81, 126.94, 128.26, 128.65, 129.16, 129.60, 131.82, 132.11, 132.96, 133.19, 145.50, 151.35, 180.45 (CO). HRMS (ESI) m/z calcd for C₂₀H₁₈N₃O₂SCl [M]⁺: 399.8938, found [M+H]⁺: 400.0880.

### Example 36

### 10-Chloro-6-((2-morpholinoethyl)amino)-12H-thiochromeno[2, 3-c]quinolin-12-one (N16)

Product **N16** was prepared from **3** and 4-(2-aminoethyl)morpholine. The pure compound was obtained as a yellow solid (yield 87%) (*R_{f}* = 0.48 at EA). Mp 189-190 °C (MeOH). ¹H NMR (400 MHz, CDCl₃): δ (ppm) 2.63 (4H, br, -CH₂-), 2.81 (2H, br, -CH₂-), 3.81 (6H, br, -CH₂-), 5.92 (1H, br, -NH-), 6.70 (2H, d, *J* = 8.4 Hz, Ar'-H), 7.45 (1H, td, *J* = 7.8, 1.6 Hz, Ar-H), 7.60-7.64 (3 H, m, Ar-H), 7.81 (1H, d, *J* = 8.4 Hz, Ar-H), 8.57 (1H, s, Ar-H), 9.46 (1H, dd, *J* = 8.8, 1.2 Hz, Ar-H). ¹³C NMR (100 MHz, CDCl₃): δ (ppm) 38.28, 53.31, 56.52, 67.13, 120.73, 123.94, 124.58, 125.91, 127.01, 127.56, 129.37, 129.49, 129.64, 131.27, 132.47, 132.54, 134.10, 145.66, 150.76, 180.99 (CO). HRMS (ESI) m/z calcd for C₂₂H₂₀N₃O₂SCl [M]⁺: 425.0965, found [M+H]⁺: 426.1058, [M-H]⁻: 424.0885.

### Example 37

### 10-Chloro-6-((3-(dimethylamino)propyl)amino)-12H-thiochrom eno[2,3-c]quinolin-12-one (N17)

Product **N17** was prepared from **3** and 3-(dimethylamino)-1-propylamine. The pure compound was obtained as a yellow crystal (yield 43%) (*R_{f}* = 0.71 at EA: MeOH: ammonia water = 10: 5: 1). Mp 194-195 °C (MeOH). ¹H NMR (400 MHz, CDCl₃): δ (ppm) 1.92 (2H, quin, *J* =6.0 Hz, -CH₂-), 2.41 (6H, s, -N(CH₃)₂), 2.60 (2H, t, *J* = 5.6 Hz, -CH₂N-), 3.81 (2H, q, *J* = 5.6 Hz, -NCH₂-), 7.95 (1H, br, -NH), 7.40 (1H, td, *J* = 7.6, 1.6 Hz, Ar-H), 7.56-7.63 (4H, m, Ar-H), 7.80 (1H, d, *J* = 8.4 Hz, Ar-H), 8.57 (1H, d, *J* = 2.4 Hz, Ar-H), 9.44 (1H, dd, *J* = 7.6, 0.8 Hz, Ar-H). ¹³C NMR (100 MHz, CDCl₃): δ (ppm) 24.83, 43.64, 45.68, 59.72, 120.46, 123.97, 124.56, 125.84, 126.83, 127.54, 129.32, 129.48, 131.84, 132.29, 132.56, 133.86, 146.01, 151.27, 181.14 (CO). HRMS (ESI) m/z calcd for C₂₁H₂₀N₃OSCl [M]⁺: 397.1016, found [M+H]⁺: 398.1072.

### Example 38

### 10-Chloro-6-((3-(diethylamino)propyl)amino)-12H-thiochrome no[2,3-c]quinolin-12-one (N18)

Product **N18** was prepared from **3** and 3-(diethylamino)-1-propylamine. The pure compound was obtained as a yellow acicular crystal (yield 70%) (*R_{f}* = 0.68 at EA: MeOH: ammonia water = 10: 5: 1). Mp 142-143 °C (MeOH). ¹H NMR (400 MHz, CDCl₃): δ (ppm) 1.15 (6H, t, *J* = 6.8 Hz, -CH₃), 1.91 (2H, quin, *J* = 6.0 Hz, -CH₂-), 2.66-2.72 (6H, m, -NCH₂-), 3.81 (2H, q, *J =* 4.8 Hz, -NCH₂-), 7.40 (1H, td, *J* = 7.2, 1.2 Hz, Ar-H), 7.55-7.58 (1H, dd, *J* = 8.4, 3.6 Hz, Ar-H), 7.60-7.64 (2H, m, Ar-H), 7.81 (1H, d, *J* = 8.0 Hz, Ar-H), 7.93 (1H, br, Ar-H), 8.58 (1H,t, *J* = 2.0 Hz, Ar-H), 9.45 (1H, dd, *J* = 8.4, 0.8 Hz, Ar-H). ¹³C NMR (100 MHz, CDCl₃): δ (ppm) 11.44, 24.71, 44.25, 47.09, 53.57, 120.45, 123.99, 124.50, 125.86, 126.87, 127.46, 129.34, 129.43, 131.85, 132.32, 132.57, 133.88, 146.04, 151.30, 181.15 (CO). HRMS (ESI) m/z calcd for C₂₃H₂₄N₃SOCm [M]⁺: 425.1329, found [M+H]⁺: 426.1396, [M-H]⁻: 424.1284.

### Example 39

### 10-Chloro-6-((3-((2-hydroxyethyl)amino)propyl)amino)-12H-th iochromeno[2,3-c]quinolin-12-one (N19)

Product **N19** was prepared from **3** and *N*-(2-Hydroxyethyl)-1,3-diaminopropane. The pure compound was obtained as a brown solid (yield 75%) (*R_{f}* = 0.65 at EA: MeOH: ammonia water = 10: 5: 1). Mp 65-67 °C (MeOH). ¹H NMR (400 MHz, CDCl₃): δ (ppm) 1.89 (2H, quin, *J* = 6.0 Hz, -CH₂-), 2.15 (2H, br, -OH & -NH-), 2.85 (4H, quin, -CH₂-), 3.74 (2H, t, *J* = 6.0 Hz, -CH₂-), 3.80 (2H, t, *J* = 5.2 Hz, -CH₂-), 6.53 (1H, br, -NH-), 7.39 (1H, td, *J* = 7.6, 0.8 Hz, Ar-H), 7.44 (1H, d, *J* = 8.8 Hz, Ar-H), 7.50 (1H, dd, *J* = 8.4, 2.4 Hz, Ar-H), 7.58 (1H, td, *J* = 7.2, 1.2 Hz, Ar-H), 7.76 (1H, d, *J* = 8.0 Hz, Ar-H), 8.46 (1H, d, *J* = 2.0 Hz, Ar-H), 9.39 (1H, d, *J* = 8.4 Hz, Ar-H). ¹³C NMR (100 MHz, CDCl₃): δ (ppm) 28.37, 42.23, 48.74, 51.65, 61.42, 120.48, 123.99, 124.22, 125.85, 126.88, 127.42, 129.13, 129.33, 129.38, 131.23, 132.21, 132.33, 133.95, 145.69, 150.82, 180.92 (CO). HRMS (ESI) m/z calcd for C₂₁H₂₀N₃O₂SCl [M]⁺: 413.0965, found [M+H]⁺: 414.1053, [M+H+2]⁺: 416.1037.

### Example 40

### 10-Chloro-6-((2,3-dihydro-1H-inden-2-yl)amino)-12H-thiochro meno[2,3-c]quinolin-12-one (N20)

Product **N20** was prepared from **3** and 2-aminoindane. The pure compound was obtained as a brown solid (yield 65%) (*R_{f}* = 0.7 at CH₂Cl₂: n-hexane= 2: 1). Mp 251-252 °C (MeOH). ¹H NMR (400 MHz, CDCl₃): δ (ppm) 3.02 (1H, d, *J* = 5.2 Hz, indane-H), 3.06 (1H, d, *J* = 5.2 Hz, indane-H), 3.59 (1H, d, *J* = 7.2 Hz, indane-H), 3.63 (1H, d, *J* = 7.2 Hz, indane-H), 5.10 (1H, d, *J* = 6.8 Hz, -NH), 5.23 (1H, q, *J* = 5.2 Hz, indane-H), 7.21-7.25 (2H, m, Ar'-H), 7.28-7.31 (2H, m, Ar'-H), 7.47 (1H, td, *J* = 6.8, 1.2 Hz, Ar-H), 7.58 (1H, d, *J* = 8.4 Hz, Ar-H), 7.61-7.67 (1H, td, *J* = 6.8, 1.2 Hz, Ar-H), 7.87 (1H, d, *J* = 7.6 Hz, Ar-H), 8.57 (1H, d, *J* = 2.0 Hz, Ar-H), 9.46 (1H, dd, *J* = 8.8, 0.8 Hz, Ar-H). ¹³C NMR (100 MHz, CDCl₃): δ (ppm) 40.41, 53.27, 120.79, 123.60, 124.81, 124.93, 125.84, 126.76, 127.37, 127.46, 129.38, 129.51, 129.71, 131.02, 132.50, 132.54, 134.12, 141.29, 145.56, 150.20, 181.00 (CO). HRMS (ESI) m/z calcd for C₂₅H₁₇N₂OSCl [M]⁺: 428.0750; found [M+H]⁺: 429.0822.

### Example 41

### 10-Chloro-6-(cyclohexylamino)-12H-thiochromeno[2,3-c]quino lin-12-one (N21)

Product **N21** was prepared from **3** and cyclohexylamine. The pure compound was obtained as a brown solid (yield 91%) (*R_{f}* = 0.7 at CH₂Cl₂: n-hexane= 2: 1). Mp 196-197 °C (MeOH). ¹H NMR (400 MHz, CDCl₃): δ (ppm) 1.25-1.40 (4H, m, cyclohexylamine-CH₂), 1.49-1.60 (2H, m, cyclohexylamine-CH₂), 1.70-1.74 (2H, m, cyclohexylamine-CH₂), 1.79-1.84 (2H, m, cyclohexylamine-CH₂), 2.21 (2H, dd, *J* = 8.8, 3.2 Hz, cyclohexylamine-CH₂), 4.30 (1H, sep, *J* = 3.6 Hz, cyclohexylamine-CH), 4.72 (1H, d, *J* = 6.8 Hz, -NH-), 7.41(1H, t, *J* = 8.0 Hz, Ar-H), 7.51-62 (3H, m, Ar-H), 7.79 (1H, d, *J* = 8.0 Hz, Ar-H), 8.51 (1H, d, *J* = 1.6 Hz, Ar-H), 9.41 (1H, d, *J* = 8.4 Hz, Ar-H). ¹³C NMR (100 MHz, CDCl₃): δ (ppm) 24.94, 25.92, 33.10, 50.26, 120.51, 123.50, 124.34, 125.77, 127.11, 127.37, 129.27, 129.35, 129.60, 131.01, 132.37, 132.41, 134.00, 145.66, 149.75, 180.95 (CO). HRMS (ESI) m/z calcd for C₂₂H₁₉N₂OSCl [M]⁺: 394.0907; found [M+H]⁺: 395.0991.

### Example 42

### 6-((1-Benzylpiperidin-4-yl)amino)-10-chloro-12H-thiochromen o[2,3-c]quinolin-12-one (N22)

Product **N22** was prepared from **3** and 1-benzylpiperidin-4-amine. The pure compound was obtained as a brown solid (yield 62%) (*R_{f}* = 0.77 at EA). Mp 194-196 °C (MeOH). ¹H NMR (400 MHz, CDCl₃): δ (ppm) 1.62-1.72 (2H, m, piperidine-H), 2.24 (2H, d, *J* = 13.2 Hz, piperidine-H), 2.32 (2H, t, *J* = 11.2 Hz, piperidine-H), 2.92 (2H, d, *J* = 11.6 Hz, piperidine-H), 3.59 (2H, s, -CH₂-), 4.35 (1H, sext, *J* = 6.4 Hz, piperidine-CH), 4.75 (1H, d, *J* = 7.2 Hz, -NH), 7.26-7.30 (1H, m, Ar'-H), 7.36-7.38 (4H, m, Ar'-H), 7.44 (1H, td, *J* = 7.6, 0.8 Hz, Ar-H), 7.59-7.64 (3H, m, Ar-H), 7.80 (1H, d, *J* = 7.6 Hz, Ar-H), 8.56 (1H, d, *J* = 1.6 Hz, Ar-H), 9.43 (1H, d, *J* = 8.8 Hz, Ar-H). ¹³C NMR (100 MHz, CDCl₃): δ (ppm) 32.26, 48.63, 52.35, 63.22, 120.63, 123.47, 124.57, 125.81, 127.07, 127.12, 127.47, 128.25, 129.21, 129.35, 129.44, 129.74, 130.99, 132.46, 132.52, 134.10, 138.37, 145.57, 149.76, 181.00 (CO). HRMS (ESI) m/z calcd for C₂₈H₂₄N₃OSCl [M]⁺: 485.1329; found [M+H]⁺: 486.1379.

### Example 43

### 10-Chloro-6-((thiophen-2ylmethyl)amino)-12H-thiochromeno[ 2,3-c]quinolin-12-one (N23)

Product **N23** was prepared from **3** and 2-thiophenemethylamine. The pure compound was obtained as a brown solid (yield 78%) (*R_{f}* = 0.7 at CH₂Cl₂: n-hexane = 2: 1). Mp 178-180 °C (MeOH). ¹H NMR (400 MHz, CDCl₃): δ (ppm) 5.07 (1H, d, *J* = 5.2 Hz, -NCH₂-), 5.17 (1H, br, -NH-),7.00 (1H, t, *J* = 4.4 Hz, thiophene-H), 7.16 (1H, d, *J* = 3.2 Hz, thiophene-H), 7.25 (1H, d, *J* = 0.8 Hz, thiophene-H), 7.47(1H, t, *J* = 8.0 Hz, Ar-H), 7.52 (1H, d, *J* = 8.4 Hz, Ar-H), 7.58 (1H, d, *J* = 8.4 Hz, Ar-H), 7.65 (1H, t, *J* = 7.6 Hz, Ar-H), 7.89 (1H, d, *J* = 8.0 Hz, Ar-H), 8.53 (1H, s, Ar-H), 9.46 (1H, d, *J* = 8.4 Hz, Ar-H). ¹³C NMR (100 MHz, CDCl₃): δ (ppm) 41.14, 121.06, 123.58, 125.05, 125.47, 125.89, 126.47, 126.73, 127.29, 127.45, 129.33, 129.57, 129.71, 130.94, 132.39, 132.51, 134.12, 141.36, 145.20, 149.82, 180.80 (CO). HRMS (ESI) m/z calcd for C₂₁H₁₃N₂OS₂Cl [M]⁺: 408.0158; found [M+H]⁺: 409.0251, [M-H]⁻: 407.0085.

### Example 44

### 10-Chloro-6-((cyclohexylmethyl)amino)-12H-thiochromeno[2,3 -c]quinolin-12-one (N24)

Product **N24** was prepared from **3** and cyclohexylmethanamine. The pure compound was obtained as a brown solid (yield 79%) (*R_{f}* = 0.7 at CH₂Cl₂: n-hexane = 2: 1). Mp 165-166 °C (MeOH). ¹H NMR (400 MHz, CDCl₃): δ (ppm) 1.07 (1H, d, *J* = 11.2 Hz, cyclohexyl-CH₂), 1.30 (1H, d, *J* = 11.2 Hz, cyclohexyl-CH₂), 1.23 (2H, q, *J* = 11.6 Hz, cyclohexyl-CH₂), 1.31 (2H, q, *J* = 11.6 Hz, cyclohexyl-CH₂), 1.78-1.81 (4H, m, cyclohexyl-CH₂), 1.90 (2H, d, *J* = 12.4 Hz, cyclohexyl-CH₂), 3.53 (2H, t, *J* = 6.0 Hz, -NCH₂-), 4.85 (1H, br, -NH-), 7.40 (1H, t, *J* = 7.2 Hz, Ar-H), 7.51 (1H, d, *J* = 8.8 Hz, Ar-H), 7.55 (1H, d, *J* = 1.6 Hz, Ar-H), 7.60 (1H, t, *J* = 8.0 Hz, Ar-H), 7.79 (1H, d, *J* = 8.0 Hz, Ar-H), 8.50 (1H, d, *J* = 1.2 Hz, Ar-H), 9.41 (1H, d, *J* = 8.4 Hz, Ar-H). ¹³C NMR (100 MHz, CDCl₃): δ (ppm) 25.97, 26.52, 31.23, 37.63, 48.50, 120.59, 123.57, 124.41, 125.80, 127.10, 127.38, 129.26, 129.38, 129.45, 130.95, 132.33, 132.41, 134.02, 145.58, 150.64, 180.87 (CO). HRMS (ESI) m/z calcd for C₂₃H₂₁N₂OCl [M]⁺: 408.1063; found [M+H]⁺: 409.1115.

### Example 45

### 6-(Benzylamino)-10-chloro-12H-thiochromeno[2,3-c]quinolin-12-tone (N25)

Product **N25** was prepared from **3** and benzylamine. The pure compound was obtained as a brown solid (yield 93%) (*R_{f}* = 0.67 at CH₂Cl₂: n-hexane = 2: 1). Mp 194-195 °C (MeOH). ¹H NMR (400 MHz, CDCl₃): δ (ppm) 4.94 (2H, d, *J* = 5.2 Hz, -CH₂-), 5.16 (1H, br, -NH-), 7.33-7.51 (6H, m, Ar-H), 7.58-7.67 (3H, m, Ar-H), 7.87 (1H, d, *J* = 8.0 Hz, Ar-H), 8.59 (1H, d, *J* = 2.0 Hz, Ar-H), 9.47 (1H, d, *J* = 8.0 Hz, Ar-H). ¹³C NMR (100 MHz, CDCl₃): δ (ppm) 37.63, 120.98, 123.54, 124.89, 125.87, 127.26, 127.50, 127.63, 128.24, 128.79, 129.43, 129.57, 129.85, 131.02, 132.58, 134.18, 138.82, 145.48, 150.33, 181.00 (CO). HRMS (ESI) m/z calcd for C₂₃H₁₅N₂OSCl [M]⁺: 402.0594; found [M+H]⁺: 403.0692.

### Example 46

### 10-Chloro-6-((pyridin-2-ylmethyl)amino)-12H-thiochromeno[2, 3-c]quinolin-12-one (N26)

Product **N26** was prepared from **3** and 2-picolylamine. The pure compound was obtained as a brown solid (yield 93%) (*R_{f}* = 0.25 at EA). Mp 187-189 °C (MeOH). ¹H NMR (400 MHz, CDCl₃): δ (ppm) 5.01 (2H, d, *J* = 4.0 Hz, -CH₂-), 6.79 (1H, br, -NH-), 7.24-7.28 (1H, m, Ar'-H), 7.45 (2H, t, *J* = 7.2 Hz, Ar'-H & Ar-H), 7.61-7.67 (3H, m, Ar-H), 7.73 (1H, td, *J* = 7.6, 1.6 Hz, Ar-H), 7.86 (1H, d, *J* = 8.4 Hz, Ar-H), 8.58 (1H, d, *J* = 2.0 Hz, Ar-H), 8.67 (1H, d, *J* = 4.8 Hz, Ar'-H), 9.47 (1H, d, *J* = 8.4 Hz, Ar-H). ¹³C NMR (100 MHz, CDCl₃): δ (ppm) 46.74, 120.81, 122.37, 124.19, 124.61, 125.93, 127.04, 127.60, 129.33, 129.44, 129.60, 131.40, 132.46, 132.52, 134.03, 136.94, 145.62, 148.94, 150.43, 156.58, 181.00 (CO). HRMS (ESI) m/z calcd for C₂₂H₁₄N₃OSCl [M]⁺: 403.0546; found [M+H]⁺: 404.0615.

### Example 47

### 6-((Benzo[d][1,3]dioxol-5-ylmethyl)amino)-10-chloro-12H-thio chromeno[2,3-c]quinolin-12-one (N27)

Product **N27** was prepared from **3** and piperonylamine. The pure compound was obtained as a brown solid (yield 90%) (*R_{f}* = 0.88 at EA). Mp 205-206 °C (MeOH). ¹H NMR (400 MHz, CDCl₃): δ (ppm) 4.82 (2H, t, *J* = 5.2 Hz, -NCH₂-), 5.08 (1H, br, -NH-), 5.97 (2H, s, -OCH₂O-), 6.82 (1H, d, *J* = 8.0 Hz, Ar'-H), 6.96 (1H, d, *J* = 8.0 Hz, Ar'-H), 7.00 (1H, d, *J* = 1.2 Hz, Ar'-H), 7.47 (1H, td, *J* = 8.0, 1.2 Hz, Ar-H), 7.57 (1H, d, *J* = 8.8 Hz, Ar-H), 7.60-7.66 (2H, m, Ar-H), 7.86 (1H, d, *J* = 8.0 Hz, Ar-H), 8.62 (1H, d, *J* = 2.0 Hz, Ar-H), 9.46 (1H, d, *J* = 8.8 Hz, Ar-H). ¹³C NMR (100 MHz, CDCl₃): δ (ppm) 46.30, 101.10, 108.40, 108.85, 120.95, 121.58, 123.52, 124.88, 125.86, 127.23, 127.46, 129.39, 129.56, 129.76, 130.98, 132.48, 132.55, 132.61, 134.15, 145.42, 147.07, 147.92, 150.21, 180.93 (CO). HRMS (ESI) m/z calcd for C₂₄H₁₅N₂O₃SCl [M]⁺: 446.0492; found [M+H]⁺: 447.0586, [M-H]⁻: 445.0440.

### Example 48

### 10-Chloro-6-((2-methoxybenzyl)amino)-12H-thiochromeno[2,3 -c]quinolin-12-one (N28)

Product **N28** was prepared from **3** and 2-methoxybenzylamine. The pure compound was obtained as a brown solid (yield 82%) (*R_{f}* = 0.65 at CH₂Cl₂: n-hexane = 2: 1). Mp 223-224 °C (MeOH). ¹H NMR (400 MHz, CDCl₃): δ (ppm) 3.95 (3H, s, -OCH₃), 4.93 (2H, d, *J* = 5.6 Hz, -NCH₂-), 5.57 (1H, t, *J* = 5.6 Hz, -NH-), 5.97 (2H, s, -OCH₂O-), 6.94-7.00 (2H, m, Ar'-H), 7.30 (1H, td, *J* = 8.0, 2.0 Hz, Ar'-H), 7.45 (1H, td, *J* = 8.0, 1.6 Hz, Ar'-H), 7.51 (1H, d, *J* = 7.2 Hz, Ar'-H), 7.59-7.66 (3H, m, Ar-H), 7.89 (1H, dd, *J* = 8.4, 1.2 Hz, Ar-H), 8.57 (1H, dd, *J* = 2.0, 0.8 Hz, Ar-H), 9.45 (1H, dd, *J* = 8.4, 0.8 Hz, Ar-H). ¹³C NMR (100 MHz, CDCl₃): δ (ppm) 42.33, 55.49, 110.47, 120.68, 120.81, 123.90, 124.59, 125.82, 126.68, 127.20, 127.52, 128.88, 129.38, 129.44, 129.71, 130.49, 131.23, 132.46, 132.54, 134.04, 145.59, 150.62, 157.89, 181.04 (CO). HRMS (ESI) m/z calcd for C₂₄H₁₇N₂O₂SCl [M]⁺: 432.0699; found [M+H]⁺: 433.0783.

### Example 49

### 10-Chloro-6-((3,4-dimethoxybenzyl)amino)-12H-thiochromeno [2,3-c]quinolin-12-one (N29)

Product **N29** was prepared from **3** and 3,4-dimethoxybenzylamine. The pure compound was obtained as a brown solid (yield 84%) (*R_{f}* = 0.66 at CH₂Cl₂: n-hexane = 2: 1). Mp 251-252 °C (MeOH). ¹H NMR (400 MHz, CDCl₃): δ (ppm) 3.89 (3H, s, -OCH₃), 3.90 (3H, s, -OCH₃), 4.86 (2H, d, *J* = 4.8 Hz, -NCH₂-), 5.11 (1H, t, *J* = 5.2 Hz, -NH-), 6.89 (1H, d, *J* = 8.0 Hz, Ar'-H), 7.05 (1H, dd, *J* = 8.0, 2.0 Hz, Ar'-H), 7.08 (1H, d, *J* = 2.0 Hz, Ar'-H), 7.48 (1H, td, *J* = 7.6, 1.2 Hz, Ar-H), 7.60 (1H, dd, *J* = 8.4, 0.4 Hz, Ar-H), 7.65 (1H, dd, *J* = 8.4, 1.5 Hz, Ar-H), 7.66 (1H, td, *J* = 8.0, 1.2 Hz, Ar-H), 7.88 (1H, dd, *J* = 8.4, 0.8 Hz, Ar-H), 8.59 (1H, dd, *J* = 1.5, 0.4 Hz, Ar-H), 9.48 (1H, dd, *J* = 8.4, 1.2 Hz, Ar-H). ¹³C NMR (100 MHz, CDCl₃): δ (ppm) 46.45, 55.96, 55.99, 111.31, 111.82, 120.59, 120.99, 123.56, 124.88, 125.91, 127.20, 127.50, 129.45, 129.60, 129.88, 131.04, 131.34, 132.59, 134.21, 145.52, 148.63, 149.20, 150.34, 181.01 (CO). HRMS (ESI) m/z calcd for C₂₅H₁₉N₂O₃SCl [M]⁺: 462.0805; found [M+H]⁺: 463.0900, [M-H]⁻: 461.0754.

### Example 50

### 10-Chloro-6-(phenethylamino)-12H-thiochromeno[2,3-c]quinol in-12-one (N30)

Product **N30** was prepared from **3** and phenethylamine. The pure compound was obtained as a brown solid (yield 94%) (*R_{f}* = 0.52 at CH₂Cl₂: n-hexane= 2: 1). Mp 151-152 °C (MeOH). ¹H NMR (400 MHz, CDCl₃): δ (ppm) 3.10 (2H, t, *J* = 6.8 Hz, -CH₂-), 3.98 (2H, q, *J* = 6.4 Hz, -NCH₂-),4.91 (1H, t, *J* = 4.8 Hz, -NH-), 7.27-7.39 (5H, m, Ar'-H), 7.45 (1H, t, *J* = 8.0 Hz, Ar-H), 7.54 (1H, d, *J* = 8.4 Hz, Ar-H), 7.59 (1H, d, *J* = 1.2 Hz, Ar-H), 7.63 (1H, t, *J* = 7.6 Hz, Ar-H), 7.85 (1H, d, *J* = 8.4 Hz, Ar-H), 8.54 (1H, d, *J* = 1.6 Hz, Ar-H), 9.44 (1H, d, *J* = 8.4 Hz, Ar-H). ¹³C NMR (100 MHz, CDCl₃): δ (ppm) 35.33, 43.52, 120.77, 123.66, 124.69, 125.85, 126.56, 127.23, 127.50, 128.73, 128.94, 129.33, 129.48, 129.50, 131.03, 132.54, 134.08, 139.30, 145.57, 150.36, 180.94 (CO). HRMS (ESI) m/z calcd for C₂₄H₁₇N₂OSCl [M]⁺: 416.9226; found [M+H]⁺: 417.0857, [M+H+2]⁺: 419.0834.

### Example 51

### 10-Chloro-6-((4-methoxyphenethyl)amino)-12H-thiochromeno[ 2,3-c]quinolin-12-one (N31)

Product **N31** was prepared from **3** and 2-(4-methoxyphenyl)ethylamine. The pure compound was obtained as a yellow solid (yield 95%) (*R_{f}* = 0.89 at CH₂Cl₂). Mp 214-215 °C (MeOH). ¹H NMR (400 MHz, CDCl₃): δ (ppm) 3.03 (2H, t, *J* = 6.8 Hz, -CH₂-), 3.81 (3H, s, -OCH₃), 3.94 (2H, q, *J* = 6.4 Hz, -NCH₂-), 4.90 (1H, t, *J* = 4.8 Hz, -NH-), 6.90 (2H, d, *J* = 8.4 Hz, Ar'-H), 7.23 (2H, d, *J* = 8.4 Hz, Ar'-H), 7.45 (1H, t, *J* = 7.6 Hz, Ar-H), 7.55 (1H, d, *J* = 8.8 Hz, Ar-H), 7.59 (1H, d, *J* = 2.0 Hz, Ar-H), 7.63 (1H, t, *J* = 7.6 Hz, Ar-H), 7.85 (1H, d, *J* = 8.0 Hz, Ar-H), 8.54 (1H, d, *J* = 2.0 Hz, Ar-H), 9.44 (1H, d, *J* = 8.8 Hz, Ar-H). ¹³C NMR (100 MHz, CDCl₃): δ (ppm) 34.39, 43.68, 55.31, 114.13, 120.76, 123.67, 124.67, 125.85, 127.22, 127.52, 129.36, 129.49, 129.68, 129.87, 131.05, 131.24, 132.48, 134.09, 145.59, 150.41, 158.31, 180.99 (CO). HRMS (ESI) m/z calcd for C₂₅H₁₉N₂O₂SCl [M]⁺: 446.0856; found [M+H]⁺: 447.0938.

### Example 52

### 6-((4-Aminophenethyl)amino)-10-chloro-12H-thiochromeno[2, 3-c]quinolin-12-one (N32)

Product **N32** was prepared from **3** and 2-(4-aminophenyl)ethylamine. The pure compound was obtained as a yellow solid (yield 82%) (*R_{f}* = 0.52 at CH₂Cl₂). Mp 208-210 °C (MeOH). ¹H NMR (400 MHz, CDCl₃): δ (ppm) 2.97 (2H, t, *J* = 6.8 Hz, -CH₂-), 3.63 (2H, br, -NH₂), 3.91 (2H, q, *J* = 6.4 Hz, -NCH₂-),4.91 (1H, t, *J =* 4.8 Hz, -NH-), 6.70 (2H, d, *J* = 8.4 Hz, Ar'-H), 7.09 (2H, d, *J* = 8.0 Hz, Ar'-H), 7.44 (1H, t, *J* = 7.6 Hz, Ar-H), 7.55 (1H, d, *J* = 8.0 Hz, Ar-H), 7.60 (1H, d, *J* = 8.4 Hz, Ar-H), 7.63 (1H, t, *J* = 7.6 Hz, Ar-H), 7.84 (1H, d, *J* = 8.0 Hz, Ar-H), 8.54 (1H, d, *J* = 2.0 Hz, Ar-H), 9.44 (1H, d, *J* = 8.4 Hz, Ar-H). ¹³C NMR (100 MHz, CDCl₃): δ (ppm) 34.37, 43.70, 115.52, 120.72, 123.73, 124.59, 125.83, 127.19, 127.51, 129.06, 129.31, 129.45, 129.59, 129.75, 131.09, 132.42, 134.04, 144.89, 145.60, 150.45, 180.96 (CO). HRMS (ESI) m/z calcd for C₂₄H₁₆N₃OSCl [M]⁺: 431.0859; found [M+H]⁺: 432.0950.

### Example 53

### 2-(10-Chloro-12-oxo-12H-thiochromeno[2,3-c]quinolin-6-yl)gu anidine (TC-SCl-A-41) (N33)

Product **N33** was a yellow solid (yield 85%). Mp: 370 °C.(dec.) ¹H NMR (400 MHz, DMSO-*d*₆): δ ppm. 7.40 (3H, td, *J* = 8.4, 1.2 Hz, Ar-H & -NH₂), 7.59 (1H, td, *J* = 8.7, 1.2 Hz, Ar-H), 7.59 (1H, dd, *J* = 8.4, 0.8 Hz, Ar-H), 7.83 (1H, dd, *J* = 8.4, 2.0 Hz, Ar-H), 7.95 (1H, d, *J* = 8.8 Hz, Ar-H), 8.40 (1H, d, *J* = 2.4 Hz, Ar-H), 9.49 (1H, dd, *J* = 8.4, 0.8 Hz, Ar-H). ¹³C NMR (100 MHz, DMSO-*d*₆): δ ppm. 120.77, 124.49, 125.86, 126.82, 128.11, 128.60, 129.21, 129.85, 132.13, 132.52, 132.68, 136.17, 136.80, 144.49, 159.19, 181.16. HRMS (ESI) calcd for C₁₇H₁₁N₄OSCl [M]⁺ 354.0342; found [M+H]⁺ 355.0438.

### Example 54

### 10-Chloro-6-(piperidin-1-ylamino)-12H-thiochromeno[2,3-c]qu inolin-12-one (TC-SCl-A-26) (N34)

Product **N34** was a yellow solid (yield 60%). Mp: 180-181 °C. ¹H NMR (400 MHz, CDCl₃): δ ppm. 1.72-1.74 (2H, m, -CH₂-), 1.89 (4H, quin, *J* = 5.2 Hz, -CH₂-), 3.32 (4H, *J* = 4.8 Hz, -CH₂-), 7.36(1H, tt, *J* = 8.7,2.1 Hz, Ar-H₁₀), 7.47 (1H, dd, *J* = 8.4, 2.7 Hz, Ar-H₈), 7.61-7.73 (3H, m, Ar-H), 8.00 (1H, d, *J* = 8.0 Hz, Ar-H), 8.59 (1H,d, *J* = 2.0 Hz, Ar-H), 9.63 (1H, d, *J* = 8.8 Hz, Ar-H). ¹³C NMR (100 MHz, CDCl₃): δ ppm. 24.28, 25.91, 52.36, 123.49, 125.77, 127.32, 127.90, 128.59, 129.06, 129.27, 130.51, 131.66, 132.18, 132.39, 133.49, 134.70, 144.98, 158.53, 181.58.

### Pharmacological activity assay

In pharmacological tests, compounds synthesized chemically including **2-21,** N**-1** to N-**34** (a total of 54 drugs) are subjected to the following pharmacological activity tests: (1) MTT assay; (2) Topoisomerase I and II activities assay; (3) cytotoxicity assays conducted by NCI on the 26 screened compounds in 60 cancer cell lines.

### Example 55

### MTT assay for cell cytotoxicity

All of synthesis compounds were evaluated cell cytotoxicity by using MTT colorimetric assay on PC-3 and DU-145 cell lines. DU-145 and PC-3 are human hormone-refractory (androgen-independent) prostatic cancer cell lines from American Type Culture Collection (HTB-81™, ATCC, Rockville, MD)¹²⁵ and Bioresource Collection and Research Center (60122, BCRC, Taiwan)¹²⁶, respectively. Two of the "classical" cell lines were cultivated in RPMI-1640 medium supplemented with 5% fetal bovine serum (v/v), 100 U/mL penicillin, and 50 mg/mL streptomycin. Approximately 2 x 10³ cells were seeded into each well of a 96-well plate and incubated in 5% CO₂ at 37 °C for 24 h. To evaluate the *in vitro* cytotoxicity, all the synthetic compounds were dissolved in DMSO, prepared immediately before the experiments and diluted into the complete medium before being added to each well of a 96-well plate. Each compound was then added to the culture medium for designated various concentrations (0.15, 0.5, 1.5, 5, 15 µM). After 72 h, an amount of 100 µL of MTT (1 mg/mL) was added to each well, and the samples were incubated at 37 °C for 4 h. After removing the MTT solution, 100 µL of DMSO was added to each well and incubated at 37 °C for another 20 mins. The absorbency at 560 nm was measured by using an ELISA reader.

Results are expressed as mean values of at least three independent experiments. The IC₅₀ values of testing compounds were described in Table 1.

**Table1. Effects of 10-Chloro-12H-thiochromeno[2,3-c]quinolin -12-one derivatives on cytotoxicity by MTT assay.**

| | | | |
|---|---|---|---|
| No. | Compound structure or R substitutions | MTT assay (IC₅₀ ± SD) ^{a} | |
| | | DU-145 (µM) | PC-3 (µM) |
| **2** | 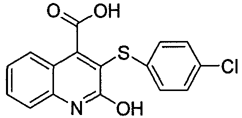 | > 15 | > 15 |
| **3** | 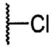 | > 15 | > 15 |
| **4** | 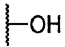 | > 15 | 10.10 ± 1.81 |
| **5** | 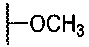 | 10.84 ± 6.55 | 3.89 ± 0.54 |
| **6** | 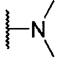 | > 15 | > 15 |
| **7** | 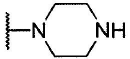 | 5.01 ± 1.68 | 2.84 ± 0.64 |
| **8** | 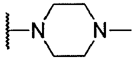 | 12.94 ± 0.26 | 7.18 ± 2.45 |
| **9** | 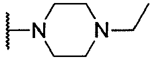 | > 15 | 12.04 ± 3.41 |
| **10** | 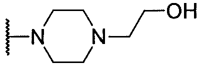 | > 15 | > 15 |
| **11** | 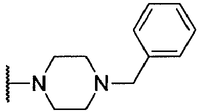 | > 15 | > 15 |
| **12** | 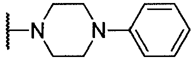 | > 15 | > 15 |
| **13** | 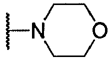 | > 15 | > 15 |
| **14** | 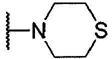 | > 15 | > 15 |
| **15** | 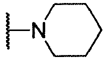 | 13.38 ± 2.87 | > 15 |
| **16** | 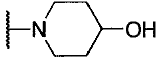 | 11.12 ± 4.18 | 9.55 ± 2.42 |
| **17** | 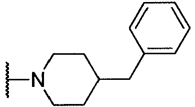 | > 15 | > 15 |
| **18** | 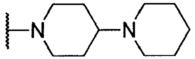 | 10.23 ± 2.03 | 14.16 ± 1.41 |
| **19** | 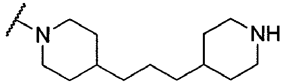 | 9.02 ± 1.20 | 6.36 ± 0.17 |
| **20** | 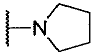 | > 15 | > 15 |
| **21** | 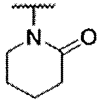 | 11.12 ± 4.18 | 9.55 ± 2.42 |
| **N1** | | 11.65 ± 5.15 | 14.48 ± 2.70 |
| **N2** | | 6.70 ± 1.62 | 5.18 ± 2.20 |
| **N3** | | 7.64 ± 2.74 | 7.41 ± 3.43 |
| **N4** | | 10.35 ± 2.46 | 10.11 ± 1.32 |
| **N5** | | > 15 | > 15 |
| **N6** | | > 15 | > 15 |
| **N7** | | 5.94 ± 3.45 | 2.25 ± 0.61 |
| **N8** | | 1.73 ± 0.72 | 1.11 ± 0.69 |
| **N9** | | 11.55 ± 7.23 | 7.48 ± 3.35 |
| **N10** | | 5.94 ± 2.03 | 6.31 ± 4.11 |
| **N11** | | 4.95 ± 0.58 | 8.38 ± 3.39 |
| **N12** | | 6.74 ± 2.14 | 7.05 ± 2.48 |
| **N13** | | 14.16 ± 1.18 | 10.38 ± 2.30 |
| **N14** | | 1.80 ± 0.40 | 1.64 ± 0.55 |
| **N15** | 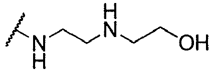 | 3.81 ± 1.88 | 3.44 ± 1.81 |
| **N16** | 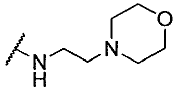 | > 15 | >15 |
| **N17** | 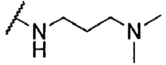 | 2.70 ± 0.16 | 2.27 ± 0.09 |
| **N18** | 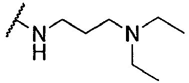 | 3.53 ± 1.05 | 2.22 ± 0.21 |
| **N19** | 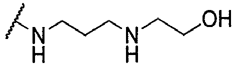 | 1.50 ± 1.32 | 1.98 ± 0.72 |
| **N20** | 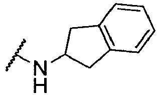 | 4.86 ± 0.99 | 6.20 ± 2.52 |
| **N21** | 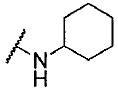 | 8.06 ± 2.08 | 7.51 ± 0.22 |
| **N22** | 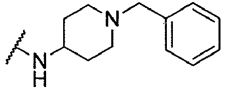 | 8.39 ± 1.84 | 6.47 ± 0.30 |
| **N23** | 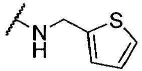 | 14.65 ± 3.84 | > 15 |
| **N24** | 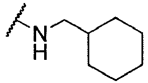 | 13.55 ± 3.88 | 14.80 ± 3.26 |
| **N25** | 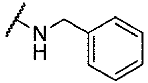 | 13.49 ± 0.69 | 11.16 ± 1.55 |
| **N26** | 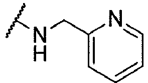 | 14.75 ± 1.99 | 14.20 ± 2.22 |
| **N27** | 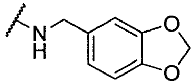 | 11.82 ± 5.83 | > 15 |
| **N28** | 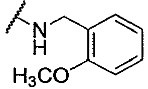 | 9.61 ± 4.88 | 6.01 ± 3.31 |
| **N29** | 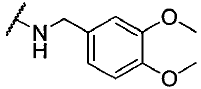 | 4.86 ± 0.99 | 6.20 ± 2.52 |
| **N30** | 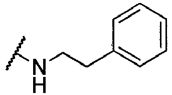 | > 15 | > 15 |
| **N31** | | > 15 | > 15 |
| **N32** | | 13.59 ± 1.39 | 10.11 ± 0.94 |
| **N33** | 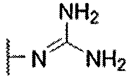 | 8.11 ± 1.37 | 6.13 ± 6.98 |
| **N34** | 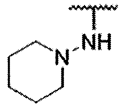 | 9.06 ± 1.95 | 8.22 ± 1.02 |
| - | Mitoxantrone | 0.10 ± 0.01 | 0.39 ± 0.02 |
| - | Doxorubicin | 0.12 ± 0.03 | 0.63 ± 0.26 |
| - | Camptothecin | 0.10 ± 0.01 | 0.10 ± 0.01 |
| - | Etoposide (VP-16) | 0.40 ± 0.01 | 4.33 ± 0.86 |

| | | | |
|---|---|---|---|
| ^{a} SD: standard derivatives, all experiments were independently performed at least three times. | | | |

Besides, compounds **N7, N8, N14, N15, N17,** and **N18** containing more than one nitrogen atom in the side chains showed the outstanding cytotoxic activities than having a hydroxyl group, alkyl group, or aromatic rings. Compounds **5, 7, 8, 16, 19, N2, N7, N8, N9, N14, N15, N16, N17, N18, N19,** and **N25** were selected for TOPs activities assay.

### Example 56

### Topoisomerase I and II activities assay

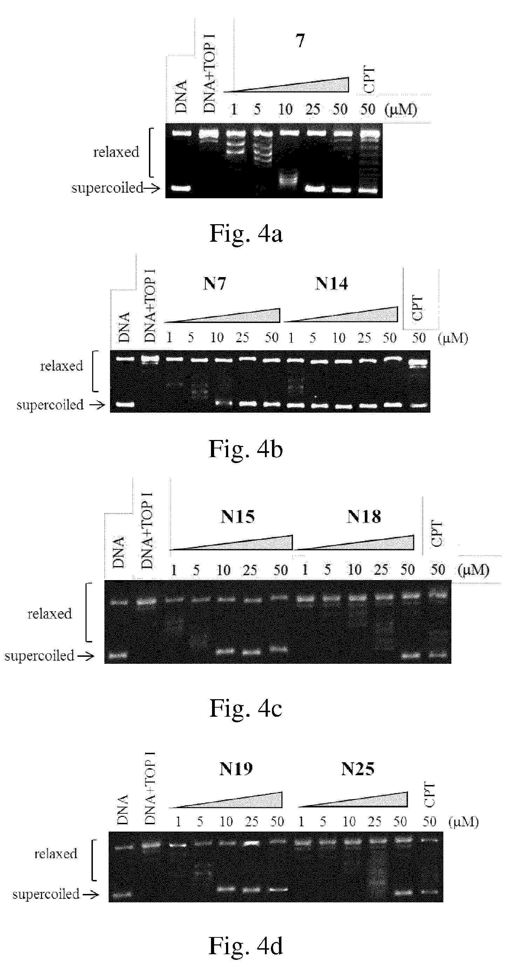

According to the cell cytotoxicity, compounds **5, 7, 8, 16, 19, N2, N7, N8, N9, N14-N19,** and **N25** were also selected for primary TOP I and II activities assays at 25 and/or 50 µM (Fig. 2-4). In TOP I activity assay, compounds **7, N7, N14, N15, N17, N18,** and **N25** were showed more potent inhibitory effects than CPT and selected for further evaluation by using five different concentrations (Figure 4). The IC₅₀ value of compounds **7, N7, N14, N15, N18, N19,** and **N25** were about 10, 10, 1, 5, 25, 5, and 25 µM, respectively (detect by TopoGEN TG2005H, TG-2000H-1).

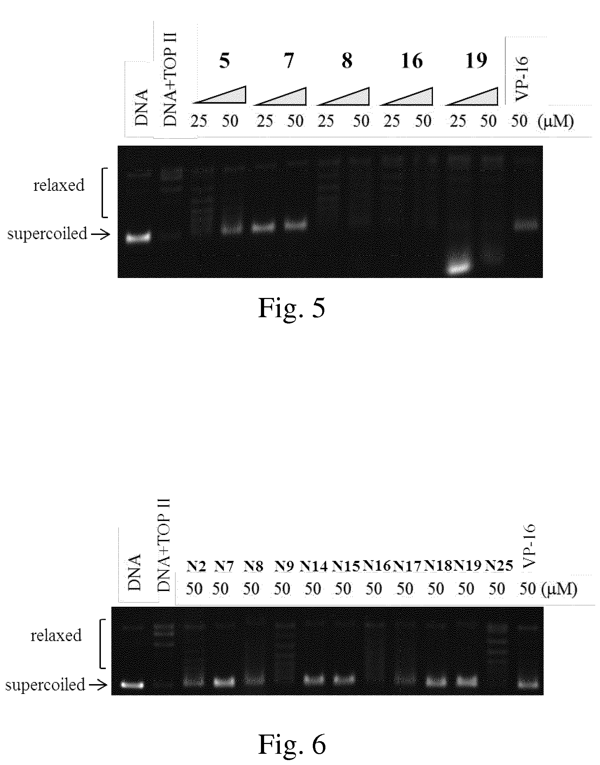

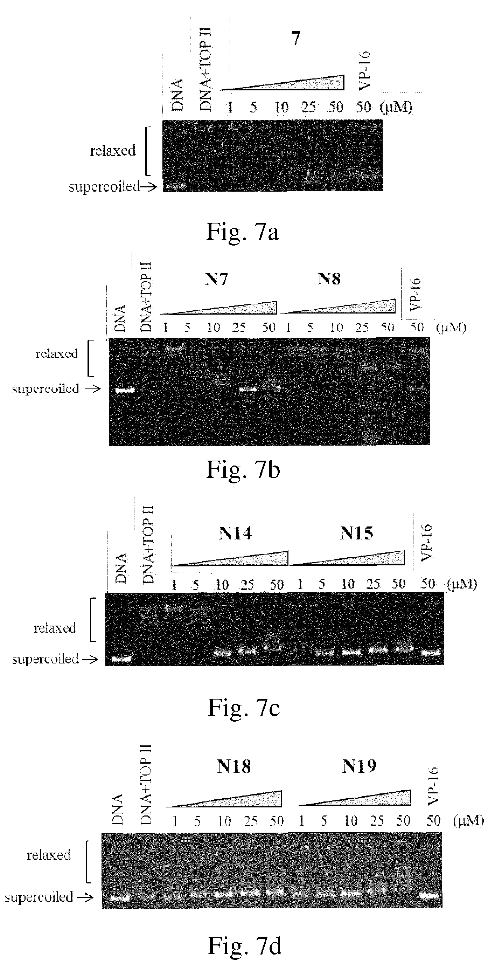

We also performed TOP 11-catalyzed relaxation of plasmid DNA assays(Fig. 5-7) to evaluate if compounds could inhibit TOP II. Respond to our drug design, compounds **7, N7, N8, N14, N15, N18,** and **N19** were showed more potent inhibitory effects than the positive agent VP-16 and selected for further evaluation by using five different concentrations (Figure 7). The IC₅₀ value of compounds **7, N7, N8, N14, N15, N18,** and **N19** were about 10, 10, 1, 10, 5, 1, and 1 µM, respectively (detect by TopoGEN TG2005H, TG-2000H-1).

### Example 57

### National Cancer Institute Cancer Cell Cytotoxicity assay:

The test results shown in this section are the compound cytotoxicities in vitro against cancer cell lines National Cancer Institute (NCI)'s anticancer drug screen and 26 compounds (2, 3, 4, 5, 6, 8, 10, 11, 12, 13, N1, N2, N6, N7, N9, N12, N13, N14, N16, N17, N19, N21, N25, N27, N30, N31) screened. In the first stage, cytotoxicity of the 26 compounds at the concentration of 10 µM was conducted on 60 cell lines and SRB assay was performed after 48 hours of incubation. The results are shown in Tables 2 to 4 and are represented by growth percentage.

Furthermore, among five compounds were active drugs for further their cytostatic and cytotoxic activities against the 60 cell panel by using five dose studies (0.01, 0.1, 1, 10 and 100 µM) (Table 5).

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

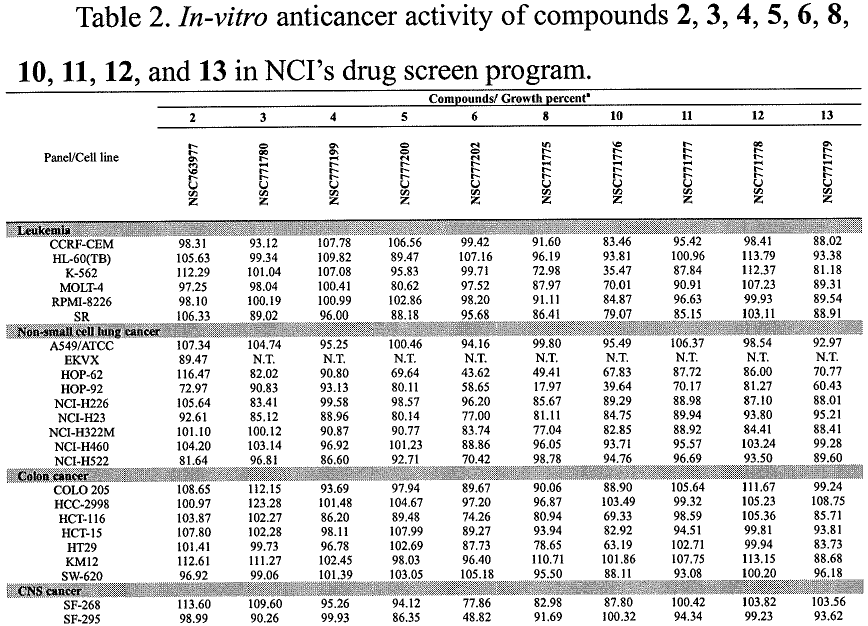

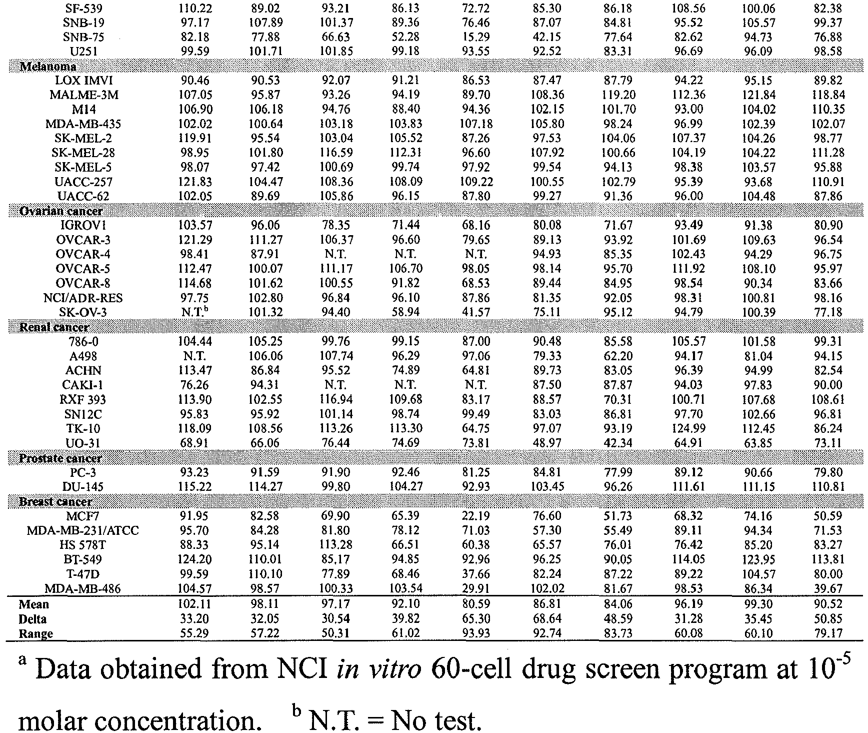

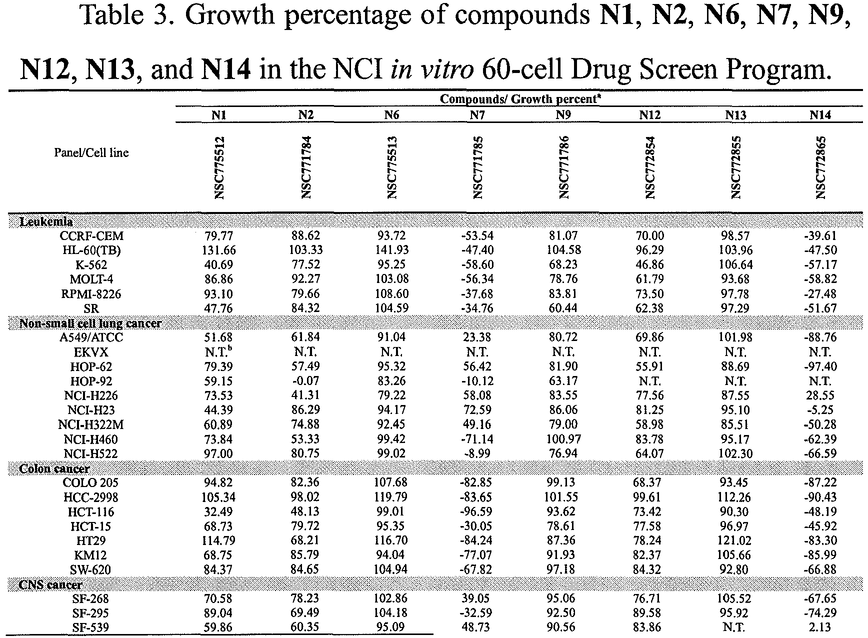

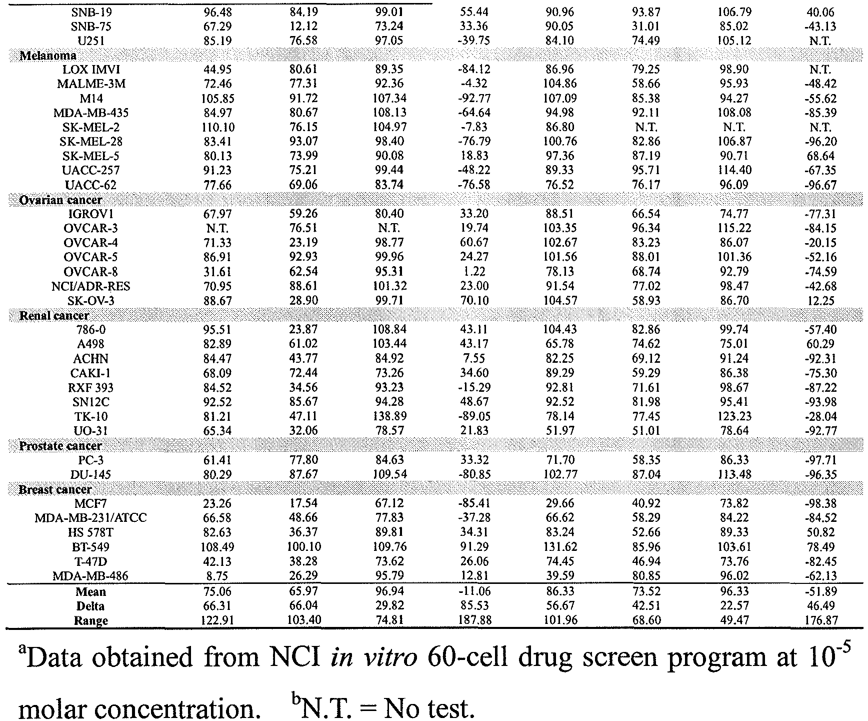

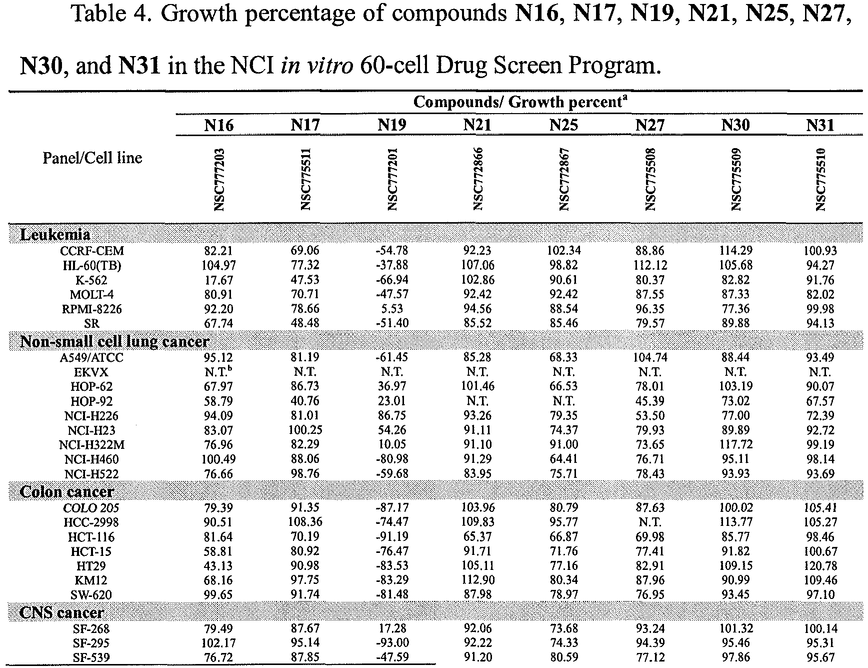

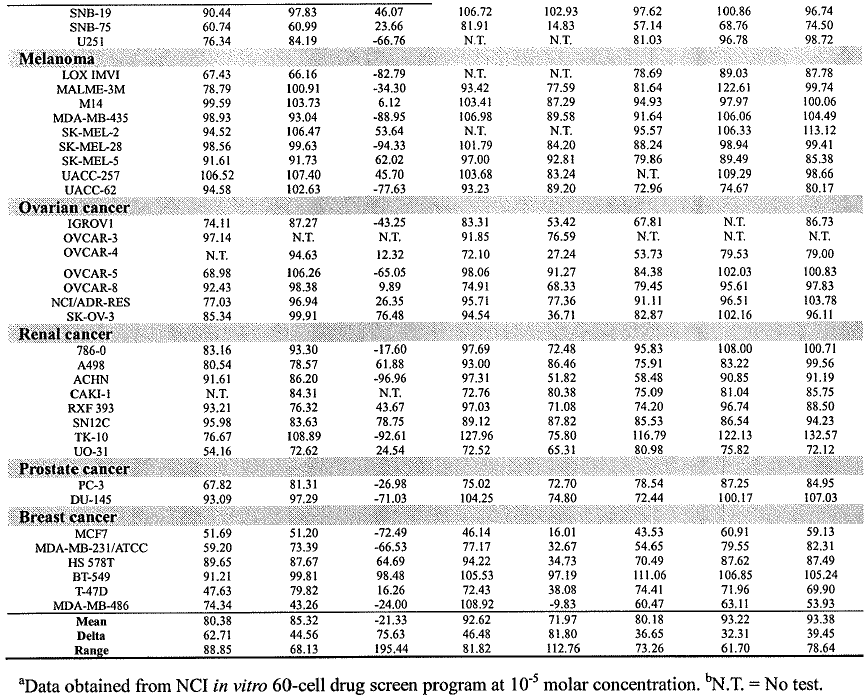

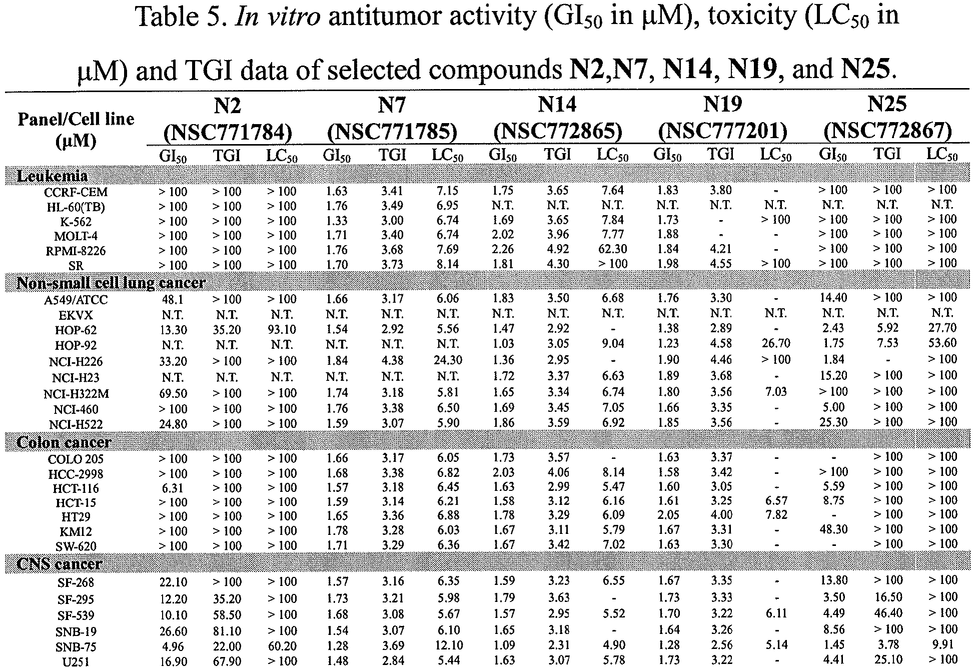

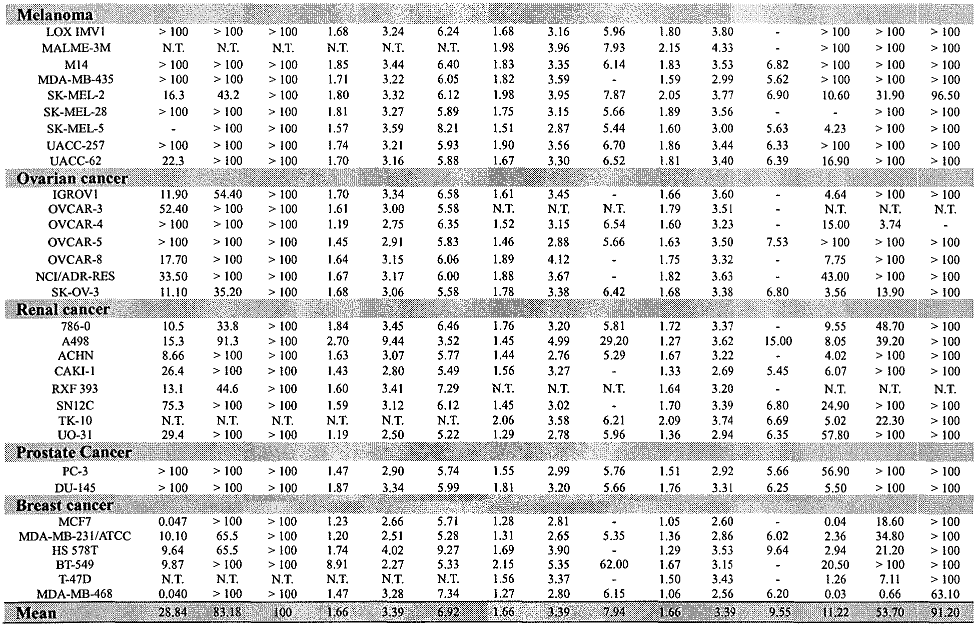

## Claims

1. A compound as shown in formulation (I): 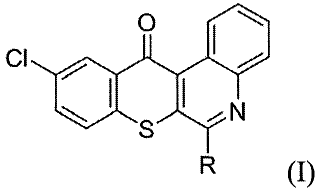 wherein the R is selected from the groups consisting of:
i) halo, amino, hydroxyl and thiol groups;
ii) nitrogen-containing linear alkyl chains of NH(CH₂)ₙH, alkyl groups with substituted side chains, alkyl side chains with a substituted amino group and alkyl side chains with a substituted hydroxyl group, wherein 1 ≤ n ≤ 10;
iii) O(CH₂)ₙH, N(CH₃)₂, NH(CH₂)ₙNH(CH₂)ₙOH, wherein 1 ≤ n ≤ 10;
iv) nitrogen-containing cycloalkyl groups and heterocyclic compounds of C₃₋₁₂ which contain 1 to 3 heteroatoms selected from the group consisting of O, S and N, wherein the ortho-, para- and meta- position can be further selected independently from one of the groups consisting of: hydrogen group, (CH₂)ₙH alkyl groups, (CH₂)ₙ hydroxyl groups, (CH₂)ₙ C₃₋₁₂ cycloalkyl groups, (CH₂)ₙC₃₋₁₂ nitrogen-containing cycloalkyl groups, (CH₂)ₙ benzene rings, formyl group and (CH₂)ₙCO C₃₋₁₂ nitrogen-containing cycloalkyl groups, wherein 0 ≤ n ≤ 10;
v) NH(CH₂)ₙR₁, 0 ≤ n ≤ 10, wherein R₁ is selected from the groups consisting of: N(CH₃)₂, C(NH₂)₂, linear alkyl chains of NH(CH₂)ₙH, alkyl groups with substituted side chains, alkyl side chains with a substituted amino group and alkyl side chains with a substituted hydroxyl group;
vi) NH(CH₂)ₙR₂, 0 ≤ n ≤ 10, wherein R₂ is selected from the groups consisting of: benzene rings, C₃₋₁₂ cycloalkyl groups and heterocyclic groups of which contain 1 to 3 heteroatoms selected from the group consisting of O, S and N, wherein the ortho-, para- and meta- position can be further selected independently from one of the groups consisting of: Methoxyl group, amino group, benzene rings, alkyl, amino, nitro,hydroxyl groups with substituted C1-C3 side chains and C₃₋₁₂ heterocyclic groups; wherein the C₃₋₁₂ heterocyclic groups which contain 1 to 3 heteroatoms selected from the group consisting of O, S and N;
and their pharmaceutically acceptable salts, stereoisomers and enantiomers.

2. The compound according to claim 1, wherein the R group consisting of i) ∼ vi) are selected from the group consisting of chlorine, hydroxyl, methoxyl, dimethylamino, piperazin-1-yl, 4-methylpiperazin-1-yl, 4-ethylpiperazin-1-yl, 4-(2-hydroxyethyl)piperazin-1-yl, 4-Benzylpiperazin-1-yl, 4-phenylpiperazin-1-yl, morpholino, thiomorpholino, piperidin-1-yl, 4-hydroxypiperidin-1-yl, 4-Benzylpiperidin-1-yl, (1,4'-Bipiperidin)-1'-yl, 4-(3-(piperidin-4-yl)propyl)piperidin-1-yl, pyrrolidin-1-yl, 2-oxopiperidin-1-yl, methylamino, ethylamino, propylamino, butylamino, isobutylamino, pentan-3-ylamino, (2-(dimethylamino)ethyl)amino, (2-(diethylamino)ethyl)amino, 2-ethanolamino, 3-propanolamino, 5-pentanolamino, (1-hydroxybutan-2-yl)amino, (4-methylpentan-2-yl)amino, (2-Aminoethyl)amino, (2-((2-hydroxyethyl)amino)ethyl)amino, (2-morpholinoethyl)amino, (3-(dimethylamino)propyl)amino, (3-(diethylamino)propyl)amino, (3-((2-hydroxyethyl)amino)propyl)amino, (2,3-dihydro-1H-inden-2-yl)amino, cyclohexylamino, (1-Benzylpiperidin-4-yl)amino, (thiophen-2-ylmethyl)amino, (cyclohexylmethyl)amino, benzylamino, (pyridin-2-ylmethyl)amino, (Benzo[d][1,3]dioxol-5-ylmethyl)amino, (2-methoxybenzyl)amino, (3,4-dimethoxybenzyl)amino, phenethylamino, (4-methoxyphenethyl)amino, (4-aminophenethyl)amino, guanidine and piperidin-1-ylamino.

3. The compound according to claim 1, wherein the compound is selected from the group consisting of:
3-((4-Chlorophenyl)thio)-2-hydroxyquinoline-4-carboxylic acid,
6,9-Dichloro-12H-thiochromeno[2,3-c]quinolin-12-one,
10-Chloro-6-hydroxy-12H-thiochromeno[2,3-c]quinolin-12-one,
10-Chloro-6-methoxy-12H-thiochromeno[2,3-c]quinolin-12-one
10-Chloro-6-dimethylamino-12H-thiochromeno[2,3-c]quinolin-12-one,
10-Chloro-6-(piperazin-1-yl)-12H-thiochromeno[2,3-c]quinolin-12-one,
10-Chloro-6-(4-methylpiperazin-1-yl)-12H-thiochromeno[2,3-c] quinolin-12-one,
10-Chloro-6-(4-ethylpiperazin-1-yl)-12H-thiochromeno[2,3-c]q uinolin-12-one,
10-Chloro-6-(4-(2-hydroxyethyl)piperazin-1-yl)-12H-thiochrom eno[2,3-c]quinolin-12one,
6-(4-Benzylpiperazin-1-yl)-10-chloro-12H-thiochromeno[2,3-c] quinolin-12-one,
10-Chloro-6-(4-phenylpiperazin-1-yl)-12H-thiochromeno[2,3-c] quinolin-12-one,
10-Chloro-6-morpholino-12H-thiochromeno[2,3-c]quinolin-12-one,
10-Chloro-6-thiomorpholino-12H-thiochromeno[2,3-c]quinolin-12-one,
10-Chloro-6-(piperidin-1-yl)-12H-thiochromeno[2,3-c]quinolin-12-one,
10-Chloro-6-(4-hydroxypiperidin-1-yl)-12H-thiochromeno[2,3-c ]quinolin-12-one,
6-(4-Benzylpiperidin-1-yl)-10-chloro-12H-thiochromeno[2,3-c] quinolin-12-one,
6-([1,4'-Bipiperidin]-1'-yl)-10-chloro-12H-thiochromeno[2,3-c]q uinolin-12-one,
10-Chloro-6-(4-(3-(piperidin-4-yl)propyl)piperidin-1-yl)-12H-th iochromeno[2,3-c]quinolin-12-one,
10-Chloro-6-(pyrrolidin-1-yl)-12H-thiochromeno[2,3-c]quinolin -12-one,
10-Chloro-6-(2-oxopiperidin-1-yl)-12H-thiochromeno[2,3-c]qui nolin-12-one,
10-Chloro-6-methylamino-12H-thiochromeno[2,3-c]quinolin-12 -one,
10-Chloro-6-ethylamino-12H-thiochromeno[2,3-c]quinolin-12-o ne,
10-Chloro-6-propylamino-12H-thiochromeno[2,3-c]quinolin-12 -one,
6-(Butylamino)-10-chloro-12H-thiochromeno[2,3-c]quinolin-12 -one,
10-Chloro-6-isobutylamino-12H-thiochromeno[2,3-c]quinolin-1 2-one,
10-Chloro-6-(pentan-3-ylamino)-12H-thiochromeno[2,3-c]quino lin-12-one,
10-Chloro-6-((2-(dimethylamino)ethyl)amino)-12H-thiochrome no[2,3-c]quinolin-12-one,
10-Chloro-6-((2-(diethylamino)ethyl)amino)-12H-thiochromeno [2,3-c]quinolin-12-one,
10-Chloro-6-(2-ethanolamino)-12H-thiochromeno[2,3-c]quinoli n-12-one,
10-Chloro-6-(3-propanolamino)-12H-thiochromeno[2,3-c]quino lin-12-one,
10-Chloro-6-(5-pentanolamino)-12H-thiochromeno[2,3-c]quinol in-12-one,
10-Chloro-6-((1-hydroxybutan-2-yl)amino)-12H-thiochromeno[ 2,3-c]quinolin-12-one,
10-Chloro-6-((4-methylpentan-2-yl)amino)-12H-thiochromeno[ 2,3-c]quinolin-12-one,
6-((2-Aminoethyl)amino)-10-chloro-12H-thiochromeno[2,3-c]q uinolin-12-one,
10-Chloro-6-((2-((2-hydroxyethyl)amino)ethyl)amino)-12H-thio chromeno[2,3-c]quinolin-12-one,
10-Chloro-6-((2-morpholinoethyl)amino)-12H-thiochromeno[2, 3-c]quinolin-12-one,
10-Chloro-6-((3-(dimethylamino)propyl)amino)-12H-thiochrom eno[2,3-c]quinolin-12-one,
10-Chloro-6-((3-(diethylamino)propyl)amino)-12H-thiochromen o[2,3-c]quinolin-12-one,
10-Chloro-6-((3-((2-hydroxyethyl)amino)propyl)amino)-12H-th iochromeno[2,3-c]quinolin-12-one,
10-Chloro-6-((2,3-dihydro-1H-inden-2-yl)amino)-12H-thiochro meno[2,3-c]quinolin-12-one,
10-Chloro-6-(cyclohexylamino)-12H-thiochromeno[2,3-c]quino lin-12-one,
6-((1-Benzylpiperidin-4-yl)amino)-10-chloro-12H-thiochromen o[2,3-c]quinolin-12-one,
10-Chloro-6-((thiophen-2-ylmethyl)amino)-12H-thiochromeno[ 2,3-c]quinolin-12-one,
10-Chloro-6-((cyclohexylmethyl)amino)-12H-thiochromeno[2,3 -c]quinolin-12-one,
6-(Benzylamino)-10-chloro-12H-thiochromeno[2,3-c]quinolin-1 2-one,
10-Chloro-6-((pyridin-2-ylmethyl)amino)-12H-thiochromeno[2, 3-c]quinolin-12-one,
6-((Benzo[d][1,3]dioxol-5-ylmethyl)amino)-10-chloro-12H-thio chromeno[2,3-c] quinolin-12-one,
10-Chloro-6-((2-methoxybenzyl)amino)-12H-thiochromeno[2,3-c]quinolin-12-one,
10-Chloro-6-((3,4-dimethoxybenzyl)amino)-12H-thiochromeno[ 2,3-c]quinolin-12-one,
10-Chloro-6-(phenethylamino)-12H-thiochromeno[2,3-c]quinoli n-12-one,
10-Chloro-6-((4-methoxyphenethyl)amino)-12H-thiochromeno[ 2,3-c]quinolin-12-one,
6-((4-Aminophenethyl)amino)-10-chloro-12H-thiochromeno[2,3 -c]quinolin-12-one,
2-(10-Chloro-12-oxo-12*H-*thiochromeno[2,3-*c*]quinolin-6-yl)gu anidine,
10-Chloro-6-(piperidin-1-ylamino)-12*H*-thiochromeno[2,3-*c*]qui nolin-12-one,
and their salts.

4. The compound according to claim 1 for use in a method for inhibiting the Topoisomerase I activity of cancer cell, the method comprising administrating a effective amount of the compound according to claim 1.

5. The compound according to claim 1 for use in a method for inhibiting the Topoisomerase II activity of cancer cell, the method comprising administrating an effective amount of the compound according to claim 1.

6. The compound according to claim 1 for use in a method for the treatment of cancer, the method comprising administrating an effective amount of said compound.

7. The compound for use according to claim 6, wherein the cancer is selected from the groups consisting of leukemia, non-small cell lung cancer, colorectal cancer, central nervous system (CNS) cancer, melanoma, ovarian cancer, renal cancer, prostate cancer and breast cancer.

8. A pharmaceutical composition comprising an effective amount of the compound according to claim 1 and at least one pharmaceutically acceptable vehicle, diluent or excipient.

9. A method for preparation of thiochromeno[2,3-*c*]quinolin-12-one derivatives according to claim 1, the method comprising:
(1) mix isatin, 2-((4-chlorophenyl)thio)acetic acid and sodium acetate was heated at 150 °C for 1 h, after cooling the mixture was added acetic acid, the precipitate was collected, washed with acetic acid, water and n-hexane, and obtained compound 2 (3-((4-Chlorophenyl)thio)-2-hydroxyquinoline-4-carboxylic acid);
(2) a solution of compound 2 (3-((4-Chlorophenyl)thio)-2-hydroxyquinoline-4-carboxylic acid) in phosphoryl trichloride was heated at 150 °C for 48 h, after cooling the mixture was poured into water 0 °C, the precipitate was collected by filtration, then added into 10% NaHCO₃ with vigorous stirring for 1 h, the resulting precipitate was collected and washed with H₂O, the crude solid was recrystallized by dichloromethane to give compound 3 (6,9-Dichloro-12H-thiochromeno[2,3-c] quinolin-12-one);
(3) a solution of compound 3 (6,9-Dichloro-12H-thiochromeno [2,3-c]quinolin-12-one) in DMF was added conc. HCl and refluxed, after 6 hours, the conc. HCl was added dropwise and refluxed for another 12 hours, the mixture was evaporated in vacuo and treated with H₂O, after filtered the crude solid was washed with EtOH to give compound 4 (10-Chloro-6-hydroxy-12H-thiochromeno[2,3-c]quinolin-12-one);
(4) a suspension of compound 3 (6,9-Dichloro-12H-thiochromeno[2,3-c]quinolin-12-one) and sodium methoxide in methanol was refluxed for 16 h, after cooled the solvent was removed, filtrated and washed with ethanol and n-hexane to collect compound 5 (10-Chloro-6-methoxy-12H-thiochromeno[2,3-c]quinolin-12-one);
(5) a solution of compound 3 (6,9-Dichloro-12H-thiochromeno[2,3-c]quinolin-12-one), appropriate secondary amines and sodium carbonate in DMSO was refluxed for 10 hours, then the reaction was added ice-water, the precipitate was filtered, washed with water/methanol to collect the following compound:
10-Chloro-6-dimethylamino-12H-thiochromeno[2,3-c]quinoli n-12-one,
10-Chloro-6-(piperazin-1-yl)-12H-thiochromeno[2,3-c]quinol in-12-one,
10-Chloro-6-(4-methylpiperazin-1-yl)-12H-thiochromeno[2,3 -c]quinolin-12-one,
10-Chloro-6-(4-ethylpiperazin-1-yl)-12H-thiochromeno[2,3-c ]quinolin-12-one,
10-Chloro-6-(4-(2-hydroxyethyl)piperazin-1-yl)-12H-thiochr omeno[2,3-c]quinolin-12one,
6-(4-Benzylpiperazin-1-yl)-10-chloro-12H-thiochromeno[2,3-c]quinolin-12-one,
10-Chloro-6-(4-phenylpiperazin-1-yl)-12H-thiochromeno[2,3 -c]quinolin-12-one,
10-Chloro-6-morpholino-12H-thiochromeno[2,3-c]quinolin-1 2-one,
10-Chloro-6-thiomorpholino-12H-thiochromeno[2,3-c]quinol in-12-one,
10-Chloro-6-(piperidin-1-yl)-12H-thiochromeno[2,3-c]quinol in-12-one,
10-Chloro-6-(4-hydroxypiperidin-1-yl)-12H-thiochromeno[2, 3-c]quinolin-12-one,
6-(4-Benzylpiperidin-1-yl)-10-chloro-12H-thiochromeno[2,3-c]quinolin-12-one,
6-([1,4'-Bipiperidin]-1'-yl)-10-chloro-12H-thiochromeno[2,3-c]quinolin-12-one,
10-Chloro-6-(4-(3-(piperidin-4-yl)propyl)piperidin-1-yl)-12H -thiochromeno[2,3-c]quinolin-12-one,
10-Chloro-6-(pyrrolidin-1-yl)-12H-thiochromeno[2,3-c]quino lin-12-one, or
10-Chloro-6-(2-oxopiperidin-1-yl)-12H-thiochromeno[2,3-c] quinolin-12-one respectively;
(6) a solution of compound 3 (6,9-Dichloro-12H-thiochromeno[2,3-c]quinolin-12-one) in DMSO was added appropriate primary amines and refluxed for 8 hours, after cooled the reaction was added water, the precipitate was filtered and washed with water and methanol to collect the following compound:
10-Chloro-6-methylamino-12H-thiochromeno[2,3-c]quinolin-12-one,
10-Chloro-6-ethylamino-12H-thiochromeno[2,3-c]quinolin-1 2-one,
10-Chloro-6-propylamino-12H-thiochromeno[2,3-c]quinolin-12-one,
6-(Butylamino)-10-chloro-12H-thiochromeno[2,3-c]quinolin-12-one,
10-Chloro-6-isobutylamino-12H-thiochromeno[2,3-c]quinoli n-12-one,
10-Chloro-6-(pentan-3-ylamino)-12H-thiochromeno[2,3-c]qu inolin-12-one,
10-Chloro-6-((2-(dimethylamino)ethyl)amino)-12H-thiochro meno[2,3-c]quinolin-12-one,
10-Chloro-6-((2-(diethylamino)ethyl)amino)-12H-thiochrome no[2,3-c]quinolin-12-one,
10-Chloro-6-(2-ethanolamino)-12H-thiochromeno[2,3-c]quin olin-12-one,
10-Chloro-6-(3-propanolamino)-12H-thiochromeno[2,3-c]qui nolin-12-one,
10-Chloro-6-(5-pentanolamino)-12H-thiochromeno[2,3-c]qui nolin-12-one,
10-Chloro-6-((1-hydroxybutan-2-yl)amino)-12H-thiochromen o[2,3-c]quinolin-12-one,
10-Chloro-6-((4-methylpentan-2-yl)amino)-12H-thiochromen o[2,3-c]quinolin-12-one,
6-((2-Aminoethyl)amino)-10-chloro-12H-thiochromeno[2,3-c ]quinolin-12-one,
10-Chloro-6-((2-((2-hydroxyethyl)amino)ethyl)amino)-12H-t hiochromeno[2,3-c]quinolin-12-one,
10-Chloro-6-((2-morpholinoethyl)amino)-12H-thiochromeno[ 2,3-c]quinolin-12-one,
10-Chloro-6-((3-(dimethylamino)propyl)amino)-12H-thiochr omeno[2,3-c]quinolin-12-one,
10-Chloro-6-((3-(diethylamino)propyl)amino)-12H-thiochro meno[2,3-c]quinolin-12-one,
10-Chloro-6-((3-((2-hydroxyethyl)amino)propyl)amino)-12H -thiochromeno[2,3-c]quinolin-12-one,
10-Chloro-6-((2,3-dihydro-1H-inden-2-yl)amino)-12H-thioch romeno[2,3-c]quinolin-12-one,
10-Chloro-6-(cyclohexylamino)-12H-thiochromeno[2,3-c]qui nolin-12-one,
6-((1-Benzylpiperidin-4-yl)amino)-10-chloro-12H-thiochrom eno[2,3-c]quinolin-12-one,
10-Chloro-6-((thiophen-2-ylmethyl)amino)-12H-thiochromen o[2,3-c]quinolin-12-one,
10-Chloro-6-((cyclohexylmethyl)amino)-12H-thiochromeno[ 2,3-c]quinolin-12-one,
6-(Benzylamino)-10-chloro-12H-thiochromeno[2,3-c]quinoli n-12-one,
10-Chloro-6-((pyridin-2-ylmethyl)amino)-12H-thiochromeno [2,3-c]quinolin-12-one,
6-((Benzo[d][1,3]dioxol-5-ylmethyl)amino)-10-chloro-12H-t hiochromeno[2,3-c]quinolin-12-one,
10-Chloro-6-((2-methoxybenzyl)amino)-12H-thiochromeno[2 ,3-c]quinolin-12-one,
10-Chloro-6-((3,4-dimethoxybenzyl)amino)-12H-thiochrome no[2,3-c]quinolin-12-one,
10-Chloro-6-(phenethylamino)-12H-thiochromeno[2,3-c]quin olin-12-one,
10-Chloro-6-((4-methoxyphenethyl)amino)-12H-thiochromen o[2,3-c]quinolin-12-one,
6-((4-Aminophenethyl)amino)-10-chloro-12H-thiochromeno[ 2,3-c]quinolin-12-one,
2-(10-Chloro-12-oxo-12*H-*thiochromeno[2,3-c]quinolin-6-yl) guanidine, or
10-Chloro-6-(piperidin-1-ylamino)-12*H-*thiochromeno[2,3-*c*] quinolin-12-one respectively.

## Patentansprüche

1. Verbindung wie in Formel (I) gezeigt: 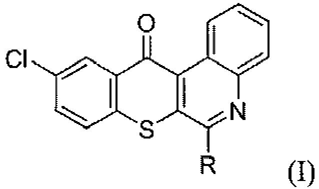 wobei R ausgewählt ist aus den Gruppen bestehend aus:
i) Halogen-, Amino-, Hydroxyl- und Thiolgruppen;
ii) stickstoffhaltigen linearen Alkylgruppen von NH(CH₂)ₙH, Alkylgruppen mit substituierten Seitenketten, Alkylseitenketten mit einer substituierten Aminogruppe und Alkylseitenketten mit einer substituierten Hydroxylgruppe, wobei 1 ≤ n ≤ 10;
iii) O (CH₂)ₙH, N(CH₃)₂, NH(CH₂)ₙNH(CH₂)ₙOH, wobei 1 ≤ n ≤ 10;
iv) stickstoffhaltigen Cycloalkylgruppen und heterocyclischen Verbindungen von C₃₋₁₂, die 1 bis 3 Heteroatome ausgewählt aus der Gruppe bestehend aus O, S und N enthalten, wobei die ortho-, para- und meta-Position des Weiteren unabhängig ausgewählt sein kann aus einer der Gruppen bestehend aus: Wasserstoffgruppe, (CH₂)ₙH-Alkylgruppen, (CH₂)ₙ-Hydroxylgruppen, (CH₂)ₙC₃₋₁₂-Cycloalkylgruppen, stickstoffhaltigen (CH₂)ₙC₃₋₁₂-Cycloalkylgruppen, (CH₂)ₙ-Benzolringen, Formylgruppe und stickstoffhaltigen (CH₂)ₙCO-C₃₋₁₂-Cycloalkylgruppen, wobei 0 ≤ n ≤ 10;
v) NH(CH₂)ₙR₁, 0 ≤ n ≤ 10, wobei R₁ ausgewählt ist aus den Gruppen bestehend aus: N(CH₃)₂, C(NH₂)₂, linearen Alkylketten von NH(CH₂)ₙH, Alkylgruppen mit substituierten Seitenketten, Alkylseitenketten mit einer substituierten Aminogruppe und Alkylseitenketten mit einer substituierten Hydroxylgruppe;
vi) NH(CH₂)ₙR₂, 0 ≤ n ≤ 10, wobei R₂ ausgewählt ist aus den Gruppen bestehend aus: Benzolringen, C₃₋₁₂-Cycloalkylgruppen und heterocyclischen Gruppen, die 1 bis 3 Heteroatome ausgewählt aus der Gruppe bestehend aus O, S und N enthalten, wobei die ortho-, para- und meta-Position des Weiteren unabhängig ausgewählt sein kann aus einer der Gruppen bestehend aus: Methoxylgruppe, Aminogruppe, Benzolringen, Alkyl-, Amino-, Nitro-, Hydroxylgruppen mit substituierten C₁-C₃-Seitenketten und heterocyclischen C₃₋₁₂-Gruppen; wobei die heterocyclischen C₃₋₁₂-Gruppen 1 bis 3 Heteroatome ausgewählt aus der Gruppe bestehend aus O, S und N enthalten;
und deren pharmazeutisch annehmbaren Salze, Stereoisomere und Enantiomere.

2. Verbindung nach Anspruch 1, wobei die R-Gruppe, die aus i) bis vi) besteht, ausgewählt ist aus der Gruppe bestehend aus Chlor, Hydroxyl, Methoxyl, Dimethylamino, Piperazin-1-yl, 4-Methylpiperazin-1-yl, 4-Ethylpiperazin-1-yl, 4-(2-Hydroxyethyl)piperazin-1-yl, 4-Benzylpiperazin-1-yl, 4-Phenylpiperazin-1-yl, Morpholino, Thiomorpholino, Piperidin-1-yl, 4-Hydroxypiperidin-1-yl, 4-Benzylpiperidin-1-yl, (1,4'-Bipiperidin)-1'-yl, 4-(3-(Piperidin-4-yl)propyl)piperidin-1-yl, Pyrrolidin-1-yl, 2-Oxopiperidin-1-yl, Methylamino, Ethylamino, Propylamino, Butylamino, Isobutylamino, Pentan-3-ylamino, (2-(Dimethylamino)ethyl)amino, (2-(Diethylamino)ethyl)amino, 2-Ethanolamino, 3-Propanolamino, 5-Pentanolamino, (1-Hydroxybutan-2-yl)amino, (4-Methylpentan-2-yl)amino, (2-Aminoethyl)amino, (2-((2-Hydroxyethyl)amino)ethyl)amino, (2-Morpholinoethyl)amino, (3-(Dimethylamino)propyl)amino, (3-(Diethylamino)propyl)amino, (3-((2-Hydroxyethyl)-amino)propyl)amino, (2,3-Dihydro-1H-inden-2-yl)-amino, Cyclohexylamino, (1-Benzylpiperidin-4-yl)-amino, (Thiophen-2-ylmethyl)amino, (Cyclohexylmethyl)amino, Benzylamino, (Pyridin-2-ylmethyl)amino, (Benzo[d][1,3]dioxol-5-ylmethyl)amino, (2-Methoxybenzyl)amino, (3,4-Dimethoxybenzyl)amino, Phenethylamino, (4-Methoxyphenethyl)amino, (4-Aminophenethyl)amino, Guanidin und Piperidin-1-ylamino.

3. Verbindung nach Anspruch 1, wobei die Verbindung ausgewählt ist aus der Gruppe bestehend aus:
3-((4-Chlorphenyl)thio)-2-hydroxychinolin-4-carbonsäure, 6,9-Dichlor-12H-thiochromeno[2,3-c]chinolin-12-on, 10-Chlor-6-hydroxy-12H-thiochromeno-[2,3-c]chinolin-12-on, 10-Chlor-6-methoxy-12H-thiochromeno[2,3-c]chinolin-12-on, 10-Chlor-6-dimethylamino-12H-thiochromeno[2,3-c]chinolin-12-on, 10-Chlor-6-(piperazin-1-yl)-12H-thiochromeno[2,3-c]chinolin-12-on, 10-Chlor-6-(4-methylpiperazin-1-yl)-12H-thiochromeno[2,3-c]chinolin-12-on, 10-Chlor-6-(4-ethylpiperazin-1-yl)-12H-thiochromeno-[2,3-c]chinolin-12-on, 10-Chlor-6-(4-(2-hydroxyethyl)piperazin-1-yl)-12H-thiochromeno[2,3-c]chinolin-12-on, 6-(4-Benzylpiperazin-1-yl)-10-chlor-12H-thiochromeno[2,3-c]chinolin-12-on, 10-Chlor-6-(4-phenylpiperazin-1-yl)-12H-thiochromeno[2,3-c]-chinolin-12-on, 10-Chlor-6-morpholino-12H-thiochromeno[2,3-c]chinolin-12-on, 10-Chlor-6-thiomorpholino-12H-thiochromeno[2,3-c]chinolin-12-on, 10-Chlor-6-(piperidin-1-yl)-12H-thiochromeno[2,3-c]-chinolin-12-on, 10-Chlor-6-(4-hydroxypiperidin-1-yl)-12H-thiochromeno[2,3-c]chinolin-12-on, 6-(4-Benzylpiperidin-1-yl)-10-chlor-12H-thiochromeno-[2,3-c]chinolin-12-on, 6-([1,4'-Bipiperidin]-1'-yl)-10-chlor-12H-thiochromeno[2,3-c]chinolin-12-on, 10-Chlor-6-(4-(3-(piperidin-4-yl)propyl)piperidin-1-yl)-12H-thiochromeno[2,3-c]chinolin-12-on, 10-Chlor-6-(pyrrolidin-1-yl)-12H-thiochromeno[2,3-c]chinolin-12-on, 10-Chlor-6-(2-oxopiperidin-1-yl)-12H-thiochromeno[2,3-c]chinolin-12-on, 10-Chlor-6-methylamino-12H-thiochromeno[2,3-c]chinolin-12-on, 10-Chlor-6-ethylamino-12H-thiochromeno-[2,3-c]chinolin-12-on, 10-Chlor-6-propylamino-12H-thiochromeno[2,3-c]chinolin-12-on, 6-(Butylamino)-10-chlor-12H-thiochromeno[2,3-c]chinolin-12-on, 10-Chlor-6-isobutylamino-12H-thiochromeno[2,3-c]-chinolin-12-on, 10-Chlor-6-(pentan-3-ylamino)-12H-thiochromeno[2,3-c]chinolin-12-on, 10-Chlor-6-((2-(dimethylamino)ethyl)amino)-12H-thiochromeno[2,3-c]chinolin-12-on, 10-Chlor-6-((2-(diethylamino)-ethyl)amino)-12H-thiochromeno[2,3-c]chinolin-12-on, 10-Chlor-6-(2-ethanolamino)-12H-thiochromeno-[2,3-c]chinolin-12-on, 10-Chlor-6-(3-propanolamino)-12H-thiochromeno[2,3-c]chinolin-12-on, 10-Chlor-6-(5-pentanolamino)-12H-thiochromeno[2,3-c]-chinolin-12-on, 10-Chlor-6-((1-hydroxybutan-2-yl)-amino)-12H-thiochromeno[2,3-c]chinolin-12-on, 10-Chlor-6-((4-methylpentan-2-yl)amino)-12H-thiochromeno[2,3-c]chinolin-12-on, 6-((2-Aminoethyl)amino)-10-chlor-12H-thiochromeno[2,3-c]chinolin-12-on, 10-Chlor-6-((2-((2-hydroxyethyl)amino)ethyl)-amino)-12H-thiochromeno[2,3-c]chinolin-12-on, 10-Chlor-6-((2-morpholinoethyl)amino)-12H-thiochromeno[2,3-c]chinolin-12-on, 10-Chlor-6-((3-(dimethylamino)propyl)amino)-12H-thiochromeno[2,3-c]chinolin-12-on, 10-Chlor-6-((3-(diethylamino)propyl)-amino)-12H-thiochromeno[2,3-c]chinolin-12-on, 10-Chlor-6-((3-((2-hydroxyethyl)amino)propyl)amino)-12H-thiochromeno[2,3-c]chinolin-12-on, 10-Chlor-6-((2,3-dihydro-1H-inden-2-yl)amino)-12H-thiochromeno[2,3-c]chinolin-12-on, 10-Chlor-6-(cyclohexylamino)-12H-thiochromeno[2,3-c]chinolin-12-on, 6-((1-Benzylpiperidin-4-yl)amino)-10-chlor-12H-thiochromeno[2,3-c]chinolin-12-on, 10-Chlor-6-((thiophen-2-ylmethyl)amino)-12H-thiochromeno[2,3-c]chinolin-12-on, 10-Chlor-6-((cyclohexylmethyl)amino)-12H-thiochromeno[2,3-c]chinolin-12-on, 6-(Benzylamino)-10-chlor-12H-thiochromeno[2,3-c]chinolin-12-on, 10-Chlor-6-((pyridin-2-ylmethyl)amino)-12H-thiochromeno[2,3-c]chinolin-12-on, 6-((Benzo[d]-[1,3]dioxol-5-ylmethyl)amino)-10-chlor-12H-thiochromeno[2,3-c]chinolin-12-on, 10-Chlor-6-((2-methoxybenzyl)amino)-12H-thiochromeno[2,3-c]chinolin-12-on, 10-Chlor-6-((3,4-dimethoxybenzyl)amino)-12H-thiochromeno[2,3-c]chinolin-12-on, 10-Chlor-6-(phenethylamino)-12H-thiochromeno[2,3-c]-chinolin-12-on, 10-Chlor-6-((4-methoxyphenethyl)-amino)-12H-thiochromeno[2,3-c]chinolin-12-on, 6-((4-Aminophenethyl)amino)-10-chlor-12H-thiochromeno[2,3-c]chinolin-12-on, 2-(10-Chlor-12-oxo-12*H-*thiochromeno[2,3-c]chinolin-6-yl)guanidin, 10-Chlor-6-(piperidin-1-ylamino)-12*H*-thiochromeno-[2,3-c]chinolin-12-on, und deren Salze.

4. Verbindung nach Anspruch 1 zur Verwendung in einem Verfahren zum Hemmen der Topoisomerase I-Aktivität von Krebszellen, wobei das Verfahren Verabreichen einer effektiven Menge der Verbindung gemäß Anspruch 1 umfasst.

5. Verbindung nach Anspruch 1 zur Verwendung in einem Verfahren zum Hemmen der Topoisomerase II-Aktivität von Krebszellen, wobei das Verfahren Verabreichen einer effektiven Menge der Verbindung gemäß Anspruch 1 umfasst.

6. Verbindung nach Anspruch 1 zur Verwendung in einem Verfahren zur Behandlung von Krebs, wobei das Verfahren Verabreichen einer effektiven Menge der Verbindung umfasst.

7. Verbindung zur Verwendung nach Anspruch 6, wobei der Krebs ausgewählt ist aus den Gruppen bestehend aus Leukämie, nicht-kleinzelligem Lungenkrebs, Kolorektalkrebs, Krebs des zentralen Nervensystems (ZNS), Melanom, Eierstockkrebs, Nierenkrebs, Prostatakrebs und Brustkrebs.

8. Pharmazeutische Zusammensetzung, umfassend eine effektive Menge der Verbindung nach Anspruch 1 und mindestens ein pharmazeutisch annehmbares Vehikel, Verdünnungsmittel oder einen pharmazeutisch annehmbaren Hilfsstoff.

9. Verfahren zur Herstellung von Derivaten von Thiochromeno[2,3-c]chinolin-12-on gemäß Anspruch 1, wobei das Verfahren umfasst:
(1) Erwärmen einer Mischung von Isatin, 2-((4-Chlorphenyl)thio)essigsäure und Natriumacetat für 1 Stunde auf 150 °C, nach Kühlen der Mischung Zufügen von Essigsäure, Auffangen des Niederschlags, Waschen mit Essigsäure, Wasser und n-Hexan, und Erhalten von Verbindung 2, (3-((4-Chlorphenyl)thio)-2-hydroxychinolin-4-carbonsäure);
(2) Erwärmen einer Lösung von Verbindung 2, (3-((4-Chlorphenyl)thio)-2-hydroxychinolin-4-carbonsäure), in Phosphoryltrichlorid für 48 Stunden auf 150 °C, nach dem Abkühlen Gießen der Mischung in Wasser mit 0 °C, Auffangen des Niederschlags durch Filtration, dann Geben in 10 % NaHCO₃ unter kräftigem Rühren für 1 Stunde, Auffangen des resultierenden Niederschlags und Waschen mit H₂O, Umkristallisieren des rohen Feststoffs aus Dichlormethan, um Verbindung 3 zu ergeben, (6,9-Dichlor-12H-thiochromeno[2,3-c]chinolin-12-on);
(3) Zugeben von konz. HCl zu einer Lösung von Verbindung 3, (6,9-Dichlor-12H-thiochromeno[2,3-c]chinolin-12-on), in DMF und Halten unter Rückfluss, nach 6 Stunden tropfenweises Zufügen der konz. HCl und Halten unter Rückfluss für weitere 12 Stunden, Eindampfen der Mischung im Vakuum und Behandeln mit H₂O, Waschen des rohen Feststoffs mit EtOH nach dem Filtrieren, um Verbindung 4 zu ergeben, (10-Chlor-6-hydroxy-12H-thiochromeno[2,3-c]chinolin-12-on);
(4) Halten einer Suspension von Verbindung 3, (6,9-Dichlor-12H-thiochromeno[2,3-c]chinolin-12-on), und Natriummethoxid in Methanol unter Rückfluss für 16 Stunden, nach dem Abkühlen Entfernen des Lösungsmittels, Filtrieren und Waschen mit Ethanol und n-Hexan, um Verbindung 5 aufzufangen, (10-Chlor-6-methoxy-12H-thiochromeno[2,3-c]chinolin-12-on);
(5) Halten einer Lösung von Verbindung 3, (6,9-Dichlor-12H-thiochromeno[2,3-c]chinolin-12-on), geeigneten sekundären Aminen und Natriumcarbonat in DMSO unter Rückfluss für 10 Stunden, dann Geben von Eiswasser zu der Reaktion, Filtrieren des Niederschlags, Waschen mit Wasser/Methanol, um die folgende Verbindung aufzufangen:
10-Chlor-6-dimethylamino-12H-thiochromeno[2,3-c]chinolin-12-on,
10-Chlor-6-(piperazin-1-yl)-12H-thiochromeno-[2,3-c]chinolin-12-on,
10-Chlor-6-(4-methylpiperazin-1-yl)-12H-thiochromeno[2,3-c]chinolin-12-on,
10-Chlor-6-(4-ethylpiperazin-1-yl)-12H-thiochromeno[2,3-c]chinolin-12-on,
10-Chlor-6-(4-(2-hydroxyethyl)piperazin-1-yl)-12H-thiochromeno[2,3-c]chinolin-12-on,
6-(4-Benzylpiperazin-1-yl)-10-chlor-12H-thiochromeno[2,3-c]chinolin-12-on,
10-Chlor-6-(4-phenylpiperazin-1-yl)-12H-thiochromeno[2,3-c]chinolin-12-on,
10-Chlor-6-morpholino-12H-thiochromeno[2,3-c]-chinolin-12-on,
10-Chlor-6-thiomorpholino-12H-thiochromeno[2,3-c]chinolin-12-on,
10-Chlor-6-(piperidin-1-yl)-12H-thiochromeno-[2,3-c]chinolin-12-on,
10-Chlor-6-(4-hydroxypiperidin-1-yl)-12H-thiochromeno[2,3-c]chinolin-12-on,
6-(4-Benzylpiperidin-1-yl)-10-chlor-12H-thiochromeno[2,3-c]chinolin-12-on,
6-([1,4'-Bipiperidin]-1'-yl)-10-chlor-12H-thiochromeno[2,3-c]chinolin-12-on,
10-Chlor-6-(4-(3-(piperidin-4-yl)propyl)piperidin-1-yl)-12H-thiochromeno[2,3-c]chinolin-12-on,
10-Chlor-6-(pyrrolidin-1-yl)-12H-thiochromeno-[2,3-c]chinolin-12-on beziehungsweise
10-Chlor-6-(2-oxopiperidin-1-yl)-12H-thiochromeno[2,3-c]chinolin-12-on;
(6) Zufügen von geeigneten primären Aminen zu einer Lösung von Verbindung 3, (6,9-Dichlor-12H-thiochromeno[2,3-c]chinolin-12-on), in DMSO und Halten unter Rückfluss für 8 Stunden, nach Abkühlen der Reaktion Zugeben von Wasser, Filtrieren des Niederschlags und Waschen mit Wasser und Methanol, um die folgende Verbindung aufzufangen:
10-Chlor-6-methylamino-12H-thiochromeno[2,3-c]-chinolin-12-on,
10-Chlor-6-ethylamino-12H-thiochromeno[2,3-c]-chinolin-12-on,
10-Chlor-6-propylamino-12H-thiochromeno[2,3-c]-chinolin-12-on,
6-(Butylamino)-10-Chlor-12H-thiochromeno[2,3-c]-chinolin-12-on,
10-Chlor-6-isobutylamino-12H-thiochromeno[2,3-c]chinolin-12-on,
10-Chlor-6-(pentan-3-ylamino)-12H-thiochromeno-[2,3-c]chinolin-12-on,
10-Chlor-6-((2-(dimethylamino)ethyl)amino)-12H-thiochromeno[2,3-c]chinolin-12-on,
10-Chlor-6-((2-(diethylamino)ethyl)amino)-12H-thiochromeno[2,3-c]chinolin-12-on,
10-Chlor-6-(2-ethanolamino)-12H-thiochromeno-[2,3-c]chinolin-12-on,
10-Chlor-6-(3-propanolamino)-12H-thiochromeno-[2,3-c]chinolin-12-on,
10-Chlor-6-(5-pentanolamino)-12H-thiochromeno-[2,3-c]chinolin-12-on,
10-Chlor-6-((1-hydroxybutan-2-yl)amino)-12H-thiochromeno[2,3-c]chinolin-12-on,
10-Chlor-6-((4-methylpentan-2-yl)amino)-12H-thiochromeno[2,3-c]chinolin-12-on,
6-((2-Aminoethyl)amino)-10-Chlor-12H-thiochromeno[2,3-c]chinolin-12-on,
10-Chlor-6-((2-((2-hydroxyethyl)amino)ethyl)amino)-12H-thiochromeno[2,3-c]chinolin-12-on,
10-Chlor-6-((2-morpholinoethyl)amino)-12H-thiochromeno[2,3-c]chinolin-12-on,
10-Chlor-6-((3-(dimethylamino)propyl)amino)-12H-thiochromeno[2,3-c]chinolin-12-on,
10-Chlor-6-((3-(diethylamino)propyl)amino)-12H-thiochromeno[2,3-c]chinolin-12-on,
10-Chlor-6-((3-((2-hydroxyethyl)amino)propyl)-amino)-12H-thiochromeno[2,3-c]chinolin-12-on, 10-Chlor-6-((2,3-dihydro-1H-inden-2-yl)amino)-12H-thiochromeno[2,3-c]chinolin-12-on,
10-Chlor-6-(cyclohexylamino)-12H-thiochromeno-[2,3-c]chinolin-12-on,
6-((1-Benzylpiperidin-4-yl)amino)-10-chlor-12H-thiochromeno[2,3-c]chinolin-12-on,
10-Chlor-6-((thiophen-2-ylmethyl)amino)-12H-thiochromeno[2,3-c]chinolin-12-on,
10-Chlor-6-((cyclohexylmethyl)amino)-12H-thiochromeno[2,3-c]-chinolin-12-on,
6-(Benzylamino)-10-Chlor-12H-thiochromeno[2,3-c]chinolin-12-on,
10-Chlor-6-((pyridin-2-ylmethyl)amino)-12H-thiochromeno[2,3-c]chinolin-12-on,
6-((Benzo[d][1,3]dioxol-5-ylmethyl)amino)-10-chlor-12H-thiochromeno[2,3-c]chinolin-12-on,
10-Chlor-6-((2-methoxybenzyl)amino)-12H-thiochromeno[2,3-c]chinolin-12-on,
10-Chlor-6-((3,4-dimethoxybenzyl)amino)-12H-thiochromeno[2,3-c]chinolin-12-on,
10-Chlor-6-(phenethylamino)-12H-thiochromeno-[2,3-c]chinolin-12-on,
10-Chlor-6-((4-methoxyphenethyl)amino)-12H-thiochromeno[2,3-c]chinolin-12-on,
6-((4-Aminophenethyl)amino)-10-chlor-12H-thiochromeno[2,3-c]chinolin-12-on,
2-(10-Chlor-12-oxo-12*H*-thiochromeno[2,3-c]chinolin-6-yl)guanidin beziehungsweise
10-Chlor-6-(piperidin-1-ylamino)-12*H*-thiochromeno[2,3-c]chinolin-12-on.

## Revendications

1. Composé tel que représenté dans la formule (I) : 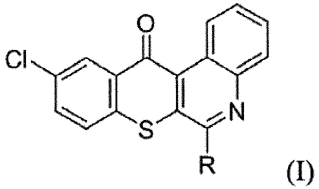 dans lequel le R est choisi dans les groupes constitués par :
i) les groupes halogéno, amino, hydroxyle et thiol ;
ii) les chaînes alkyle linéaires contenant de l'azote de NH(CH₂)ₙH, les groupes alkyle portant des chaînes latérales substituées, des chaînes latérales alkyle portant un groupe amino substitué et des chaînes latérales alkyle portant un groupe hydroxyle substitué, avec 1 ≤ n ≤ 10 ;
iii) O (CH₂)ₙH, N(CH₃)₂, NH(CH₂)ₙNH(CH₂)ₙOH, avec 1 ≤ n ≤ 10 ;
iv) les groupes cycloalkyle contenant de l'azote et les composés hétérocycliques en C₃₋₁₂ qui contiennent 1 à 3 hétéroatomes choisis dans le groupe constitué par O, S et N, les positions ortho, para et méta pouvant être en outre choisies indépendamment dans l'un des groupes constitués par : le groupe hydrogène, les groupes alkyle (CH₂)ₙH, les groupes (CH₂)ₙ-hydroxyle, les groupes (CH₂)ₙ- (cycloalkyle en C₃₋₁₂) , les groupes (CH₂)ₙ-(cycloalkyle en C₃₋₁₂ contenant de l'azote), les noyaux (CH₂)ₙ-benzène, le groupe formyle et les groupes (CH₂)ₙCO-(cycloalkyle en C₃₋₁₂ contenant de l'azote), avec 0 ≤ n ≤ 10 ;
v) NH(CH₂)ₙR₁, avec 0 ≤ n ≤ 10, R₁ étant choisi dans le groupe constitué par :N(CH₃)₂, C(NH₂)₂, les chaînes alkyle linéaires de NH(CH₂)ₙH, les groupes alkyle portant des chaînes latérales substituées, des chaînes latérales alkyle portant un groupe amino substitué et des chaînes latérales alkyle portant un groupe hydroxyle substitué ;
vi) NH(CH₂)ₙR₂, avec 0 ≤ n ≤ 10, R₂ étant choisi dans le groupe constitué par :les noyaux benzéniques, les groupes cycloalkyle en C₃₋₁₂ et les groupes hétérocycliques qui contiennent 1 à 3 hétéroatomes choisis dans le groupe constitué par O, S et N, les positions ortho, para et méta pouvant être en outre choisies indépendamment dans l'un des groupes constitués par : le groupe méthoxyle, un groupe amino, les noyaux benzéniques, les groupes alkyle, amino, nitro, hydroxyle portant des chaînes latérales en C1-C3 substituées et les groupes hétérocycliques en C₃₋₁₂ ; les groupes hétérocycliques en C₃₋₁₂ contenant 1 à 3 hétéroatomes choisis dans le groupe constitué par O, S et N ;
et leurs sels pharmaceutiquement acceptables, stéréoisomères et énantiomères.

2. Composé selon la revendication 1, dans lequel le groupe des R constitué par i)-vi) est choisi dans le groupe constitué par les groupes chloro, hydroxyle, méthoxyle, diméthylamino, pipérazin-1-yle, 4-méthylpipérazin-1-yle, 4-éthylpipérazin-1-yle, 4-(2-hydroxyéthyl)pipérazin-1-yle, 4-benzylpipérazin-1-yle, 4-phénylpipérazin-1-yle, morpholino, thiomorpholino, pipéridin-1-yle, 4-hydroxypipéridin-1-yle, 4-benzylpipéridin-1-yle, (1,4'-bipipéridin)-1'-yle, 4-(3-(pipéridin-4-yl)propyl)pipéridin-1-yle, pyrrolidin-1-yle, 2-oxopipéridin-1-yle, méthylamino, éthylamino, propylamino, butylamino, isobutylamino, pentan-3-ylamino, (2-(diméthylamino)éthyl)amino, (2-(diéthylamino)éthyl)amino, 2-éthanolamino, 3-propanolamino, 5-pentanolamino, (1-hydroxybutan-2-yl)amino, (4-méthylpentan-2-yl)amino, (2-aminoéthyl)amino, (2-((2-hydroxyéthyl)amino)éthyl)amino, (2-morpholinoéthyl)amino, (3-(diméthylamino)propyl)amino, (3-(diéthylamino)propyl)amino, (3-((2-hydroxyéthyl)amino)propyl)amino, (2,3-dihydro-1*H*-indén-2-yl)amino, cyclohexylamino, (1-benzylpipéridin-4-yl)amino, (thiophén-2-ylméthyl)amino, (cyclohexylméthyl)amino, benzylamino, (pyridin-2-ylméthyl)amino, (benzo[*d*][1,3]dioxol-5-ylméthyl)amino, (2-méthoxybenzyl)amino, (3,4-diméthoxybenzyl)amino, phénéthylamino, (4-méthoxyphénéthyl)amino, (4-aminophénéthyl)amino, guanidino et pipéridin-1-ylamino.

3. Composé selon la revendication 1, le composé étant choisi dans le groupe constitué par :
l'acide 3-((4-chlorophényl)thio)-2-hydroxyquinoléine-4-carboxylique,
la 6,9-dichloro-12*H*-thiochroméno[2,3-c]quinoléin-12-one,
la 10-chloro-6-hydroxy-12*H*-thiochroméno[2,3-*c*]quinoléin-12-one,
la 10-chloro-6-méthoxy-12*H*-thiochroméno[2,3-*c*]quinoléin-12-one,
la 10-chloro-6-diméthylamino-12*H*-thiochroméno[2,3-*c*]quinoléin-12-one,
la 10-chloro-6-(pipérazin-1-yl)-12*H*-thiochroméno[2,3-*c*]quinoléin-12-one,
la 10-chloro-6-(4-méthylpipérazin-1-yl)-12*H-*thiochroméno[2,3-*c*]quinoléin-12-one,
la 10-chloro-6-(4-éthylpipérazin-1-yl)-12*H-*thiochroméno[2,3-*c*]quinoléin-12-one,
la 10-chloro-6-(4-(2-hydroxyéthyl)pipérazin-1-yl)-12*H-*thiochroméno[2,3-*c*]quinoléin-12-one,
la 6-(4-benzylpipérazin-1-yl)-10-chloro-12*H-*thiochroméno[2,3-*c*]quinoléin-12-one,
la 10-chloro-6-(4-phénylpipérazin-1-yl)-12*H-*thiochroméno[2,3-*c*]quinoléin-12-one,
la 10-chloro-6-morpholino-12*H*-thiochroméno[2,3-*c*]quinoléin-12-one,
la 10-chloro-6-thiomorpholino-12*H*-thiochroméno[2,3-*c*]quinoléin-12-one,
la 10-chloro-6-(pipéridin-1-yl)-12*H*-thiochroméno[2,3-*c*]quinoléin-12-one,
la 10-chloro-6-(4-hydroxypipéridin-1-yl)-12*H-*thiochroméno[2,3-*c*]quinoléin-12-one,
la 6-(4-benzylpipéridin-1-yl)-10-chloro-12*H-*thiochroméno[2,3-*c*]quinoléin-12-one,
la 6-([1,4'-bipipéridin]-1'-yl)-10-chloro-12*H-*thiochroméno[2,3-*c*]quinoléin-12-one,
la 10-chloro-6-(4-(3-(pipéridin-4-yl)propyl)pipéridin-1-yl)-12*H*-thiochroméno[2,3-*c*]quinoléin-12-one,
la 10-chloro-6-(pyrrolidin-1-yl)-12*H*-thiochroméno[2,3-*c*]quinoléin-12-one,
la 10-chloro-6-(2-oxopipéridin-1-yl)-12*H-*thiochroméno[2,3-*c*]quinoléin-12-one,
la 10-chloro-6-méthylamino-12*H*-thiochroméno[2,3-*c*]quinoléin-12-one,
la 10-chloro-6-éthylamino-12*H*-thiochroméno[2,3-c]quinoléin-12-one,
la 10-chloro-6-propylamino-12*H*-thiochroméno[2,3-*c*]quinoléin-12-one,
la 6-(butylamino)-10-chloro-12*H*-thiochroméno[2,3-*c*]quinoléin-12-one,
la 10-chloro-6-isobutylamino-12*H*-thiochroméno[2,3-*c*]quinoléin-12-one,
la 10-chloro-6-(pentan-3-ylamino)-12*H*-thiochroméno[2,3-*c*]quinoléin-12-one,
la 10-chloro-6-((2-(diméthylamino)éthyl)amino)-12*H-*thiochroméno[2,3-*c*]quinoléin-12-one,
la 10-chloro-6-((2-(diéthylamino)éthyl)amino)-12*H-*thiochroméno[2,3-*c*]quinoléin-12-one,
la 10-chloro-6-(2-éthanolamino)-12*H*-thiochroméno[2,3-*c*]quinoléin-12-one,
la 10-chloro-6-(3-propanolamino)-12*H*-thiochroméno[2,3-*c*]quinoléin-12-one,
la 10-chloro-6-(5-pentanolamino)-12*H*-thiochroméno[2,3-*c*]quinoléin-12-one,
la 10-chloro-6-((1-hydroxybutan-2-yl)amino)-12*H-*thiochroméno[2,3-*c*]quinoléin-12-one,
la 10-chloro-6-((4-méthylpentan-2-yl)amino)-12*H-*thiochroméno[2,3-*c*]quinoléin-12-one,
la 6-((2-aminoéthyl)amino)-10-chloro-12*H-*thiochroméno[2,3-*c*]quinoléin-12-one,
la 10-chloro-6-((2-((2-hydroxyéthyl)amino)éthyl)amino)-12*H*-thiochroméno[2,3-*c*]quinoléin-12-one,
la 10-chloro-6-((2-morpholinoéthyl)amino)-12*H-*thiochroméno[2,3-*c*]quinoléin-12-one,
la 10-chloro-6-((3-(diméthylamino)propyl)amino)-12*H-*thiochroméno[2,3-*c*]quinoléin-12-one,
la 10-chloro-6-((3-(diéthylamino)propyl)amino)-12*H-*thiochroméno[2,3-*c*]quinoléin-12-one,
la 10-chloro-6-((3-((2-hydroxyéthyl)amino)propyl)amino)-12*H*-thiochroméno[2,3-*c*]quinoléin-12-one,
la 10-chloro-6-((2,3-dihydro-1*H*-indén-2-yl)amino)-12*H-*thiochroméno[2,3-*c*]quinoléin-12-one,
la 10-chloro-6-(cyclohexylamino)-12*H*-thiochroméno[2,3-*c*]quinoléin-12-one,
la 6-((1-benzylpipéridin-4-yl)amino)-10-chloro-12*H-*thiochroméno[2,3-*c*]quinoléin-12-one,
la 10-chloro-6-((thiophén-2-ylméthyl)amino)-12*H-*thiochroméno[2,3-*c*]quinoléin-12-one,
la 10-chloro-6-((cyclohexylméthyl)amino)-12*H-*thiochroméno[2,3-*c*]quinoléin-12-one,
la 6-(benzylamino)-10-chloro-12*H*-thiochroméno[2,3-*c*]quinoléin-12-one,
la 10-chloro-6-((pyridin-2-ylméthyl)amino)-12*H-*thiochroméno[2,3-*c*]quinoléin-12-one,
la 6-((benzo[*d*][1,3]dioxol-5-ylméthyl)amino)-10-chloro-12*H*-thiochroméno[2,3-*c*]quinoléin-12-one,
la 10-chloro-6-((2-méthoxybenzyl)amino)-12*H-*thiochroméno[2,3-*c*]quinoléin-12-one,
la 10-chloro-6-((3,4-diméthoxybenzyl)amino)-12*H-*thiochroméno[2,3-*c*]quinoléin-12-one,
la 10-chloro-6-(phénéthylamino)-12*H*-thiochroméno[2,3-*c*]quinoléin-12-one,
la 10-chloro-6-((4-méthoxyphénéthyl)amino)-12*H-*thiochroméno[2,3-*c*]quinoléin-12-one,
la 6-((4-aminophénéthyl)amino)-10-chloro-12*H-*thiochroméno[2,3-*c*]quinoléin-12-one,
la 2-(10-chloro-12-oxo-12*H*-thiochroméno[2,3-*c*]quinoléin-6-yl)guanidine,
la 10-chloro-6-(pipéridin-1-ylamino)-12*H-*thiochroméno[2,3-*c*]quinoléin-12-one,
et leurs sels.

4. Composé selon la revendication 1 destiné à être utilisé dans une méthode pour l'inhibition de l'activité de la topoisomérase I d'une cellule cancéreuse, la méthode comprenant l'administration d'une quantité efficace du composé selon la revendication 1.

5. Composé selon la revendication 1 destiné à être utilisé dans une méthode pour l'inhibition de l'activité de la topoisomérase II d'une cellule cancéreuse, la méthode comprenant l'administration d'une quantité efficace du composé selon la revendication 1.

6. Composé selon la revendication 1 destiné à être utilisé dans une méthode pour le traitement d'un cancer, la méthode comprenant l'administration d'une quantité efficace dudit composé.

7. Composé destiné à être utilisé selon la revendication 6, le cancer étant choisi dans les groupes constitués par la leucémie, le cancer du poumon non à petites cellules, le cancer colorectal, le cancer du système nerveux central (SNC), le mélanome, le cancer de l'ovaire, le cancer du rein, le cancer de la prostate et le cancer du sein.

8. Composition pharmaceutique comprenant une quantité efficace du composé selon la revendication 1 et au moins un véhicule, diluant ou excipient pharmaceutiquement acceptable.

9. Procédé pour la préparation de dérivés de thiochroméno[2,3-c]quinoléin-12-one selon la revendication 1, dans lequel procécé :
(1) un mélange d'isatine, d'acide 2-((4-chlorophényl)thio)acétique et d'acétate de sodium a été chauffé à 150 °C pendant 1 h, après refroidissement du mélange de l'acide acétique a été ajouté, le précipité a été recueilli, lavé avec de l'acide acétique, de l'eau et du *n*-hexane et le composé 2 (l'acide 3-((4-chlorophényl)thio)-2-hydroxyquinoléine-4-carboxylique) a été obtenu ;
(2) une solution de composé 2 (l'acide 3-((4-chlorophényl)thio)-2-hydroxyquinoléine-4-carboxylique) dans du trichlorure de phosphoryle a été chauffée à 150 °C pendant 48 h, après refroidissement le mélange a été versé dans de l'eau à 0 °C, le précipité a été recueilli par filtration, puis ajouté du NaHCO₃ à 10 % avec agitation vigoureuse pendant 1 h, le précipité résultant a été recueilli et lavé avec de l'H₂O, le solide brut a été recristallisé dans du dichlorométhane pour donner le composé 3 (la 6,9-dichloro-12*H*-thiochroméno[2,3-c]quinoléin-12-one) ;
(3) de l'HCl concentré a été ajouté à une solution de composé 3 (la 6,9-dichloro-12*H*-thiochroméno[2,3-*c*]quinoléin-12-one) dans du DMF et la solution a été portée au reflux, au bout de 6 heures, l'HCl concentré a été ajouté goutte à goutte et la solution a été portée au reflux pendant 12 heures supplémentaires, le mélange a été évaporé sous vide et traité avec de l'H₂O, après avoir été filtré le solide brut a été lavé avec de l'EtOH pour donner le composé 4 (la 10-chloro-6-hydroxy-12*H*-thiochroméno[2,3-*c*]quinoléin-12-one) ;
(4) une suspension de composé 3 (la 6,9-dichloro-12*H-*thiochroméno[2,3-*c*]quinoléin-12-one) et de méthylate de sodium dans du méthanol a été portée au reflux pendant 16 h, après avoir été refroidie le solvant a été enlevé, la suspension a été filtrée et lavée avec de l'éthanol et du *n*-hexane pour recueillir le composé 5 (la 10-chloro-6-méthoxy-12*H*-thiochroméno[2,3-*c*]quinoléin-12-one),
(5) une solution de composé 3 (la 6,9-dichloro-12*H-*thiochroméno[2,3-*c*]quinoléin-12-one), d'amines secondaires appropriées et de carbonate de sodium dans du DMSO a été portée au reflux pendant 10 heures, ensuite un mélange glace-eau a été ajouté à la réaction, le précipité a été filtré, lavé avec un mélange eau/méthanol pour recueillir le composé suivant :
la 10-chloro-6-diméthylamino-12*H*-thiochroméno[2,3-*c*]quinoléin-12-one,
la 10-chloro-6-(pipérazin-1-yl)-12*H-*thiochroméno[2,3-*c*]quinoléin-12-one,
la 10-chloro-6-(4-méthylpipérazin-1-yl)-12*H-*thiochroméno[2,3-*c*]quinoléin-12-one,
la 10-chloro-6-(4-éthylpipérazin-1-yl)-12*H-*thiochroméno[2,3-*c*]quinoléin-12-one,
la 10-chloro-6-(4-(2-hydroxyéthyl)pipérazin-1-yl)-12*H*-thiochroméno[2,3-*c*]quinoléin-12-one,
la 6-(4-benzylpipérazin-1-yl)-10-chloro-12*H-*thiochroméno[2,3-*c*]quinoléin-12-one,
la 10-chloro-6-(4-phénylpipérazin-1-yl)-12*H-*thiochroméno[2,3-*c*]quinoléin-12-one,
la 10-chloro-6-morpholino-12*H*-thiochroméno[2,3-*c*]quinoléin-12-one,
la 10-chloro-6-thiomorpholino-12*H-*thiochroméno[2,3-*c*]quinoléin-12-one,
la 10-chloro-6-(pipéridin-1-yl)-12*H-*thiochroméno[2,3-*c*]quinoléin-12-one,
la 10-chloro-6-(4-hydroxypipéridin-1-yl)-12*H-*thiochroméno[2,3-*c*]quinoléin-12-one,
la 6-(4-benzylpipéridin-1-yl)-10-chloro-12*H-*thiochroméno[2,3-*c*]quinoléin-12-one,
la 6-([1,4'-bipipéridin]-1'-yl)-10-chloro-12*H-*thiochroméno[2,3-*c*]quinoléin-12-one,
la 10-chloro-6-(4-(3-(pipéridin-4-yl)propyl)pipéridin-1-yl)-12*H*-thiochroméno[2,3-c]quinoléin-12-one,
la 10-chloro-6-(pyrrolidin-1-yl)-12*H-*thiochroméno[2,3-*c*]quinoléin-12-one ou
la 10-chloro-6-(2-oxopipéridin-1-yl)-12*H-*thiochroméno[2,3-c]quinoléin-12-one respectivement ;
(6) des amines primaires appropriées ont été ajoutées à une solution de composé 3 (la 6,9-dichloro-12*H-*thiochroméno[2,3-*c*]quinoléin-12-one) dans du DMSO et la solution a été portée au reflux pendant 8 heures, après avoir été refroidie de l'eau a été ajoutée à la réaction, le précipité a été filtré et lavé avec de l'eau et du méthanol pour recueillir le composé suivant :
la 10-chloro-6-méthylamino-12*H*-thiochroméno[2,3-*c*]quinoléin-12-one,
la 10-chloro-6-éthylamino-12*H*-thiochroméno[2,3-*c*]quinoléin-12-one,
la 10-chloro-6-propylamino-12*H*-thiochroméno[2,3-*c*]quinoléin-12-one,
la 6-(butylamino)-10-chloro-12*H*-thiochroméno[2,3-*c*]quinoléin-12-one,
la 10-chloro-6-isobutylamino-12*H*-thiochroméno[2,3-*c*]quinoléin-12-one,
la 10-chloro-6-(pentan-3-ylamino)-12*H-*thiochroméno[2,3-*c*]quinoléin-12-one,
la 10-chloro-6-((2-(diméthylamino)éthyl)amino)-12*H*-thiochroméno[2,3-*c*]quinoléin-12-one,
la 10-chloro-6-((2-(diéthylamino)éthyl)amino)-12*H-*thiochroméno[2,3-*c*]quinoléin-12-one,
la 10-chloro-6-(2-éthanolamino)-12*H-*thiochroméno[2,3-*c*]quinoléin-12-one,
la 10-chloro-6-(3-propanolamino)-12*H-*thiochroméno[2,3-*c*]quinoléin-12-one,
la 10-chloro-6-(5-pentanolamino)-12*H-*thiochroméno[2,3-*c*]quinoléin-12-one,
la 10-chloro-6-((1-hydroxybutan-2-yl)amino)-12*H-*thiochroméno[2,3-*c*]quinoléin-12-one,
la 10-chloro-6-((4-méthylpentan-2-yl)amino)-12*H-*thiochroméno[2,3-*c*]quinoléin-12-one,
la 6-((2-aminoéthyl)amino)-10-chloro-12*H-*thiochroméno[2,3-*c*]quinoléin-12-one,
la 10-chloro-6-((2-((2-hydroxyéthyl)amino)éthyl)amino)-12*H-*thiochroméno[2,3-*c*]quinoléin-12-one,
la 10-chloro-6-((2-morpholinoéthyl)amino)-12*H-*thiochroméno[2,3-*c*]quinoléin-12-one,
la 10-chloro-6-((3-(diméthylamino)propyl)amino)-12*H*-thiochroméno[2,3-*c*]quinoléin-12-one,
la 10-chloro-6-((3-(diéthylamino)propyl)amino)-12*H*-thiochroméno[2,3-*c*]quinoléin-12-one,
la 10-chloro-6-((3-((2-hydroxyéthyl)amino)propyl)amino)-12*H-*thiochroméno[2,3-*c*]quinoléin-12-one,
la 10-chloro-6-((2,3-dihydro-1*H*-indén-2-yl)amino)-12*H*-thiochroméno[2,3-*c*]quinoléin-12-one,
la 10-chloro-6-(cyclohexylamino)-12*H-*thiochroméno[2,3-*c*]quinoléin-12-one,
la 6-((1-benzylpipéridin-4-yl)amino)-10-chloro-12*H*-thiochroméno[2,3-*c*]quinoléin-12-one,
la 10-chloro-6-((thiophén-2-ylméthyl)amino)-12*H-*thiochroméno[2,3-*c*]quinoléin-12-one,
la 10-chloro-6-((cyclohexylméthyl)amino)-12*H-*thiochroméno[2,3-*c*]quinoléin-12-one,
la 6-(benzylamino)-10-chloro-12*H*-thiochroméno[2,3-*c*]quinoléin-12-one,
la 10-chloro-6-((pyridin-2-ylméthyl)amino)-12*H-*thiochroméno[2,3-*c*]quinoléin-12-one,
la 6-((benzo[*d*][1,3]dioxol-5-ylméthyl)amino)-10-chloro-12*H*-thiochroméno[2,3-*c*]quinoléin-12-one,
la 10-chloro-6-((2-méthoxybenzyl)amino)-12*H-*thiochroméno[2,3-*c*]quinoléin-12-one,
la 10-chloro-6-((3,4-diméthoxybenzyl)amino)-12*H-*thiochroméno[2,3-*c*]quinoléin-12-one,
la 10-chloro-6-(phénéthylamino)-12*H-*thiochroméno[2,3-*c*]quinoléin-12-one,
la 10-chloro-6-((4-méthoxyphénéthyl)amino)-12*H-*thiochroméno[2,3-*c*]quinoléin-12-one,
la 6-((4-aminophénéthyl)amino)-10-chloro-12*H-*thiochroméno[2,3-*c*]quinoléin-12-one,
la 2-(10-chloro-12-oxo-12*H*-thiochroméno[2,3-*c*]quinoléin-6-yl)guanidine ou
la 10-chloro-6-(pipéridin-1-ylamino)-12*H-*thiochroméno[2,3-*c*]quinoléin-12-one respectivement.
